# EUROPEAN PATENT APPLICATION

(11) **EP 2 443 992 A2**
(43) Date of publication of application: **25.04.2012**
(21) Application number: 11186357.7
(22) Date of filing: 24.10.2011
(51) Int. Cl.: A61B 5/00, A61B 1/273

(54) **Diagnosis support apparatus, diagnosis support method, lesioned part detection apparatus, and lesioned part detection method**

(30) Priority: 25.10.2010 JP 2010238985; 27.12.2010 JP 2010290593; 27.12.2010 JP 2010290594; 27.12.2010 JP 2010290595; 28.01.2011 JP 2011017101
(71) Applicant: Fujifilm Corporation, Minato-ku Tokyo 106-8620 (JP)
(72) Inventor: Teramura, Yuichi, Kanagawa, 258-8538 (JP); Omori, Toshihiko, Kanagawa, 258-8538 (JP); Watanabe, Daisuke, Kanagawa, 258-8538 (JP)
(74) Representative: Klunker . Schmitt-Nilson . Hirsch

(57) **Abstract**

A diagnosis support apparatus (1) and method are provided that, when a "region in which a papillary protrusion has arisen on a surface (outermost surface) of an inner wall portion", a "region in which cells (epithelial cells) are randomly proliferating on a surface (outermost surface) of an inner wall portion", or a "region in which fibrosis is progressing" which is suspected of being a lesion part exists at an inner wall portion in a living organism having a flat surface, such as the biliary tract or pancreatic duct, automatically detect the region to support diagnosis. An arithmetic processing apparatus (90) of an OCT apparatus (1), for example, detects a position of a surface of an inner wall portion of the biliary tract or pancreatic duct by means of spatial structure data, and calculates the surface roughness based on the surface shape. A region with a high degree of surface roughness is detected as a "region in which a papillary protrusion has arisen on a surface (outermost surface) of an inner wall portion" or a "region in which cells (epithelial cells) are randomly proliferating on a surface (outermost surface) of an inner wall portion".

## Description

### BACKGROUND OF THE INVENTION

### Field of the Invention

The present invention relates to a diagnosis support apparatus, a diagnosis support method, a lesion part detection apparatus, and a lesion part detection method, and more particularly to a diagnosis support apparatus, a diagnosis support method, a lesion part detection apparatus, and a lesion part detection method that support diagnosis using light intensity data (or spatial structure information) that is acquired by an OCT (Optical Coherence Tomography) apparatus that utilizes OCT measurement.

### Description of the Related Art

Optical tomographic image acquisition apparatuses (OCT apparatuses) which utilize OCT measurement are conventionally used to acquire optical tomographic images of living tissue. Such an OCT apparatus splits low coherent light emitted from a light source into a measuring light and a reference light, then multiplexes the reference light with a back-scattered light from a measurement target that is obtained when the measuring light is irradiated on the measurement target, and acquires an optical tomographic image on the basis of the intensity of an interference light between the back-scattered light and the reference light (see Japanese Patent Application Laid-Open No. 2007-225349). The above described OCT measurement is roughly divided into two types: TD-OCT (Time Domain OCT) measurement and FD-OCT (Fourier Domain OCT) measurement. TD-OCT measurement is a method for acquiring a back-scattered light intensity distribution corresponding to a position in a depth direction (hereinafter referred to as a "depth position") of a measurement target by measuring the intensity of an interference light while changing the optical path length of a reference light.

In contrast, FD-OCT measurement is a method for acquiring a reflected light intensity distribution corresponding to a depth position by measuring the intensity of an interference light for each spectral component of the light without changing the optical path lengths of a reference light and a signal light, and performing frequency analysis, typically a Fourier transform, on the obtained spectral interference intensity signals using a computer. Since FD-OCT measurement does not require mechanical scanning used in TD-OCT measurement, it has recently been drawing attention as a method which allows high-speed measurement.

Typical apparatus configurations for performing FD-OCT measurement include two types: an SD-OCT (Spectral Domain OCT) apparatus and an SS-OCT (Swept Source OCT) apparatus. An SD-OCT apparatus uses a broadband, low-coherence light, such as an SLD (Super Luminescence Diode) light source, an ASE (Amplified Spontaneous Emission) light source, or a white light, as a light source, and forms an optical tomographic image in the following manner. The broadband, low-coherence light is split into a measuring light and a reference light using a Michelson interferometer or the like. Thereafter, the measuring light is irradiated onto a measurement target, a back-scattered light that returns at that time is caused to interfere with the reference light, and the resulting interference light is split into frequency components using a spectrometer. The SD-OCT apparatus measures the interference light intensity for each frequency component using a detector array in which elements such as photodiodes are arranged in an array, and forms an optical tomographic image by performing a Fourier transform on the obtained spectral interference intensity signals using a computer.

The above described OCT apparatus can acquire three-dimensional structure information (referred to as "spatial structure information") of a measurement target by two-dimensionally scanning an optical axis of the measuring light. Technology has already been proposed that supports diagnostic imaging by acquiring spatial structure information of a site inside a body cavity of the human body by means of an OCT apparatus, and picturizes (visualizes) the spatial structure information and displays a three-dimensional image on a monitor, or analyzes the spatial structure information to automatically detect a lesion part (for example, see Japanese Patent Application Laid-Open No. 2010-145297 or Japanese Patent Application Laid-Open No. 2010-158343).

Further, International Publication No. WO 2006-022045 discloses technology that acquires tomographic information of living tissue of, for example, the mucous membrane, the trachea, or the alimentary canal that has a layered structure using an OCT apparatus, and measures a layer thickness by means of the acquired tomographic information. For example, it is suggested that measurement of a layer thickness in the retina is useful for diagnosing an increase or decrease in retinal thickness caused by retinal edema, or the degree of progress of glaucoma.

Japanese Patent Application Laid-Open No. 2009-72280 proposes technology that takes into consideration a fact that, when performing OCT measurement, a region in which an OCT probe (probe outer casing) and a surface of a measurement target are in contact (contact region) and a region in which the OCT probe and the surface of the measurement target are not in contact (non-contact region) exist, and that the resolution of tomographic information is lower in the non-contact region compared to the contact region, and therefore performs measurement of only a contact region or subjects tomographic information of only a contact region to image processing and displays only that tomographic information.

In this connection, the biliary tract and the pancreatic duct are known as regions in which the cure rate is extremely low when cancer develops therein. For diagnosis of the biliary tract or the pancreatic duct, first, abdominal ultrasonography or a blood test is performed as a primary screening and, as a secondary screening, diagnostic imaging is carried out using X-ray computed tomography (CT) or magnetic resonance cholangiopancreatography (MRCP), and modalities (medical equipment) such as endoscopic retrograde cholangio-pancreatography (ERCP), endoscopic ultrasoundscopy (EUS), and intraductal ultrasonography (IDUS) are used to specify the cancer location and diagnose the depth of infiltration and the level of progression. When it is determined as the result of an overall decision that surgical resection is effective, the region to be resected is decided and is surgically resected. The resected tissue is subjected to a histological search, and diagnosis is performed with respect to whether the cancer has advanced as far as the resection stump. If cancer remains in the resection stump, it can be strongly presumed that there is left-over cancer, and additional resection or radiation and chemotherapy are necessary.

### SUMMARY OF THE INVENTION

However, abdominal ultrasonography and endoscopic ultrasoundscopy (EUS) are not suited to observation of morphological changes of a duct wall. In the case of X-ray CT and ERCP, although diagnosis can be performed using these techniques when the shape of a duct wall is picturized and there is a large tumor of a size of 1 mm or more, it is difficult to discern changes less than that size. IDUS is a technique in which a probe is inserted directly into a duct, and although the technique is therefore suitable for observation of a duct wall, the resolution is around 100 µm. However, the size of morphological changes in cancer cells that spread laterally inside the epithelium of the pancreatic duct or the bile duct may be around 20 µm in some cases, and thus the resolution obtained with IDUS is inadequate for observing such morphological changes.

Consequently, according to the conventional diagnosis method, the accuracy with respect to cancer cells that spread laterally is poor and the occurrence of cases in which it is identified that a resection stump is positive, i.e. cancer cells are left over, by histological diagnosis after performing a surgical resection has been a problem. Accordingly, high-sensitivity diagnosis of laterally spreading cancer cells as well as highly precise determination of surgical resection lines is an important issue.

OCT apparatuses offer a high resolution and are effective for performing diagnostic imaging of the biliary tract and the pancreatic duct, and automatic diagnosis that analyzes spatial structure information of the biliary tract or the pancreatic duct and automatically detects a lesion part is also considered to be useful for supporting diagnosis by a physician. However, thus far, technology that attempts to perform automatic diagnosis to discern a normal site and an abnormal site in the biliary tract or the pancreatic duct has not been available. Although technology that attempts to perform automatic diagnosis is proposed in Japanese Patent Application Laid-Open No. 2010-145297 and Japanese Patent Application Laid-Open No. 2010-158343, the target of the automatic diagnosis is principally the colon, and such automatic diagnosis does not detect morphological changes of a lesion part that are unique to the biliary tract and the pancreatic duct.

In this case, as morphological changes (characteristic structures) which are suspected of being cancer or the like at an inner wall portion of the biliary tract or the pancreatic duct, a case in which a papillary protrusion has arisen on a surface (epithelium) of the inner wall portion and a case in which cells (epithelial cells) have proliferated randomly are known. Although other morphological changes in which cancer or the like is suspected are also known, if at least these two morphological changes can be automatically detected, such automatic detection will be useful for supporting diagnosis of the biliary tract or the pancreatic duct.

Further, irrespective of the biliary tract and the pancreatic duct, with respect also to organs that have a flat epithelial structure at a normal time such as, for example, a bronchial tube, the pharynx, the esophagus, and the urinary duct, if similar morphological changes due to cancer or the like can be automatically detected, such automatic detection will be useful for supporting diagnosis.

Furthermore, a case in which fibrosis has progressed is also known as a morphological change (characteristic structure) which is suspected of being cancer or the like at an inner wall portion of the biliary tract or the pancreatic duct. Although other morphological changes in which cancer or the like is suspected are also known, if at least the above described morphological change can be automatically detected, such automatic detection will be useful for supporting diagnosis of the biliary tract or the pancreatic duct.

Irrespective of the biliary tract and the pancreatic duct, with respect also to organs that have a layered structure at a normal time such as, for example, a bronchial tube, the pharynx, the esophagus, the stomach, the colon, the uterus, and the urinary duct, if a similar morphological change due to cancer or the like can be automatically detected, such automatic detection will be useful for supporting diagnosis.

It is desirable to enable an observer to easily ascertain a region in which there is a high possibility of a lesion part such as a cancer being present, since the workload can be thereby reduced. Further, irrespective of the biliary tract and the pancreatic duct, with respect also to organs such as, for example, a bronchial tube, the pharynx, the esophagus, the stomach, the colon, the uterus, and the urinary duct, likewise, if it is possible to enable ascertainment of a region for which there is a high possibility of a lesion part being present, it will be useful for supporting diagnosis.

In addition, at an inner wall portion of an organ in which an epithelial layer has an approximately constant thickness at a normal time such as, for example, the biliary tract, the pancreatic duct, a bronchial tube, the pharynx, the esophagus, the stomach, the colon, the uterus, and the urinary duct, a site at which a thickness of the epithelial layer is enlarged in comparison to a normal time (normal site) is suspected of being a lesion part, such as cancer. For example, as morphological changes (characteristic structures) in an epithelial layer which are suspected of being cancer or the like, a case in which a papillary protrusion has arisen on the surface (epithelium), a case in which the epithelial layer has thickened, and a case in which cells (epithelial cells) have proliferated randomly are known, and the thickness of the epithelial layer at sites at which such morphological changes have occurred increases in comparison to a normal site. Accordingly, it will be useful for supporting diagnosis if the thickness of an epithelial layer can be measured based on tomographic information acquired by OCT measurement, and a site at which the thickness has increased in comparison to a normal site can be automatically detected and displayed.

However, a measurement region for which tomographic information is acquired by OCT measurement includes a contact region at which the OCT probe contacts the surface of the measurement target and a non-contact region at which the OCT probe does not contact the surface of the measurement target, and due to a pressing effect of the OCT probe at the contact region, the thickness of an epithelial layer at a normal site and a lesion part differs between a contact region and a non-contact region. Consequently, when detecting a lesion part based on the thickness of an epithelial layer, it is necessary to take into consideration whether or not there is contact between the OCT probe and the surface of the measurement target at the time of measurement.

Although International Publication No. WO 2006-022045 and Japanese Patent Application Laid-Open No. 2009-72280 disclose a method of measuring a layer thickness based on tomographic information and a method of distinguishing between a region of contact between an OCT probe and a measurement target and a non-contact region, International Publication No. WO 2006-022045 and Japanese Patent Application Laid-Open No. 2009-72280 do not propose automatically detecting a lesion part or taking into consideration whether a region is a contact region or a non-contact region at such time. Likewise, Japanese Patent Application Laid-Open Nos. 2007-225349, 2010-145297, and 2010-158343 do not contain any description regarding taking into consideration whether a region is a contact region or a non-contact region when detecting a lesion part.

The present invention has been conceived in view of the above described situation, and an object of the present invention is to provide a diagnosis support apparatus that, when a "region in which a papillary protrusion has arisen on a surface (outermost surface) of an inner wall portion" and a "region in which cells (epithelial cells) are randomly proliferating on a surface (outermost surface) of an inner wall portion" which are suspected of being a lesion part exist at an inner wall portion within a living organism that has a flat surface at a normal time, such as the biliary tract or the pancreatic duct, automatically detects the region to thereby support diagnosis.

Another object of the present invention is to provide a diagnosis support apparatus, a diagnosis support method, a lesion part detection apparatus, and a lesion part detection method that, when a "region in which fibrosis is progressing" which is suspected of being a lesion part exists at an inner wall portion within a living organism, such as the biliary tract or the pancreatic duct, automatically detect the region to thereby support diagnosis.

A further object of the present invention is to provide a diagnosis support apparatus, a diagnosis support method, a lesion part detection apparatus, and a lesion part detection method that, when a "region in which fibrosis is progressing" which is suspected of being a lesion part exists at an inner wall portion within a living organism that has a layered structure at a normal time, such as the biliary tract or the pancreatic duct, automatically detect the region to thereby support diagnosis.

A further object of the present invention is to provide a diagnosis support apparatus, a diagnosis support method, a lesion part detection apparatus, and a lesion part detection method that enable an observer to easily ascertain a region which has a high possibility of being a lesion part, to thereby enable a reduction in a workload.

A still further object of present invention is to provide a diagnosis support apparatus, a diagnosis support method, a lesion part detection apparatus, and a lesion part detection method that, based on tomographic information (light intensity data) acquired by performing OCT measurement with respect to an inner wall portion within a living organism that has an epithelial layer (first layer) of a predetermined thickness on a surface thereof such as, for example, the biliary tract, the pancreatic duct, a bronchial tube, the pharynx, the esophagus, the stomach, the colon, the uterus and the urinary duct, when automatically detecting a lesion part at which a thickness of the epithelial layer is greater than a predetermined threshold value, appropriately perform detection of a lesion part by taking into consideration whether or not there is contact between an OCT probe and the surface of the inner wall portion at a time of measurement.

To achieve the above described objects, a diagnosis support apparatus according to a first aspect of the present invention includes: a spatial structure data acquisition means that acquires spatial structure data including tomographic information of a three-dimensional region of an inner wall portion within a living organism that has a flat surface at a normal time that is obtained by performing optical coherence tomography measurement with respect to the inner wall portion; a surface roughness calculation means that calculates an evaluation value of a surface roughness at respective positions on the surface of the inner wall portion based on the spatial structure data that is acquired by the spatial structure data acquisition means; a lesion part extraction means that extracts a region of a lesion part based on a position on the surface at which the evaluation value that is calculated by the surface roughness calculation means exceeds a predetermined threshold value; and a lesion part display means that displays information showing the region of the lesion part that is extracted by the lesion part extraction means on an image in which the spatial structure data is visualized.

According to the present invention, in a case where a "region in which a papillary protrusion has arisen on a surface of an inner wall portion" or a "region in which cells (epithelial cells) are randomly proliferating on a surface of an inner wall portion" which are suspected of being a lesion part, such as a cancer, exist at an inner wall portion within a living organism that has a flat surface at a normal time, such as the biliary tract or the pancreatic duct, since the surface of the inner wall portion becomes rough, by detecting and displaying regions in which the surface of the inner wall portion is rough, it is possible for an observer to easily ascertain the regions which are suspected of being a lesion part.

A diagnosis support apparatus according to a second aspect of the present invention is in accordance with the invention according to the first aspect, wherein the surface roughness calculation means includes: a surface detection means that detects a position of the surface based on a change in a value of the spatial structure data with respect to a depth direction of the inner wall portion; and a mean position calculation means that determines a mean position in the depth direction in a predetermined range of the surface based on positions of the surface that are detected by the surface detection means; wherein when a mean position that is calculated by the mean position calculation means in the predetermined range of the surface is taken as a fixed position in the depth direction, a difference amount between a deepest position and a shallowest position of the surface with regard to the depth direction is calculated as the evaluation value.

The present invention illustrates a preferred specific embodiment for calculating an evaluation value of surface roughness.

A diagnosis support apparatus according to a third aspect of the present invention is in accordance with the invention according to the first or second aspect, wherein when a continuous region of a size that is equal to or greater than a predetermined size is formed by positions on the surface at which the evaluation value exceeds a predetermined threshold value, the lesion part extraction means extracts the region as a region of a lesion part.

In a case in which it is determined that only a very small region of a surface of an inner wall is rough, since such a region does not correspond to a "region in which a papillary protrusion has arisen on an outermost surface of an inner wall portion" or a "region in which cells (epithelial cells) are randomly proliferating on a surface of an inner wall portion", the present invention excludes such a region.

A diagnosis support apparatus according to a fourth aspect of the present invention is in accordance with the invention according to the first, second, or third aspect, wherein the lesion part display means applies a predetermined color to a region of the lesion part on an image in which the spatial structure data is visualized and displays a resulting image.

According to the present invention, an observer can ascertain at a glance a detected region of a lesion part.

A diagnosis support apparatus according to a fifth aspect of the present invention is in accordance with the invention according to any one of the first to fourth aspects, wherein, as an image in which the spatial structure data is visualized, the lesion part display means displays at least any one image among a group including a three-dimensional image obtained by perspective projection processing, a three-dimensional image obtained by parallel projection processing, and a tomogram of a predetermined cross section.

The present invention shows that, since spatial structure data has three-dimensional structure information, images of various forms can be generated, and it is possible to display a three-dimensional image to appear as a solid object or a tomogram of only a specific cross section. A lesion part can be displayed in any of the images.

A diagnosis support apparatus according to a sixth aspect is in accordance with the invention according to any one of the first to fifth aspects, wherein the inner wall portion is an inner wall portion of a biliary tract, a pancreatic duct, a bronchial tube, a pharynx, an esophagus, or a urinary duct that has a flat epithelial structure at a normal time.

The present invention limits in-vivo sites at which the inventions according to the first to fifth aspects are particularly effective.

To achieve the above described objects, a diagnosis support apparatus according to a seventh aspect of the present invention includes: a light intensity data acquisition means that acquires light intensity data of an inner wall portion within a living organism that is obtained by performing optical coherence tomography measurement with respect to the inner wall portion; an evaluation value calculation means that, based on light intensity data acquired by the light intensity data acquisition means, calculates an evaluation value corresponding to a length from a measurement point that is set on a surface of the inner wall portion to a boundary point at which the light intensity data with regard to a depth direction of the inner wall portion becomes a noise level; a detection means that compares a size of the evaluation value that is calculated by the evaluation value calculation means and a size of a predetermined threshold value, and detects a measurement point at which the evaluation value is less than the threshold value as a lesion part; and a lesion part display means that displays information showing a region of the lesion part that is detected by the detection means on an image in which the light intensity data is visualized.

According to the present invention, in a case where a "region in which fibrosis is progressing" which is suspected of being a lesion part such as a cancer exists at an inner wall portion within a living organism, such as the biliary tract or the pancreatic duct, since a scattered light intensity at that portion increases and an effective signal length in the depth direction of the inner wall portion (length in the depth direction from the surface of the inner wall portion to a position at a boundary of a region of a signal boundary) is shortened by a corresponding amount, by automatically detecting and displaying such a region, an observer can easily ascertain a region which is suspected of being a lesion part.

A diagnosis support apparatus according to an eighth aspect of the present invention is in accordance with the invention according to the seventh aspect, wherein the detection means detects a measurement point at which the evaluation value is less than the threshold value, and when a set of the measurement points forms a continuous region of a size that is equal to or greater than a predetermined size, the detection means detects the region as a lesion part.

When a region in which an intensity of a back-scattered light increases is limited to a minute area, since the region does not correspond to a "region in which fibrosis is progressing", the present invention excludes the region.

A diagnosis support apparatus according to a ninth aspect of the present invention is in accordance with the invention according to the seventh or eighth aspect, further including: a contact region distinguishing means that, based on the light intensity data, with respect to the surface of the inner wall portion, distinguishes between an optical probe contact region which an optical probe that irradiates a measuring light on the inner wall portion contacts and an optical probe non-contact region which the optical probe does not contact at a time of the optical coherence tomography measurement; and a threshold value changing means that changes the threshold value in the detection means so as to set respectively different threshold values for the optical probe contact region and the optical probe non-contact region that are distinguished by the contact region distinguishing means.

According to the present invention, since a threshold value for extracting a lesion part is changed in accordance with the existence or non-existence of contact between an outer circumferential face of an optical probe and an inner wall portion within a living organism, it is possible to appropriately distinguish between a normal site and a lesion part.

A diagnosis support apparatus according to a tenth aspect of the present invention is in accordance with the invention according to the ninth aspect, further including a probe detection means that detects a position of an outer circumferential face of the optical probe based on the light intensity data, wherein when a position of the outer circumferential face of the optical probe that is detected by the probe detection means and a position of the surface of the inner wall portion that is detected by the surface detection means are in a range in which the positions are regarded as being at an identical position, the contact region distinguishing means determines that a region in question is an optical probe contact region, and in other cases the contact region distinguishing means determines that a region in question is an optical probe non-contact region.

The present invention illustrates a preferred specific embodiment that distinguishes between an optical probe contact region and an optical probe non-contact region.

A diagnosis support apparatus according to an eleventh aspect of the present invention is in accordance with the invention according to the ninth or tenth aspect, wherein, as the threshold value, the threshold value changing means sets a value for the optical probe contact region that is smaller than a value for the optical probe non-contact region.

According to the present invention, taking into consideration a fact that an effective length of a signal shortens due to a pressing effect of the optical probe in an optical probe contact region compared to an optical probe non-contact region, a threshold value for detecting a lesion part is set to a smaller value for a contact region compared to a non-contact region. It is thereby possible to appropriately distinguish between a normal site and a lesion part.

A diagnosis support apparatus according to a twelfth aspect of the present invention is in accordance with the invention according to any one of the seventh to eleventh aspects, wherein the lesion part display means applies a predetermined color to the lesion part on an image in which the light intensity data is visualized and displays a resulting image.

According to the present invention, an observer can ascertain a detected lesion part at a glance.

A diagnosis support apparatus according to a thirteenth aspect of the present invention is in accordance with the invention according to any one of the seventh to twelfth aspects, wherein the light intensity data acquisition means acquires light intensity data of a three-dimensional region of the inner wall portion.

The present invention is a preferred specific embodiment in which the light intensity data acquisition means acquires light intensity data of a three-dimensional region of an inner wall portion.

A diagnosis support apparatus according to a fourteenth aspect of the present invention is in accordance with the invention according to the thirteenth aspect, wherein, as an image in which the light intensity data is visualized, the lesion part display means displays at least any one image among a group including a three-dimensional image obtained by perspective projection processing, a three-dimensional image obtained by parallel projection processing, and a tomogram of a predetermined cross section.

The present invention shows that since light intensity data of a three-dimensional region of an inner wall portion is acquired, various forms of images can be generated, and it is possible to display a three-dimensional image to appear as a solid object or a tomogram of only a specific cross section. A lesion part can be displayed in any of the images.

A diagnosis support apparatus according to a fifteenth aspect of the present invention is in accordance with the invention according to any one of the seventh to fourteenth aspects, wherein the inner wall portion is an inner wall portion of a biliary tract, a pancreatic duct, a bronchial tube, a pharynx, an esophagus, a stomach, a colon, a uterus, or a urinary duct.

The present invention limits in-vivo sites at which the inventions according to the seventh to fourteenth aspects are particularly effective.

Further, to achieve the above described objects, a diagnosis support method according to sixteenth aspect of the present invention includes: a light intensity data acquisition step of acquiring light intensity data of an inner wall portion within a living organism that is obtained by performing optical coherence tomography measurement with respect to the inner wall portion; an evaluation value calculation step of, based on light intensity data acquired by the light intensity data acquisition step, calculating an evaluation value that shows a length from a measurement point that is set on a surface of the inner wall portion to a boundary point at which the light intensity data with regard to a depth direction of the inner wall portion becomes a noise level; a detection step of comparing a size of the evaluation value that is calculated by the evaluation value calculation step and a size of a predetermined threshold value, and detecting a measurement point at which the evaluation value is less than the threshold value as a lesion part; and a lesion part display step of displaying information showing a region of the lesion part that is detected by the detection step on an image in which the light intensity data is visualized.

Furthermore, to achieve the above described objects, a lesion part detection apparatus according to seventeenth aspect of the present invention includes: a light intensity data acquisition means that acquires light intensity data of an inner wall portion within a living organism that is obtained by performing optical coherence tomography measurement with respect to the inner wall portion; an evaluation value calculation means that, based on light intensity data acquired by the light intensity data acquisition means, calculates an evaluation value corresponding to a length from a measurement point that is set on a surface of the inner wall portion to a boundary point at which the light intensity data with regard to a depth direction of the inner wall portion becomes a noise level; and a detection means that compares a size of the evaluation value that is calculated by the evaluation value calculation means and a size of a predetermined threshold value, and detects a measurement point at which the evaluation value is less than the threshold value as a lesion part.

Further, to achieve the above described objects, a lesion part detection method according to an eighteenth aspect of the present invention includes: a light intensity data acquisition step of acquiring light intensity data of an inner wall portion within a living organism that is obtained by performing optical coherence tomography measurement with respect to the inner wall portion; an evaluation value calculation step of, based on light intensity data that is acquired by the light intensity data acquisition step, calculating an evaluation value corresponding to a length from a measurement point that is set on a surface of the inner wall portion to a boundary point at which the light intensity data with regard to a depth direction of the inner wall portion becomes a noise level; and a detection step of comparing a size of the evaluation value that is calculated by the evaluation value calculation step and a size of a predetermined threshold value, and detecting a measurement point at which the evaluation value is less than the threshold value as a lesion part.

To achieve the above described objects, a diagnosis support apparatus according to a nineteenth aspect of the present invention includes: a light intensity data acquisition means that acquires light intensity data of an inner wall portion within a living organism that has a layered structure that is obtained by performing optical coherence tomography measurement with respect to the inner wall portion; an addition means that, based on light intensity data that is acquired by the light intensity data acquisition means, adds light intensity data of a predetermined range at a same depth with regard to a depth direction of the inner wall portion from a measurement point set on a surface of the inner wall portion; a detection means that detects the measurement point as a lesion part when an addition value that is calculated by the addition means shows a predetermined change along the depth direction; and a lesion part display means that displays information showing the lesion part that is detected by the detection means on an image in which the light intensity data is visualized.

According to the present invention, in a case where a "region in which fibrosis is progressing" which is suspected of being a lesion part such as a cancer exists at an inner wall portion within a living organism that has a layered structure at a normal time, such as the biliary tract or the pancreatic duct, since a layered structure of the inner wall portiondisappears, and consequently an addition value that is obtained by adding light intensity data of a predetermined range that, when respective positions in the depth direction of the inner wall portion are taken as a reference position, exists at the same depth position as the reference position, shows a constant attenuation along the depth direction, by automatically detecting and displaying such a region, an observer can easily ascertain a region which is suspected of being a lesion part.

A diagnosis support apparatus according to a twentieth aspect of the present invention is in accordance with the invention according to the nineteenth aspect, wherein when the addition value that is calculated by the addition means is in a range that is regarded as attenuating in a constant manner along the depth direction, the detection means detects the measurement point as a lesion part.

According to the present invention, it is possible to prevent a detection oversight with regard to a region in which a layered structure has disappeared.

A diagnosis support apparatus according to a twenty-first aspect of the present invention is in accordance with the invention according to the nineteenth or twentieth aspect, wherein the detection means detects a measurement point at which an addition value that is calculated by the addition means shows a predetermined change along the depth direction, and when a set of the measurement points forms a continuous region that is equal to or greater than a predetermined size, the detection means detects the region as a lesion part.

When a region in which an addition value shows a constant attenuation is limited to a minute range, since the region does not correspond to a "region in which fibrosis is progressing", the present invention excludes such a region.

A diagnosis support apparatus according to a twenty-second aspect of the present invention is in accordance with the invention according to any one of the nineteenth to twenty-first aspects, wherein the lesion part display means applies a predetermined color to a region of the lesion part on an image in which the light intensity data is visualized and displays a resulting image.

According to the present invention, an observer can ascertain a region of a detected lesion part at a glance.

A diagnosis support apparatus according to a twenty-third aspect of the present invention is in accordance with the invention according to any one of the nineteenth to twenty-second aspects, wherein the light intensity data acquisition means acquires light intensity data of a three-dimensional region of the inner wall portion.

The present invention is a preferred specific embodiment in which, preferably, the light intensity data acquisition means acquires light intensity data of a three-dimensional region of an inner wall portion.

A diagnosis support apparatus according to a twenty-fourth aspect of the present invention is in accordance with the invention according to any one of the nineteenth to twenty-third aspects, wherein, as an image in which the light intensity data is visualized, the lesion part display means displays at least any one image among a group including a three-dimensional image obtained by perspective projection processing, a three-dimensional image obtained by parallel projection processing, and a tomogram of a predetermined cross section.

The present invention shows that since light intensity data of a three-dimensional region of an inner wall portion is acquired, various forms of images can be generated, and it is possible to display a three-dimensional image to appear as a solid object or a tomogram of only a specific cross section. A lesion part can be displayed in any of the images.

A diagnosis support apparatus according to a twenty-fifth aspect of the present invention is in accordance with the invention according to any one of the nineteenth to twenty-fourth aspects, wherein the inner wall portion is an inner wall portion of a biliary tract, a pancreatic duct, a bronchial tube, a pharynx, an esophagus, a stomach, a colon, a uterus, or a urinary duct.

The present invention limits in-vivo sites at which the inventions according to the nineteenth to twenty-fourth aspects are particularly effective.

Further, to achieve the above described objects, a diagnosis support method according to a twenty-sixth aspect of the present invention includes: a light intensity data acquisition step of acquiring light intensity data of an inner wall portion within a living organism that has a layered structure that is obtained by performing optical coherence tomography measurement with respect to the inner wall portion; an addition step of, based on light intensity data that is acquired by the light intensity data acquisition step, adding light intensity data of a predetermined range at a same depth with regard to a depth direction of the inner wall portion from a measurement point set on a surface of the inner wall portion; a detection step of detecting the measurement point as a lesion part when an addition value that is calculated by the addition step shows a predetermined change along the depth direction; and a lesion part display step of displaying information showing the lesion part that is detected by the detection step on an image in which the light intensity data is visualized.

Furthermore, to achieve the above described objects, a lesion part detection apparatus according to a twenty-seventh aspect of the present invention includes: a light intensity data acquisition means that acquires light intensity data of an inner wall portion within a living organism that has a layered structure that is obtained by performing optical coherence tomography measurement with respect to the inner wall portion; an addition means that, based on light intensity data that is acquired by the light intensity data acquisition means, adds light intensity data of a predetermined range at a same depth with regard to a depth direction of the inner wall portion from a measurement point set on a surface of the inner wall portion; and a detection means that detects the measurement point as a lesion part when an addition value that is calculated by the addition means shows a predetermined change along the depth direction.

Further, to achieve the above described objects, a lesion part detection method according to a twenty-eighth aspect of the present invention includes: a light intensity data acquisition step of acquiring light intensity data of an inner wall portion within a living organism that has a layered structure that is obtained by performing optical coherence tomography measurement with respect to the inner wall portion; an addition step of, based on light intensity data that is acquired by the light intensity data acquisition step, adding light intensity data of a predetermined range at a same depth with regard to a depth direction of the inner wall portion from a measurement point set on a surface of the inner wall portion; and a detection step of detecting the measurement point as a lesion part when an addition value that is calculated by the addition step shows a predetermined change along the depth direction.

According to the present invention, when a "region in which fibrosis is progressing" which is suspected of being a lesion part exists at an inner wall portion within a living organism that has a layered structure at a normal time, such as the biliary tract or the pancreatic duct, the region can be automatically detected and thus diagnosis can be supported.

To achieve the above described objects, a diagnosis support apparatus according to a twenty-ninth aspect of the present invention includes: a light intensity data acquisition means that acquires light intensity data of an inner wall portion within a living organism that is obtained by performing optical coherence tomography measurement with respect to the inner wall portion; an extraction means that extracts a plurality of feature regions which are suspected of being a lesion part based on light intensity data that is acquired by the light intensity data acquisition means; a weight assigning means that assigns points in accordance with a degree of possibility of being a lesion part to each feature region that is extracted by the extraction means; a degree of risk setting means that sets a degree of risk at respective positions of the inner wall portion based on points that are assigned to each feature region by the weight assigning means; and a display means that, on an image in which the light intensity data is visualized, assigns a different color for each degree of risk at the respective positions of the inner wall portion for which a degree of risk is set by the degree of risk setting means, and displays a resulting image.

According to the present invention, a plurality of feature regions which are suspected of being a lesion part such as a cancer are detected on an inner wall portion within a living organism, and points are assigned to each feature region in accordance with a degree of possibility of being a lesion part. Subsequently, a degree of risk is set for respective positions of the inner wall portion based on the points assigned to each feature region, and the respective positions of the inner wall portion are color coded according to the respective degrees of risk and displayed. Thus, an observer can easily ascertain a region which has a high possibility of being a lesion part, and therefore the workload can be reduced.

A diagnosis support apparatus according to a thirtieth aspect of the present invention is in accordance with the invention according to the twenty-ninth aspect, further including a classifying means that classifies the plurality of feature regions extracted by the extraction means into predetermined categories, wherein the weight assigning means assigns points to each feature region in accordance with a degree of possibility of being a lesion part with respect to each category into which the plurality of feature regions are classified by the classifying means.

According to the present invention, since the plurality of feature regions extracted by the extraction means are classified into predetermined categories, assignment of points in accordance with a degree of possibility of being a lesion part can be easily carried out with respect to each category.

A diagnosis support apparatus according to a thirty-first aspect of the present invention is in accordance with the invention according to the thirtieth aspect, wherein the classifying means classifies each feature region according to a kind of detection processing that extracts each feature region.

According to the present invention, feature regions extracted by means of respective kinds of detection processing can be efficiently classified.

A diagnosis support apparatus according to a thirty-second aspect of the present invention is in accordance with the invention according to the thirtieth or thirty-first aspect, wherein the classifying means classifies the plurality of feature regions into at least any one category among a group including a region in which a layered structure of the inner wall portion has disappeared, a region in which an outermost surface layer of the inner wall portion is thickened, a region in which a surface roughness of a surface of the inner wall portion is abnormal, and a region in which a lumen exists in the inner wall portion.

The present invention illustrates specific forms of categories into which respective feature regions are classified.

A diagnosis support apparatus according to a thirty-third aspect of the present invention is in accordance with the invention according to any one of the thirtieth to thirty-second aspects, further including a criterion setting means that sets a criterion for classifying the plurality of feature regions into predetermined categories, wherein the classifying means classifies the plurality of feature regions according to the criterion that is set by the criterion setting means.

According to the present invention, it is possible to appropriately set a criterion for classifying each feature region according to the experience or preference of the operator. It is thereby possible to add a color to a region that should be observed (region with a high degree of risk) to place emphasis on the relevant region, and thus the workload can be reduced.

A diagnosis support apparatus according to a thirty-fourth aspect of the present invention is in accordance with the invention according to any one of the twenty-ninth to thirty-third aspects, wherein, with respect to an overlapping region in which at least some of the plurality of feature regions overlap, the degree of risk setting means adds points that are assigned to each feature region, respectively, and sets a degree of risk with respect to the overlapping region based on the addition value.

According to the present invention, by making a degree of risk with respect to an overlapping region in which at least some of a plurality of feature regions overlap a value that is obtained by adding points assigned to the respective feature regions, an observer can instantly ascertain a region for which a risk of being a lesion part is particularly high.

A diagnosis support apparatus according to a thirty-fifth aspect of the present invention is in accordance with the invention according to any one of the twenty-ninth to thirty-fourth aspects, wherein the light intensity data acquisition means acquires light intensity data of a three-dimensional region of the inner wall portion.

The present invention is a preferred specific embodiment in which, preferably, the light intensity data acquisition means acquires light intensity data of a three-dimensional region of an inner wall portion.

A diagnosis support apparatus according to a thirty-sixth aspect of the present invention is in accordance with the invention according to any one of the twenty-ninth to thirty-fifth aspects, wherein, as an image in which the spatial structure data is visualized, the display means displays at least any one image of a group including a three-dimensional image obtained by perspective projection processing, a three-dimensional image obtained by parallel projection processing, and a tomogram of a predetermined cross section.

The present invention shows that, since spatial structure data has three-dimensional structure information, images of various forms can be generated, and it is possible to display a three-dimensional image to appear as a solid object or a tomogram of only a specific cross section. A lesion part can be displayed in any of the images.

A diagnosis support apparatus according to a thirty-seventh aspect of the present invention is in accordance with the invention according to any one of the twenty-ninth to thirty-sixth aspects, wherein the inner wall portion is an inner wall portion of a biliary tract, a pancreatic duct, a bronchial tube, a pharynx, an esophagus, a stomach, a colon, a uterus, or a urinary duct.

The present invention limits in-vivo sites at which the inventions according to the twenty-ninth to thirty-sixth aspects are particularly effective.

Further, to achieve the above described objects, a diagnosis support method according to a thirty-eighth aspect of the present invention includes: a light intensity data acquisition step of acquiring light intensity data of an inner wall portion within a living organism that is obtained by performing optical coherence tomography measurement with respect to the inner wall portion; an extraction step of extracting a plurality of feature regions which are suspected of being a lesion part based on light intensity data that is acquired by the light intensity data acquisition step; a weight assigning step of assigning points in accordance with a degree of possibility of being a lesion part to each feature region that is extracted by the extraction step; a degree of risk setting step of setting a degree of risk at respective positions of the inner wall portion based on points that are assigned to each feature region by the weight assigning step; and a display step of assigning a different color for each degree of risk at the respective positions of the inner wall portion for which a degree of risk is set by the degree of risk setting step on an image in which the light intensity data is visualized, and displaying a resulting image.

Further, to achieve the above described objects, a lesion part detection apparatus according to a thirty-ninth aspect of the present invention includes: a light intensity data acquisition means that acquires light intensity data of an inner wall portion within a living organism that is obtained by performing optical coherence tomography measurement with respect to the inner wall portion; an extraction means that extracts a plurality of feature regions which are suspected of being a lesion part based on light intensity data that is acquired by the light intensity data acquisition means; a weight assigning means that assigns points in accordance with a degree of possibility of being a lesion part to each feature region that is extracted by the extraction means; and a degree of risk setting means that sets a degree of risk at respective positions of the inner wall portion based on points that are assigned to each feature region by the weight assigning means.

Furthermore, to achieve the above described objects, a lesion part detection method according to a fortieth aspect of the present invention includes: a light intensity data acquisition step of acquiring light intensity data of an inner wall portion within a living organism that is obtained by performing optical coherence tomography measurement with respect to the inner wall portion; an extraction step of extracting a plurality of feature regions which are suspected of being a lesion part based on light intensity data that is acquired by the light intensity data acquisition step; a weight assigning step of assigning points in accordance with a degree of possibility of being a lesion part to each feature region that is extracted by the extraction step; and a degree of risk setting step of setting a degree of risk at respective positions of the inner wall portion based on points that are assigned to each feature region by the weight assigning step.

To achieve the above objects, a diagnosis support apparatus according to a forty-first aspect of the present invention includes: a light intensity data acquisition means that acquires light intensity data of an inner wall portion within a living organism that has a first layer of at least a predetermined thickness on a surface thereof that is obtained by performing optical coherence tomography measurement with respect to the inner wall portion; a detection means that, based on light intensity data that is acquired by the light intensity data acquisition means, compares a thickness of the first layer at a measurement point that is set on the surface of the inner wall portion and a predetermined threshold value, and detects the measurement point as a lesion part when the thickness of the first layer is a value that is greater than the threshold value; a lesion part display means that displays information that shows a lesion part that is detected by the detection means on an image in which the light intensity data is visualized; a contact region distinguishing means that, based on the light intensity data, with respect to the surface of the inner wall portion, distinguishes between an optical probe contact region which an optical probe that irradiates a measuring light on the inner wall portion contacts and an optical probe non-contact region which the optical probe does not contact at a time of the optical coherence tomography measurement; and a threshold value changing means that changes the threshold value in the detection means so as to set respectively different threshold values for the optical probe contact region and the optical probe non-contact region that are distinguished by the contact region distinguishing means.

According to the present invention, with respect to an inner wall portion within a living organism having a first layer (epithelial layer) of a predetermined thickness on a surface thereof, such as the biliary tract or the pancreatic duct, a region in which the thickness of the first layer is greater than a predetermined threshold value is automatically detected as a region which is suspected of being a lesion part such as a cancer, and is displayed. At such time, since the threshold value is changed so as to set respectively different threshold values for an optical probe contact region at which the optical probe (outer circumferential face) and the surface of the inner wall portion contact and an optical probe non-contact region at which the optical probe and the surface of the inner wall portion do not contact, a normal site and a lesion part are appropriately distinguished in a manner that takes into consideration the existence or non-existence of contact between the optical probe and the surface of the inner wall portion.

A diagnosis support apparatus according to a forty-second aspect of the present invention is in accordance with the invention according to the forty-first aspect, further including a probe detection means that detects a position of an outer circumferential face of the optical probe based on the light intensity data, wherein when a position of the outer circumferential face of the optical probe that is detected by the probe detection means and a position of the surface of the inner wall portion are in a range in which the positions are regarded as being at an identical position, the contact region distinguishing means determines that a region in question is an optical probe contact region, and in other cases the contact region distinguishing means determines that a region in question is an optical probe non-contact region.

The present invention specifically illustrates one form of a contact region distinguishing means that distinguishes between an optical probe contact region and an optical probe non-contact region.

A diagnosis support apparatus according to a forty-third aspect of the present invention is in accordance with the invention according to the forty-first or forty-second aspect, wherein, as the threshold value, the threshold value changing means sets a value for the optical probe contact region that is smaller than a value for the optical probe non-contact region.

According to the present invention, in consideration of the fact that a thickness of a first layer in an optical probe contact region is less than in an optical probe non-contact region due to a pressing effect of the optical probe, a value that is set as the threshold value to be compared with a thickness of the first layer is set to a smaller value for an optical probe contact region than for an optical probe non-contact region.

A diagnosis support apparatus according to a forty-fourth aspect of the present invention is in accordance with the invention according to the forty-first, forty-second, or forty-third aspect, wherein the detection means includes: a surface detection means that detects a surface of the first layer based on the light intensity data; a boundary detection means that detects a boundary between the first layer and a second layer that adjoins the first layer in a depth direction; a thickness calculation means that calculates a thickness of the first layer based on a difference in the depth direction between a surface that is detected by the surface detection means and a boundary that is detected by the boundary detection means; and a comparison means that compares a thickness of the first layer that is calculated by the thickness calculation means at a measurement point that is set on the surface of the inner wall portion and the threshold value.

The present invention specifically illustrates one form of the detection means, and specifically illustrates mainly a configuration for calculating a thickness of a first layer.

A diagnosis support apparatus according to a forty-fifth aspect of the present invention is in accordance with the invention according to any one of the forty-first to forty-fourth aspects, wherein the detection means detects a measurement point at which a thickness of the first layer is a value that is greater than the threshold value, and when a set of the measurement points form a continuous region of a predetermined size, the detection means detects the region as a lesion part.

When a region in which a thickness of a first layer is a value that is greater than a predetermined threshold value is limited to a minute range, the present invention determines that the region that does not correspond to a lesion part and excludes the region.

A diagnosis support apparatus according to a forty-sixth aspect of the present invention is in accordance with the invention according to any one of the forty-first to forty-fifth aspects, wherein the lesion part display means applies a predetermined color to a region of the lesion part on an image in which the light intensity data is visualized, and displays a resulting image.

According to the present invention, an observer can ascertain a detected region of a lesion part at a glance.

A diagnosis support apparatus according to a forty-seventh aspect of the present invention is in accordance with the invention according to any one of the forty-first to forty-sixth aspects, wherein, as an image in which the light intensity data is visualized, the lesion part display means displays at least any one image among a group including a three-dimensional image obtained by perspective projection processing, a three-dimensional image obtained by parallel projection processing, and a tomogram of a predetermined cross section.

The present invention shows that, since spatial structure data has three-dimensional structure information, images of various forms can be generated, and it is possible to display a three-dimensional image to appear as a solid object or a tomogram of only a specific cross section. A lesion part can be displayed in any of the images.

A diagnosis support apparatus according to a forty-eighth aspect of the present invention is in accordance with the invention according to any one of the forty-first to forty-seventh aspects, wherein the inner wall portion is an inner wall portion of a biliary tract, a pancreatic duct, a bronchial tube, a pharynx, an esophagus, a stomach, a colon, a uterus, or a urinary duct.

The present invention limits in-vivo sites at which the inventions according to the forty-first to forty-seventh aspects are particularly effective.

A diagnosis support apparatus according to a forty-ninth aspect of the present invention is in accordance with the invention according to any one of the forty-first to forty-eighth aspects, wherein the light intensity data acquisition means acquires light intensity data of a three-dimensional region of the inner wall portion.

The present invention is a preferred specific embodiment in which, preferably, the light intensity data acquisition means acquires light intensity data of a three-dimensional region of an inner wall portion.

A diagnosis support method according to a fiftieth aspect of the present invention includes: a light intensity data acquisition step of acquiring light intensity data of an inner wall portion within a living organism that has a first layer of at least a predetermined thickness on a surface thereof that is obtained by performing optical coherence tomography measurement with respect to the inner wall portion; a detection step of, based on light intensity data that is acquired by the light intensity data acquisition step, comparing a thickness of the first layer at a measurement point that is set on the surface of the inner wall portion and a predetermined threshold value, and detecting the measurement point as a lesion part when the thickness of the first layer is a value that is greater than the threshold value; a lesion part display step of displaying information that shows a lesion part that is detected by the detection step on an image in which the light intensity data is visualized; a contact region distinguishing step of, based on the light intensity data, with respect to the surface of the inner wall portion, distinguishing between an optical probe contact region which an optical probe that irradiates a measuring light on the inner wall portion contacts and an optical probe non-contact region which the optical probe does not contact at a time of the optical coherence tomography measurement; and a threshold value changing step of changing the threshold value in the detection step so as to set respectively different threshold values for the optical probe contact region and the optical probe non-contact region that are distinguished by the contact region distinguishing step.

According to the present invention, with respect to an inner wall portion within a living organism having a first layer (epithelial layer) of a predetermined thickness on a surface thereof, such as the biliary tract or the pancreatic duct, a region in which the thickness of the first layer is greater than a predetermined threshold value is automatically detected as a region which is suspected of being a lesion part such as a cancer, and is displayed. At such time, since the threshold value is changed so as to set respectively different threshold values for an optical probe contact region at which the optical probe (outer circumferential face) and the surface of the inner wall portion contact and an optical probe non-contact region at which the optical probe and the surface of the inner wall portion do not contact, a normal site and a lesion part are appropriately distinguished in a manner that takes into consideration the existence or non-existence of contact between the optical probe and the surface of the inner wall portion.

A lesion part detection apparatus according to a fifty-first aspect of the present invention includes: a light intensity data acquisition means that acquires light intensity data of an inner wall portion within a living organism that has a first layer of at least a predetermined thickness on a surface thereof that is obtained by performing optical coherence tomography measurement with respect to the inner wall portion; a detection means that, based on light intensity data that is acquired by the light intensity data acquisition means, compares a thickness of the first layer at a measurement point that is set on the surface of the inner wall portion and a predetermined threshold value, and detects the measurement point as a lesion part when the thickness of the first layer is a value that is greater than the threshold value; a contact region distinguishing means that, based on the light intensity data, with respect to the surface of the inner wall portion, distinguishes between an optical probe contact region which an optical probe that irradiates a measuring light on the inner wall portion contacts and an optical probe non-contact region which the optical probe does not contact at a time of the optical coherence tomography measurement; and a threshold value changing means that changes the threshold value in the detection means so as to set respectively different threshold values for the optical probe contact region and the optical probe non-contact region that are distinguished by the contact region distinguishing means.

According to the present invention, with respect to an inner wall portion within a living organism having a first layer (epithelial layer) of a predetermined thickness on a surface thereof, such as the biliary tract or the pancreatic duct, a region in which the thickness of the first layer is greater than a predetermined threshold value is automatically detected as a region which is suspected of being a lesion part such as a cancer. At such time, since the threshold value is changed so as to set respectively different threshold values for an optical probe contact region at which the optical probe (outer circumferential face) and the surface of the inner wall portion contact and an optical probe non-contact region at which the optical probe and the surface of the inner wall portion do not contact, a normal site and a lesion part are appropriately distinguished in a manner that takes into consideration the existence or non-existence of contact between the optical probe and the surface of the inner wall portion.

A lesion part detection method according to a fifty-second aspect of the present invention includes: a light intensity data acquisition step of acquiring light intensity data of an inner wall portion within a living organism that has a first layer of at least a predetermined thickness on a surface thereof that is obtained by performing optical coherence tomography measurement with respect to the inner wall portion; a detection step of, based on light intensity data that is acquired by the light intensity data acquisition step, comparing a thickness of the first layer at a measurement point that is set on the surface of the inner wall portion and a predetermined threshold value, and detecting the measurement point as a lesion part when the thickness of the first layer is a value that is greater than the threshold value; a contact region distinguishing step of, based on the light intensity data, with respect to the surface of the inner wall portion, distinguishing between an optical probe contact region which an optical probe that irradiates a measuring light on the inner wall portion contacts and an optical probe non-contact region which the optical probe does not contact at a time of the optical coherence tomography measurement; and a threshold value changing step of changing the threshold value in the detection step so as to set respectively different threshold values for the optical probe contact region and the optical probe non-contact region that are distinguished by the contact region distinguishing step.

According to the present invention, with respect to an inner wall portion within a living organism having a first layer (epithelial layer) of a predetermined thickness on a surface thereof, such as the biliary tract or the pancreatic duct, a region in which the thickness of the first layer is greater than a predetermined threshold value is automatically detected as a region which is suspected of being a lesion part such as a cancer. At such time, since the threshold value is changed so as to set respectively different threshold values for an optical probe contact region at which the optical probe (outer circumferential face) and the surface of the inner wall portion contact and an optical probe non-contact region at which the optical probe and the surface of the inner wall portion do not contact, a normal site and a lesion part are appropriately distinguished in a manner that takes into consideration the existence or non-existence of contact between the optical probe and the surface of the inner wall portion.

According to the present invention, in a case where a "region in which a papillary protrusion has arisen on a surface (outermost surface) of an inner wall portion" or a "region in which cells (epithelial cells) are randomly proliferating on a surface (outermost surface) of an inner wall portion" which is suspected of being a lesion part exists at an inner wall portion within a living organism that has a flat surface at a normal time, such as the biliary tract or the pancreatic duct, the region can be automatically detected and thus diagnosis can be supported.

According to the present invention, when a "region in which fibrosis is progressing" which is suspected of being a lesion part exists at an inner wall portion within a living organism, such as the biliary tract or the pancreatic duct, the region can be automatically detected and thus diagnosis can be supported.

According to the present invention, when a "region in which fibrosis is progressing" which is suspected of being a lesion part exists at an inner wall portion within a living organism that has a layered structure at a normal time, such as the biliary tract or the pancreatic duct, the region can be automatically detected and thus diagnosis can be supported.

According to the present invention, it is possible for an observer to easily ascertain a region which has a high possibility of being a lesion part, and thus a workload can be reduced.

According to the present invention, when automatically detecting a lesion part at which a thickness of an epithelial layer is greater than a predetermined threshold value based on tomographic information (light intensity data) acquired by performing OCT measurement with respect to an inner wall portion within a living organism that has an epithelial layer (first layer) of a predetermined thickness on the surface thereof such as, for example, the biliary tract, the pancreatic duct, a bronchial tube, the pharynx, the esophagus, the stomach, the colon, the uterus and the urinary duct, detection of a lesion part is appropriately performed by taking into consideration the existence or non-existence of contact between an optical probe and the surface of the inner wall portion at a time of measurement.

### BRIEF DESCRIPTION OF THE DRAWINGS

Fig. 1 is a configuration diagram that illustrates the overall configuration of an OCT apparatus;
Fig. 2 is a view that illustrates a medical treatment network to which the OCT apparatus is connected;
Fig. 3 is a cross-sectional view that shows a cross section including a long axis of an OCT probe;
Fig. 4 is an explanatory view that is used for describing operations when disposing the OCT probe in the biliary tract or the pancreatic duct;
Fig. 5 is an explanatory view that is used for describing operations when disposing the OCT probe in the biliary tract or the pancreatic duct;
Fig. 6 is a view that illustrates a form in which balloons are arranged on an OCT probe;
Fig. 7 is a view that illustrates a normal layered structure of an inner wall portion of the biliary tract or pancreatic duct;
Figs. 8A and 8B are explanatory views that are used for describing a lesion part detection processing type 1;
Figs. 9A, 9B, and 9C are explanatory views that are used for describing a lesion part detection processing type 2;
Figs. 10A, 10B, 10C, and 10D are explanatory views that are used for describing a lesion part detection processing type 3;
Figs. 11A and 11B are explanatory views that are used for describing a lesion part detection processing type 4;
Figs. 12A, 12B, and 12C are explanatory views that are used for describing a lesion part detection processing type 5;
Figs. 13A and 13B are explanatory views that are used for describing a lesion part detection processing type 6;
Figs. 14A, 14B, and 14C are explanatory views that are used for describing a lesion part detection processing type 7;
Figs. 15A and 15B are explanatory views that are used for describing a lesion part detection processing type 8;
Figs. 16A, 16B, and 16C are explanatory views that are used for describing a lesion part detection processing type 9;
Figs. 17A, 17B, and 17C are explanatory views that are used for describing a process of detecting a contact region and a non-contact region of a sheath;
Fig. 18 is a flowchart that illustrates processing procedures according to a first embodiment of a diagnosis support function;
Fig. 19 is a view that illustrates the configuration of a display screen that is displayed on a monitor according to the diagnosis support function;
Fig. 20 is a view that illustrates the configuration of a display screen that is displayed on a monitor according to the diagnosis support function
Fig. 21 is a flowchart that illustrates processing procedures according to a second embodiment of the diagnosis support function;
Figs. 22A and 22B are BC tomograms that are displayed according to the second embodiment of the diagnosis support function;
Figs. 23A and 23B are explanatory views that are used for describing a display mode that displays spatial structure data;
Figs. 24A and 24B are explanatory views that are used for describing an endoscope mode;
Figs. 25A and 25B are explanatory views that are used for describing a perspective mode;
Figs. 26A and 26B are explanatory views that are used for describing a long-axis cross section mode;
Figs. 27A, 27B, 27C and 27D are explanatory views that are used for describing a display mode;
Figs. 28A and 28B are explanatory views that are used for describing a display mode;
Fig. 29 is a flowchart that illustrates processing procedures of detection processing type 1;
Fig. 30 is a flowchart that illustrates processing procedures of detection processing type 2;
Fig. 31 is a flowchart that illustrates processing procedures of detection processing type 3;
Fig. 32 is a flowchart that illustrates processing procedures of detection processing type 4;
Fig. 33 is a flowchart that illustrates processing procedures of detection processing type 5;
Fig. 34 is a flowchart that illustrates processing procedures of detection processing type 6;
Fig. 35 is a flowchart that illustrates processing procedures of detection processing type 7;
Fig. 36 is a flowchart that illustrates processing procedures of detection processing type 8;
Fig. 37 is a flowchart that illustrates processing procedures of detection processing type 9; and
Fig. 38 is a flowchart that illustrates processing procedures when changing a threshold value of the detection processing type 2 in accordance with a contact region and a non-contact region of a sheath.

### DETAILED DESCRIPTION OF THE PREFERRED EMBODIMENTS

Preferred embodiments for implementing the diagnosis support apparatus according to the present invention are described hereunder.

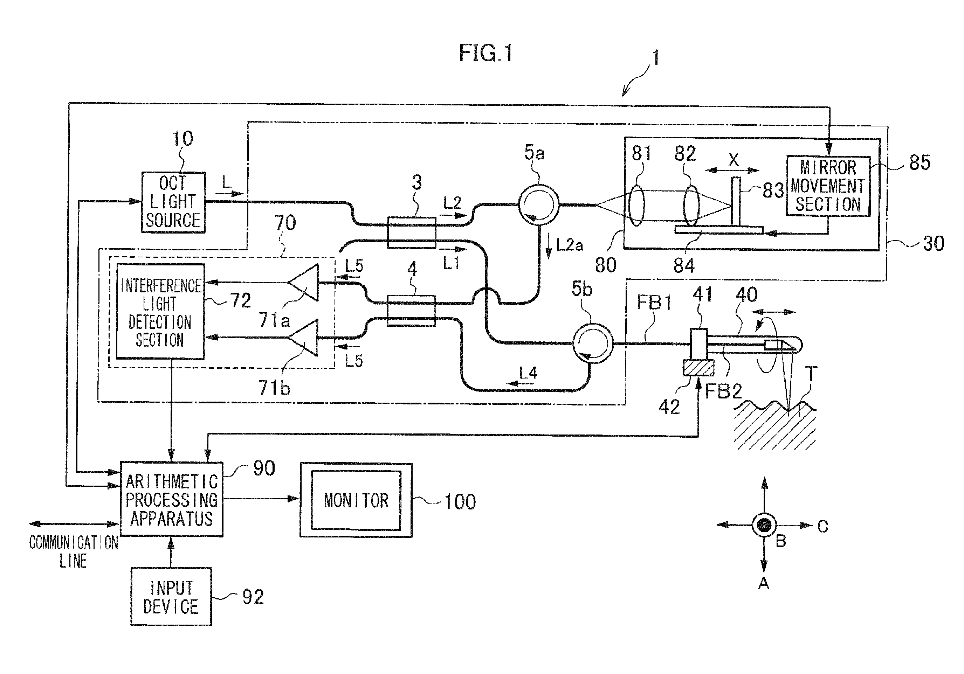

Fig. 1 is a block diagram that shows the configuration of an OCT apparatus used for measuring tomographic information of an inner wall portion of the biliary tract or the pancreatic duct of a measurement target according to the present embodiment. As shown in Fig. 1, an OCT apparatus 1 is configured to acquire tomographic information of a measurement target by SS-OCT measurement with wavelengths centered around, for example, 1.3 µm, and display an OCT image or the like by means of the acquired tomographic information, and includes an OCT light source 10, an OCT interferometer 30, an arithmetic processing apparatus (computer) 90, and a monitor 100.

The OCT light source 10 is a light source which emits a laser beam L in the infrared region while sweeping the frequency of the beam at fixed periods.

The laser beam L emitted from the OCT light source 10 is demultiplexed into a measuring light L1 and a reference light L2 by an optical demultiplexing section 3 inside the OCT interferometer 30. The optical demultiplexing section 3 includes, for example, an optical coupler with a splitting ratio of 90:10, and demultiplexes the laser beam L in a manner such that the ratio between a measuring light and a reference light is 90:10.

In the OCT interferometer 30, an optical path length adjustment section 80 adjusts, via a circulator 5a, an optical path length of the reference light L2 that is obtained through demultiplexing by the optical demultiplexing section 3 and reflects the reference light L2.

The optical path length adjustment section 80 is configured to change the optical path length of the reference light L2 in order to adjust a position where tomographic image acquisition is started, and includes collimator lenses 81 and 82 and a reflecting mirror 83. The reference light L2 from the circulator 5a passes through the collimator lenses 81 and 82, and is thereafter reflected by the reflecting mirror 83. A return light L2a of the reference light L2 enters the circulator 5a again through the collimator lenses 81 and 82.

The reflecting mirror 83 is arranged on a movable stage 84. The movable stage 84 is provided so as to be movable in a direction indicated by an arrow A by a mirror movement section 85. The optical path length of the reference light L2 is changed by movement of the movable stage 84 in the direction indicated by the arrow A. The return light L2a of the reference light L2 from the optical path length adjustment section 80 is guided to an optical multiplexing/demultiplexing section 4 through the circulator 5a.

The measuring light L1 obtained through the demultiplexing by the optical demultiplexing section 3 is guided to a light-emitting end at a distal end of an OCT probe 40 by an optical fiber FB2 via a circulator 5b, an optical fiber FB1, and an optical rotary connector section 41, and is emitted from the light-emitting end and irradiated on a measurement target T. The OCT probe 40 guides a back-scattered light L4 from the measurement target T that is generated when the measuring light L1 is irradiated on the measurement target T.

In the OCT probe 40, which is described in detail later, the optical fiber FB2 is insertedly arranged in a probe outer casing. The OCT probe 40 is configured so that the optical fiber FB2 that is located in the area ahead of the optical rotary connector section 41 rotates inside the probe outer casing by means of an unshown motor that is provided in an optical scanning section 42. The measuring light L1 that is emitted towards the measurement target T from the light-emitting end travels in a deflected direction (approximately perpendicular direction) with respect to the optical axis of the optical fiber FB2, and the configuration is such that, when the optical fiber FB2 rotates, the irradiation direction of the measuring light L1 that is irradiated from the light-emitting end also rotates around the long axis of the OCT probe 40.

According to the present embodiment, an irradiation direction of the measuring light L1 (the optical axis direction of the measuring light L1, which is a direction that is taken as the depth direction of the measurement target T) that is irradiated from the light-emitting end of the OCT probe 40 is referred to as the "A-axis direction" or "A axis", the long axis direction of the OCT probe 40 (optical axis direction of the optical fiber FB2) is referred to as the "C-axis direction" or "C axis", and a direction that is orthogonal to an AC plane including the A axis and the C axis is referred to as the "B-axis direction" or "B axis". Further, in the case of a configuration in which the irradiation direction of the measuring light L1 from the light-emitting end is caused to rotate around the long axis of the OCT probe 40, as in the configuration of the OCT probe 40 of the present embodiment, since the A-axis direction also rotates around the long axis, when the term "A-axis direction" is used, the term indicates an irradiation direction as viewed from the long axis of the OCT probe 40, and when the term "B-axis direction" is used, the term indicates a circumferential direction around the long axis of the OCT probe 40.

Thus, when the optical fiber FB2 is rotated as described above, since the measuring light L1 is scanned in the B-axis direction, tomographic information of an AB pane that includes the A axis and the B axis is acquired. In this connection, such scanning in the B-axis direction of the measuring light L1 is referred to herein as "B scanning,".

Further, the light-emitting end of the OCT probe 40 is configured to move backward and forward in the C-axis direction together with the optical fiber FB2 by means of an unshown motor provided inside the optical scanning section 42. Since the measuring light L1 is scanned in the C-axis direction thereby, tomographic information of the AC plane is acquired. In this connection, such scanning in the C-axis direction of the measuring light L1 is referred to herein as "C scanning".

Tomographic information of a three-dimensional region in a three-dimensional space including the A axis, the B axis, and the C axis is acquired by performing C scanning and B scanning simultaneously or alternately. In the present specification, tomographic information of a three-dimensional region is referred to as "spatial structure information" or "spatial structure data".

The back-scattered light L4 is guided to the OCT interferometer 30. In the OCT interferometer 30, the back-scattered light L4 is guided to the optical multiplexing/demultiplexing section 4 through the circulator 5b. In the optical multiplexing/demultiplexing section 4, the back-scattered light L4 of the measuring light L1 and the return light L2a of the reference light L2 are multiplexed, and resulting multiplexed lights are emitted toward an interference information detection section 70.

The interference information detection section 70 is configured to detect, at a predetermined sampling frequency, an interference light L5 generated from the return light L4 of the measuring light L1 and the return light L2a of the reference light L2 multiplexed by the optical multiplexing/demultiplexing section 4. The interference information detection section 70 includes InGaAs photodetectors 71a and 71b which each measure the light intensity of the interference light L5, and an interference light detection section 72 which performs balance detection with respect to a detected value from the InGaAs photodetector 71a and a detected value from the InGaAs photodetector 71b. Note that the interference light L5 is split into two light beams by the optical multiplexing/demultiplexing section 4, and that the two light beams are detected by the InGaAs photodetectors 71a and 71b and are outputted to the interference light detection section 72. The interference light detection section 72 performs a Fourier transform on the interference light L5 in synchrony with a sweep trigger signal S of the OCT light source 10, to thereby detect the intensity of the back-scattered light L4 at each position in the A-axis direction.

The arithmetic processing apparatus 90 is a component that has the same configuration as a main unit of a general-purpose computer, and includes a processor, a storage device and a communication interface and the like which are not shown in the drawings. An input device 92 such as an operation panel, a keyboard, or a mouse and a monitor 100 are connected to the arithmetic processing apparatus 90. Note that the arithmetic processing apparatus 90 may be integrally incorporated inside the casing of the main unit of the OCT apparatus 1 together with the above described OCT light source 10 and OCT interferometer 30, or may be a computer that is connected to the main unit of the OCT apparatus 1 by a cable or the like.

The arithmetic processing apparatus 90 has a control function that performs unified control of each portion of the OCT apparatus 1, such as the OCT light source 10, the mirror movement section 85, and the optical scanning section 42, a display (image generation) function that acquires information regarding the intensity of the back-scattered light L4 that is detected by the interference light detection section 72, generates an OCT image such as a tomogram of the measurement target T, and displays the generated OCT image on the monitor 100, and a diagnosis support function that supports diagnosis that is implemented in combination with the display function, and the like. Operations that are necessary when executing these functions are performed using the input device 92, and the necessary information is displayed on the monitor 100. Note that, processing such as a Fourier transform for detecting the intensity of the back-scattered light L4 in the interference light detection section 72 may be performed using the arithmetic processing apparatus 90.

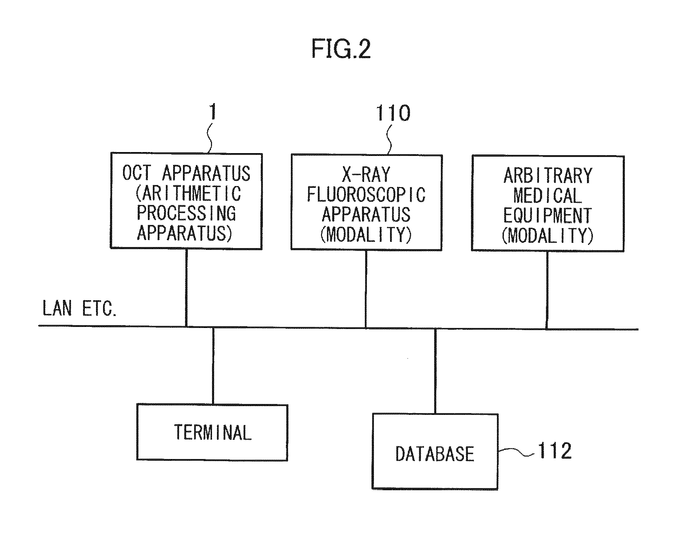

The arithmetic processing apparatus 90 is configured to be capable of performing communication with an X-ray fluoroscopic apparatus 110 or other arbitrary medical equipment (an endoscope, a CT apparatus, an MRI apparatus, an EUS apparatus, an IDUS apparatus, a THz apparatus or the like) that has a communication function via a communication line such as a LAN, as shown in Fig. 2. The arithmetic processing apparatus 90 is also connected to a database 112 via the communication line.

The database 112 is a filtering system that manages data of OCT images that are generated by the arithmetic processing apparatus 90. For example, the database 112 includes a large capacity storage apparatus that stores data and database software that manages the data stored in the large capacity storage apparatus. The arithmetic processing apparatus 90 can appropriately acquire data that is stored in the database 112, and can acquire data of OCT images or the like that were stored in the database in the past and data stored in the database by other medical equipment. An arbitrary terminal such as a personal computer can be connected to the database 112 through a communication line, data such as an OCT image that is stored in the database 112 can be read out with the terminal, and processing such as the diagnosis support function of the arithmetic processing apparatus 90 can also be executed by software processing of the terminal.

Note that, in addition to a filtering system for image data acquired with arbitrary medical equipment, for example, a filtering system for another purpose, such as for electronic clinical records, can be included in the database.

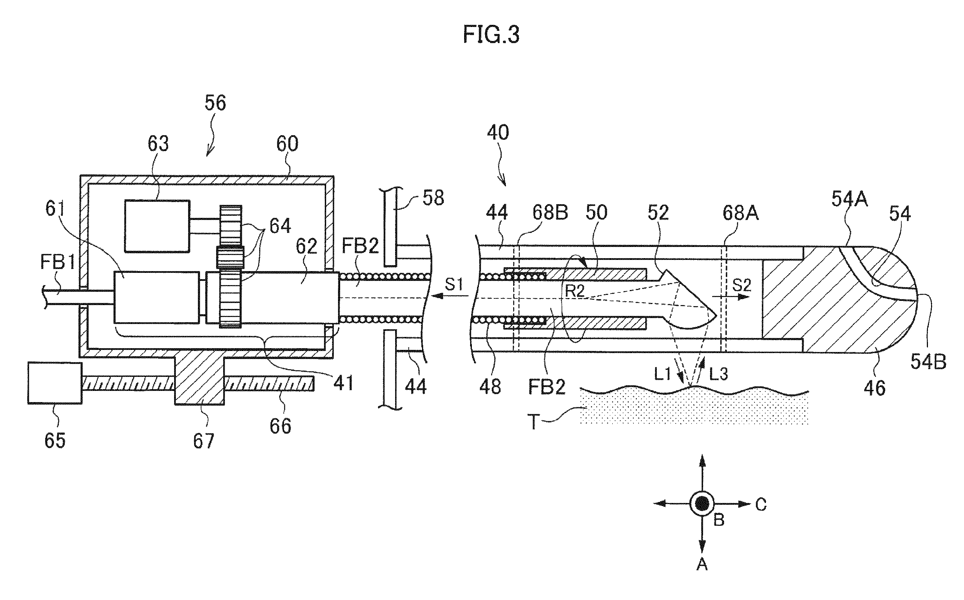

Next, the configuration of the OCT probe 40 in the OCT apparatus 1 that is used for measuring an inner wall portion of a biliary tract or a pancreatic duct as a measurement target according to the present embodiment is described using Fig. 3.

As shown in Fig. 3, a distal end portion of the OCT probe 40 has a probe outer casing (sheath) 44, a cap 46, an optical fiber FB2, a spring 48, a fixing member 50 and an optical lens 52.

The sheath 44 is a flexible cylindrical member that covers the entire OCT probe 40, and is made of a material that transmits the measuring light L1 and the back-scattered light L3.

The cap 46 is provided at the distal end of the sheath 44, and blocks the distal end of the sheath 44.

A guidewire hole 54 for inserting a guidewire is formed in the cap 46. The guidewire hole 54 has one opening 54A on a side face of the cap 46, and has another opening 54B on a front face of the cap 46. The guidewire is a wire that is disposed in advance at a measurement site, and is used to guide the OCT probe 40 to that position. By inserting the guidewire that is disposed at a measurement position into the guidewire hole 54 and advancing the OCT probe 40 forward, the OCT probe 40 can be moved as far as the measurement site while being guided by the guidewire. By guiding the OCT probe 40 using the guidewire in this manner, it is possible to easily dispose the OCT probe 40 in the bile duct or the pancreatic duct, which are locations to which it is difficult to directly advance the OCT probe 40.

As is also shown in Fig. 1, the optical fiber FB2 is insertedly arranged in the sheath 44, and is optically connected via an optical rotary connector section 41 to the optical fiber FB1 at a proximal end side of the OCT probe 40. The optical fiber FB2 guides the measuring light L1 that is irradiated from the optical fiber FB1 as far as the optical lens 52, and guides the back-scattered light L3 from the measurement target T that is acquired with the optical lens 52 to the optical fiber FB1.

The spring 48 is fixed to the outer circumference of the optical fiber FB2. As a result, a rotational force that is applied at the optical rotary connector section 41 on the proximal end side is transmitted as far as the distal end of the optical fiber FB2.

The optical lens 52 is fixed to the distal end side of the optical fiber FB2 by the fixing member 50, and is formed in a substantially hemispherical shape. The measuring light L1 emitted from the optical fiber FB2 is deflected in the A-axis direction by the optical lens 52 and converges at a predetermined position. The back-scattered light L3 from the measurement target T is converged by the optical lens 52, and the optical lens 52 causes the converged light to be incident on the optical fiber FB2.

Note that, a point at which the optical axis of the optical fiber FB2 and the optical axis of the measuring light L1 that is deflected by the optical lens 52 intersect corresponds to the light-emitting end of the OCT probe 40. Further, as described above, an irradiation direction of the measuring light L1 (the optical axis direction of the measuring light L1, which is a direction that is taken as the depth direction of the measurement target T) that is irradiated from the light-emitting end of the OCT probe 40 is defined as the "A-axis direction" or "A axis", the long axis direction of the OCT probe 40 (optical axis direction of the optical fiber FB2) is defined as the "C-axis direction" or "C axis", and a direction that is orthogonal to an AC plane including the A axis and the C axis is defined as the "B-axis direction" or "B axis". The irradiation direction of the measuring light L1 that is deflected by the optical lens 52 is the A-axis direction. According to the optical lens 52 of the present embodiment, since the measuring light L1 is deflected in a substantially orthogonal direction with respect to the C-axis direction, the A-axis direction is a direction that is orthogonal direction to the C-axis direction.

A drive apparatus 56 that contains the optical rotary connector section 41 and optical scanning section 42 shown in Fig. 1 is arranged on the proximal end side of the OCT probe 40.

In the drive apparatus 56, the above described optical rotary connector section 41 is arranged inside a frame 60 that is arranged inside a casing 58. The optical rotary connector section 41 includes an optical connector portion 61 that is fixed to the frame 60, and a rotary cylinder 62 that is rotationally supported with respect to the optical connector portion 61.

A light guiding hole is formed in the optical connector portion 61 and the rotary cylinder 62 so as to penetrate through both thereof, and a predetermined optical system is inserted in the light guiding hole. An end of the optical fiber FB1 that is shown in Fig. 1 is fixed to an open end on the optical connector portion 61 side of the light guiding hole. An end of the optical fiber FB2 of the OCT probe 40 is fixed to an open end on the rotary cylinder 62 side of the light guiding hole. Thus, the optical fiber FB1 and the optical fiber FB2 are optically connected, and the measuring light L1 emitted from the optical fiber FB 1 is incident on the optical fiber FB2, and the back-scattered light L3 emitted from the optical fiber FB2 is incident on the optical fiber FB 1.

Further, the spring 48 that is fixed to the outer circumference of the optical fiber FB2 is fixed to the rotary cylinder 62 together with the optical fiber FB2, and the sheath 44 is fixed to the casing 58. Accordingly, when the rotary cylinder 62 rotates with respect to the optical connector portion 61, the entire optical fiber FB2 rotates with respect to the optical fiber FB1 and rotates inside the sheath 44, and the optical lens 52 that is fixed to the distal end side of the optical fiber FB2 also rotates within the sheath 44.

A motor 63 that is a component of the optical scanning section 42 shown in Fig. 1 is fixed to the frame 60. The motor 63 is connected to the rotary cylinder 62 via a gear 64. Thus, the rotary cylinder 62 rotates by driving of the motor 63, and as described above, the optical fiber FB2 and the optical lens 52 rotate in the direction of an arrow R2 (B-axis direction) inside the sheath 44. Thus, the measuring light L1 irradiated from the optical lens 52 is scanned (B scanning) in the B-axis direction.

Furthermore, another motor 65 that is a component of the optical scanning section 42 shown in Fig. 1 is fixed to the casing 58. A ball screw for back and forth movement 66 rotates by means of the motor 65. The ball screw for back and forth movement 66 is screwed together with a screw-hole of a support member 67 that is provided in the frame 60. The frame 60 is configured so as to move forward or backward in the optical axis direction of the optical fiber FB2 when the ball screw for back and forth movement 66 rotates. Therefore, the optical fiber FB2 and the optical lens 52 move forward or backward in the direction of arrows S 1 and S2 (C-axis direction) inside the sheath 44 in accordance with driving of the motor 65. Thus, the measuring light L1 irradiated from the optical lens 52 is scanned (C scanning) in the C-axis direction.

The motors 63 and 65 are configured so as to drive in accordance with, for example, a drive signal that is received from the arithmetic processing apparatus 90 in accordance with an operation of the input device 92 shown in Fig. 1 by an operator or the like. Alternatively the motors 63 and 65 can be configured to be driven by an operation of an operation portion provided in the drive apparatus 56 so as to cause B scanning and C scanning of the measuring light L1 to be appropriately performed. Further, although not shown in the drawings, a position sensor that acquires information of a rotational angle of the optical fiber FB2 and information of a position in the C-axis direction of the optical fiber FB2 is provided in the drive apparatus 56. The information acquired by the position sensor is supplied to the arithmetic processing apparatus 90. Thus, the arithmetic processing apparatus 90 can perform control relating to a scanning position of B scanning or C scanning of the measuring light L1, and can ascertain a position in the B-axis direction and the C-axis direction when the intensity of the back-scattered light L4 in the A-axis direction that is acquired from the interference light detection section 72 is measured. However, a method of controlling the scanning position and a method of ascertaining a position in the B-axis direction and the C-axis direction is not limited to the above described method.

Markers (platinum marker) 68A and 68B that show the range of C scanning are adhered at two places on the outer circumferential face of the sheath 44. As described later, when performing OCT measurement of the biliary tract or the pancreatic duct, an X-ray fluoroscopic image is generated using an X-ray fluoroscopic apparatus. At that time, because the X-ray transmittance differs between the sheath 44 and the markers 68A and 68B (X-rays are transmitted through the sheath 44 but are not transmitted through the markers 68A and 68B), the positions of the markers 68A and 68B can be ascertained on the X-ray fluoroscopic image. Also, because the markers 68A and 68B are adhered at predetermined positions that are a start point and an end point of C scanning (in the drawing, the markers 68A and 68B are shown at positions that are separated by a space that is narrower than the actual space therebetween), the range of the C scanning can be ascertained by means of the X-ray fluoroscopic image using the positions of the markers 68A and 68B on the X-ray fluoroscopic image. In this connection, the markers 68A and 68B are adhered at positions that are slightly on the outer side of the start point and end point of the C scanning (outside the C scanning range) so as not to block the measuring light L1. Further, which of the markers 68A and 68B indicates the start point position and which marker indicates the end point position changes according to the orientation of the C scanning. The material of the markers 68A and 68B may be any material that has a different X-ray transmittance to the sheath 44.

Terms that are used in the description of the present embodiment will now be described. With respect to the A axis, the B axis, and the C axis that are defined above, a plane formed by the A axis and the B axis (plane orthogonal to the C axis when the A axis is orthogonal to the C axis) is referred to as an "AB plane", a plane formed by the A axis and the C axis is referred to as an "AC plane", and a plane formed by the B axis and the C axis is referred to as a "BC plane". Further, a cross section of a sectioning object that has been sectioned along the AB plane is referred to as an "AB cross section", a cross section of a sectioning object that has been sectioned along the AC plane is referred to as an "AC cross section", and a cross section of a sectioning object that is sectioned along the BC plane is referred to as a "BC cross section".

Furthermore, with respect to the arithmetic processing apparatus 90 shown in Fig. 1, a value of the intensity of the back-scattered light L4 obtained from the interference light detection section 72 or a value obtained by subjecting the aforementioned value to a predetermined conversion process is referred to as "scattered light intensity" or "scattered light intensity data" ("scattered light intensity data" is mainly used), data or information obtained by correlating respective pieces of scattered light intensity data with positions at which the respective pieces of scattered light intensity data are obtained (or positions corresponding thereto) are referred to as "tomographic information" or "tomographic data" ("tomographic data" is mainly used), tomographic data of an AB cross section is referred to as "AB tomographic data", tomographic data of an AC cross section is referred to as "AC tomographic data", and tomographic data of a BC cross section is referred to as "BC tomographic data". Further, for example, information obtained by correlating respective pieces of scattered light intensity data with respective positions of a three-dimensional region that is tomographic information of a three-dimensional region obtained by performing B scanning and C scanning simultaneously (so-called "spiral scanning") or alternately (performing B scanning while moving in steps of a fixed distance in the C-axis direction) or the like is referred to as "spatial structure information" or "spatial structure data" ("spatial structure data" is mainly used).

Furthermore, images of an AB cross section, an AC cross section, and a BC cross section that can be displayed on a monitor are referred to as an "AB tomogram", "AC tomogram", and "BC tomogram", respectively, and these are referred to by the general term "tomogram". Images that can be displayed on a monitor that are generated based on spatial structure data are referred to as "spatial structure images", and these images that can be displayed on a monitor that are obtained by measurement using the OCT apparatus 1 are referred to as "OCT images".

In this connection, in the OCT probe 40 of the embodiment described above, a configuration is adopted in which the measuring light L1 is scanned in the B-axis direction by rotating the light-emitting end that emits the measuring light L1 in the A-axis direction that is substantially orthogonal to the C-axis direction around the long axis (optical axis of the optical fiber FB2) of the OCT probe 40 in the C-axis direction, and the measuring light L1 is scanned in the C-axis direction by moving the light-emitting end in the C-axis direction. However, the present invention is not limited to this configuration. For example, a configuration may be adopted in which the measuring light L1 can be scanned in two different directions by changing at least one of the irradiation direction and irradiation position (position of the light-emitting end) of the measuring light L1 that is irradiated from the light-emitting end of the OCT probe 40, to thereby enable acquirement of spatial structure data of a three-dimensional region. In such case, the respective processes described hereunder can be applied in substantially the same manner by defining the irradiation direction of the measuring light L1 from the light-emitting end as the "A-axis direction" or "A axis", and defining one of the two directions in which the measuring light L1 is scanned as the "B-axis direction" or "B axis" and defining the other direction as the "C-axis direction" or "C axis".

Next, procedures when observing the biliary tract or the pancreatic duct using the above described OCT apparatus 1 are described.

First, in a room in which the X-ray fluoroscopic apparatus 110 that is capable of communicating with the OCT apparatus 1 in the manner shown in Fig. 2 is installed, a patient is placed on an imaging table of the X-ray fluoroscopic apparatus 110 so that X-ray fluoroscopy can be performed.

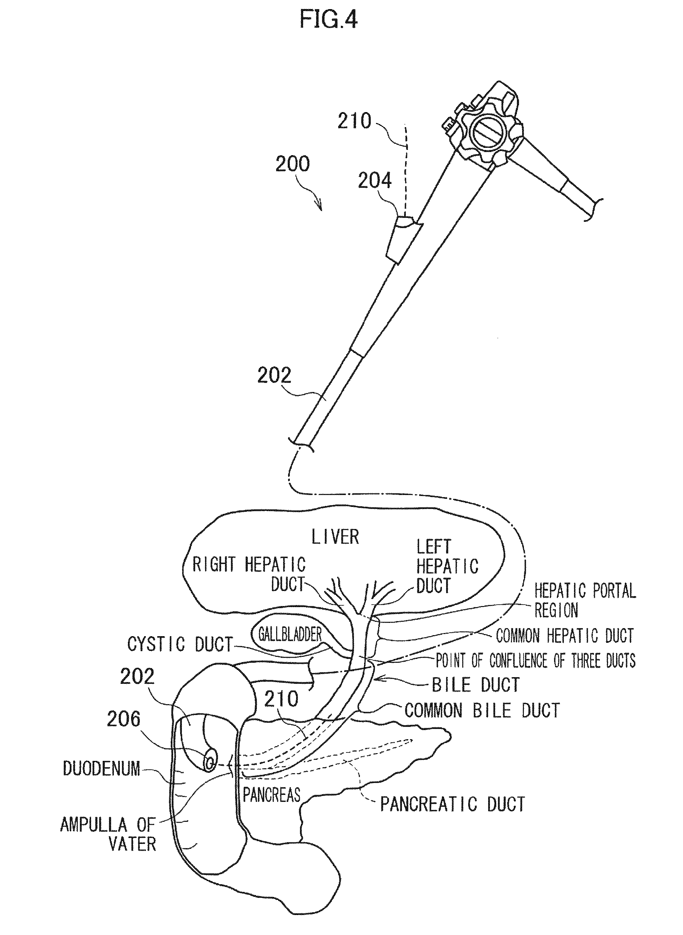

Subsequently, as illustrated in Fig. 4, an insertion portion 202 of an endoscope 200 is inserted from the mouth of the patient, which is not shown in the drawings, and a distal end of the insertion portion 202 is inserted via the esophagus and stomach as far as a position in the vicinity of the ampulla of Vater (duodenal papilla) in the duodenum.

Although a detailed description is omitted here, as is known, the endoscope 200 includes the insertion portion 202 that is inserted inside a body cavity, and a hand operation portion that is grasped by the operator and is provided with operation members for performing various operations and the like. A forceps channel through which a treatment instrument such as a forceps is inserted is provided inside the insertion portion 202. A forceps opening (forceps introduction opening) 204 through which a treatment instrument is inserted into the forceps channel is provided in the hand operation portion. A forceps opening (forceps lead-out opening) 206 through which the treatment instrument that is inserted from the forceps introduction opening 204 is lead out is provided in a distal end face of the insertion portion 202.

In Fig. 4, the pancreas is shown to one side of the duodenum, and the liver is shown above the pancreas. The gallbladder is shown below the liver.

As is known, bile that is produced by the liver is transported to the duodenum by the bile duct, and is delivered into the duodenum from an opening at the ampulla of Vater. The gallbladder is connected to the bile duct by the cystic duct, and accumulates bile.

Further, pancreatic juice produced by the pancreas is transported to the duodenum through the pancreatic duct in the pancreas. Smaller pancreatic ducts coalesce towards the duodenum to ultimately form two large ducts, the main pancreatic duct and the accessory pancreatic duct, which connect to the duodenum. The main pancreatic duct unites with the common bile duct inside the duodenal wall to form a short common duct, which opens in the duodenum at the ampulla of Vater.

A "bile duct" is a general term for a luminal structure that transports bile from the liver to the duodenum, and names are assigned thereto according to the relevant site. The bile ducts in the liver are called the intrahepatic bile ducts, while the bile ducts from outside the liver as far as the duodenum are called the extrahepatic bile ducts. The intrahepatic bile ducts branch into broadly two ducts, the left hepatic duct and the right hepatic duct. The extrahepatic bile ducts form a section from a hepatic portal region where the left hepatic duct and the right hepatic duct unite as far as the duodenum, and a section from the hepatic portal region to a point of confluence of three ducts at which the cystic duct merges with the aforementioned two ducts is called the common hepatic duct. A section from the point of confluence of the three ducts to the duodenum is called the common bile duct.

Further, a region that includes the gallbladder and the ampulla of Vater in addition to the bile ducts (intrahepatic bile ducts, extrahepatic bile ducts) is called the biliary tract. The present invention is applicable to all regions of the biliary tract as an object of diagnosis or the like.

When the distal end of the insertion portion 202 of the endoscope 200 is inserted as far as the vicinity of the ampulla of Vater in the duodenum as described above, a catheter for ERCP (endoscopic retrograde cholangiopancreatography) is inserted into the forceps channel from the forceps opening (forceps introduction opening) 204, is lead out from the forceps opening (forceps lead-out opening) 206, and inserted into the bile duct or pancreatic duct while confirming insertion using a X-ray fluoroscopic image. After contrast imaging, a guidewire 210 for guiding the OCT probe 40 shown in Fig. 3 to a desired position through the inside of the catheter is retained inside the bile duct or the pancreatic duct, and is disposed at a position which the operator desires to observe (measure) using the OCT apparatus 1 as shown in Fig. 4.

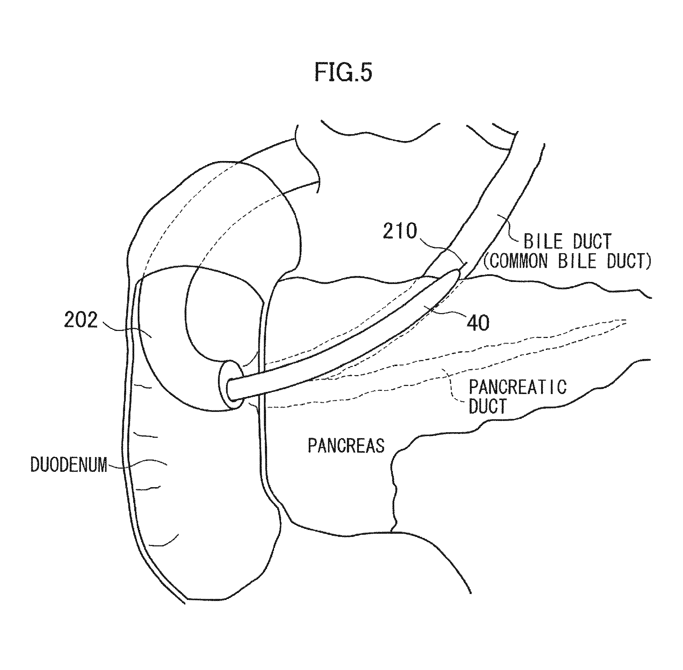

Next, the catheter is withdrawn in a state in which the guidewire is retained inside the bile duct or the pancreatic duct, and the guidewire 210 is passed through the guidewire hole 54 of the OCT probe 40 shown in Fig. 3 from the proximal end side. The OCT probe 40 is then inserted into the forceps channel from the forceps opening (forceps introduction opening) 204 and lead out from the forceps opening (forceps lead-out opening) 206 while moving along the guidewire 210, and is inserted into the biliary tract or the pancreatic duct from the opening at the ampulla of Vater along the guidewire 210 and disposed at the site to be observed (see Fig. 5).

When carrying out the above operations, by causing the arithmetic processing apparatus 90 of the OCT apparatus 1 to execute processing of a real time observation mode, the arithmetic processing apparatus 90 acquires AB tomographic data while repeatedly performing B scanning with the OCT probe 40, and displays a circular AB tomogram on the monitor 100 in real time. The observer moves the position of the OCT probe 40 while observing the AB tomogram that is being displayed in real time, and checks an outline of the center and a laterally spreading region of the lesion part. After deciding the position of the OCT probe 40, the observer uses the input device 92 to input an instruction to start C scanning measurement so as to perform C scanning in the appropriate region.

After the instruction to start C scanning measurement is inputted, before C scanning starts, a trigger signal is sent to the X-ray fluoroscopic apparatus 110 from the OCT apparatus 1 through the communication line. Upon receiving the trigger signal, the X-ray fluoroscopic apparatus 110 generates an X-ray fluoroscopic image of the region at the circumference of the OCT probe 40, and sends the X-ray fluoroscopic image to the arithmetic processing apparatus 90 through the communication line and also registers the X-ray fluoroscopic image in the database 112. As described above, markers 68A and 68B that indicate the start point and end point of C scanning are provided on the sheath 44 of the OCT probe 40, and since those markers 68A and 68B appear on the X-ray fluoroscopic image as the result of contrast imaging, based on the X-ray fluoroscopic image the observer can identify which region of the entire area of the biliary tract or the pancreatic duct has been taken as the range of C scanning (the measurement region).

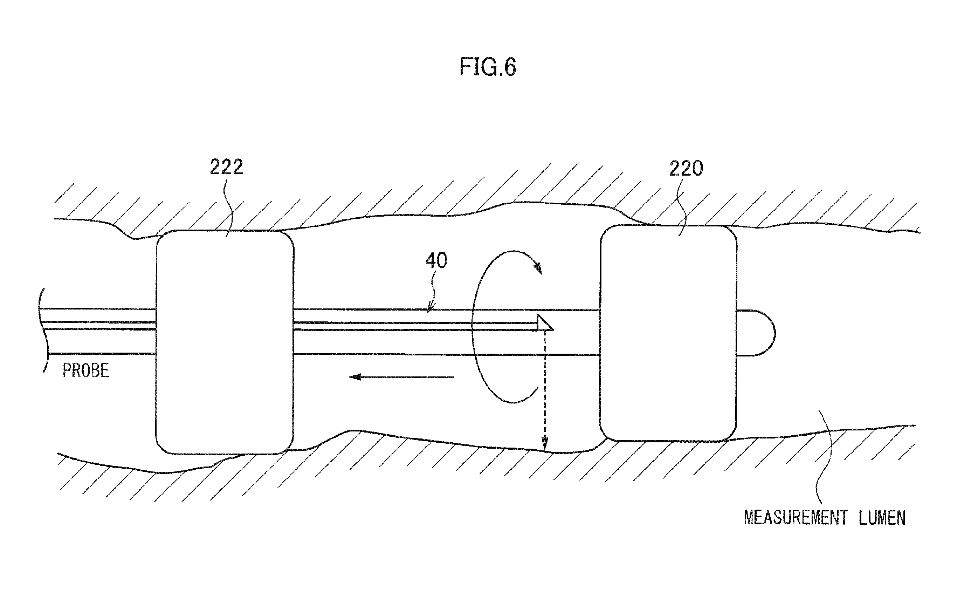

When generation and storage of the X-ray fluoroscopic image is completed, the OCT apparatus 1 moves the light-emitting end (optical lens 52) of the OCT probe 40 to the position that is the start point for the C scanning, and starts the C scanning. At this time, for example, B scanning and C scanning are performed simultaneously (so-called "spiral scanning") and, for example, spatial structure data of a range of a length of 100 mm is acquired. During C scanning, the observer maintains the endoscope 200 and the OCT probe 40 in a motionless state. A specific AC tomogram is displayed on the monitor screen in accordance with the C scanning together with an AB tomogram acquired during measurement. In this connection, as shown in Fig. 6, it is favorable to install balloons 220 and 222 that can expand and contract at, for example, two locations outside the C scanning range on the outer circumferential face of the sheath 44 of the OCT probe 40. Thus, after the OCT probe 40 is disposed at the measurement site, it is possible to expand the balloons 220 and 222 by injecting a fluid into the balloons 220 and 222, and thereby cause the balloons 220 and 222 to contact against the inner wall of the measurement site. Thus, the relative position between the OCT probe 40 and the inner wall of the measurement site can be fixed without being influenced by body motion.

When C scanning ends, a signal indicating that measurement has ended is emitted from the OCT apparatus 1 either visually by means of the monitor 100 or audibly by means of a speaker. After the optical lens 52 that is the light-emitting end of the OCT probe 40 has returned to the C scanning start position, the OCT apparatus 1 reverts to the real time observation mode, and B scanning is performed using the OCT probe 40, and an AB tomogram or AC tomogram acquired as a result is displayed on the monitor 100 in real time. When measuring a different location, the observer repeats the above described operations, and when ending observation the observer withdraws the OCT probe 40 from the forceps channel of the insertion portion 202 of the endoscope 200 and stops operation of the OCT apparatus 1.

In this connection, in addition to a trans-endoscopic observation method, a transdermal hepatic observation method is another method of inserting the OCT probe 40 into the biliary tract or pancreatic duct, and this method may also be used. Further, a method in which the OCT probe 40 is passed through the inside of a catheter and is inserted into the biliary tract or pancreatic duct from the ampulla of Vater part, that is not a method that uses a guidewire, may be used. A method in which a contrast medium is injected according to an ERCP (endoscopic retrograde cholangiopancreatography) technique, and a method that is used with insertion of an IDUS (intraductal ultrasonography) probe can also be used.

Further, although a case in which the X-ray fluoroscopic apparatus 110 is used together with the OCT apparatus 1 is described above, another fluoroscopic apparatus such as a CT, MRI, or THz imaging apparatus, and not the X-ray fluoroscopic apparatus 110, may also be used. A configuration may also be adopted in which an image taken with the relevant fluoroscopic apparatus is not displayed in real time on a monitor of the relevant fluoroscopic apparatus, but instead is incorporated into the OCT apparatus 1 through a communication line and is displayed in real time on the monitor 100 of the OCT apparatus 1 together with an OCT image.

Spatial structure data acquired by the OCT apparatus 1 in the above manner is subjected to image processing by the arithmetic processing apparatus 90 of the OCT apparatus 1 and visualized as a spatial structure image, and the spatial structure image is displayed on the monitor 100. The spatial structure image is configured so that it is possible to switch between display forms that differ according to various visualization techniques as described later, and display the relevant display form.

The arithmetic processing apparatus 90 of the OCT apparatus 1 is also with a diagnosis support function that automatically detects a lesion part (characteristic structure that is suspected of being a lesion part), and, as described above, is configured to be capable of automatically detecting a lesion part based on spatial structure data and displaying the lesion part on the monitor 100.

Further, spatial structure data acquired by the OCT apparatus 1 or a spatial structure image that is generated on the basis of the spatial structure data is associated with various kinds of additional information such as patient information and stored in the database 112 that is connected thereto by a communication line. The patient information is information that identifies the patient, such as the patient ID or patient name.

In this connection, after acquiring spatial structure data also, with regard to processing of the display function that displays a spatial structure image and the diagnosis support function that supports diagnosis at the arithmetic processing apparatus 90, this processing can also be executed at a terminal (a computer of an electronic clinical record system or the like) other than the OCT apparatus 1 that is connected to the database 112 through a communication line. More specifically, software (OCT display software) for executing the same processing as the arithmetic processing apparatus 90 of the OCT apparatus 1 can be executed on a terminal other than the arithmetic processing apparatus 90, and spatial structure data can be acquired from the database 112 to execute the processing of the arithmetic processing apparatus 90.

Next, the diagnosis support function of the OCT apparatus 1 (arithmetic processing apparatus 90) is described.

When spatial structure data of a predetermined site of the biliary tract or pancreatic duct is acquired, a spatial structure image or the like is displayed by means of the display function of the arithmetic processing apparatus 90 (described later). If a lesion part exists, the observer identifies the lesion part, and if the lesion part is to be resected, the observer determines a resecting range by deciding resection lines.

The arithmetic processing apparatus 90 is equipped with a diagnosis support function that automatically detects a lesion part (site suspected of being a lesion part), and displays information of the region of the lesion part together with the spatial structure image. The observer can appropriately cause the arithmetic processing apparatus 90 to execute the diagnosis support function by performing a predetermined operation on the input device 92, and refer to the information displayed by means of the diagnosis support function when identifying a lesion part or deciding resection lines.

In this connection, the display function that displays a spatial structure image or the like is implemented in the same manner for the diagnosis support function, and it is assumed that the function is performed in the description of the diagnosis support function.

First, prior to describing the diagnosis support function, the structure of an inner wall portion of the biliary tract or pancreatic duct and morphological changes in a case where a lesion such as a cancer arises are described.

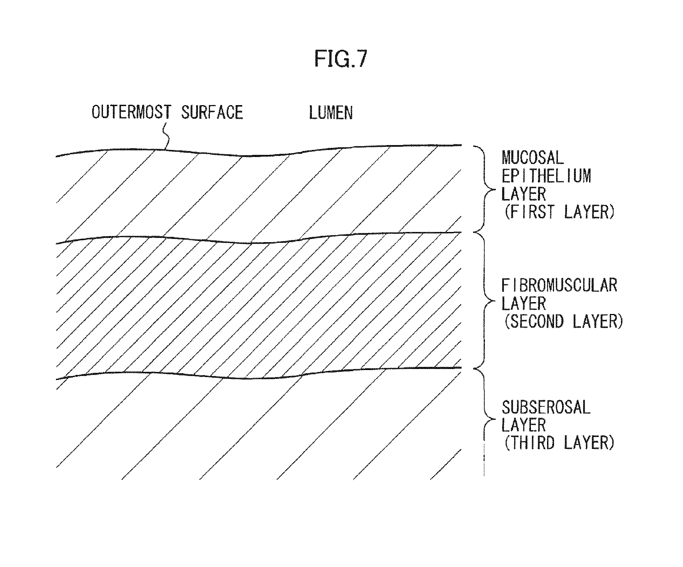

As shown in Fig. 7, the structure of an inner wall portion in the biliary tract is formed by a mucosal epithelial layer, a fibromuscular layer, and a subserosal layer that have different tissue structures, in that order from the surface on the luminal side (surface of inner wall portion) to the outside. The pancreatic duct is formed by a mucosal epithelium, fibrous connective tissue, and pancreatic acinar tissue (actual pancreas) in that order from the luminal side. According to the present embodiment, the respective layers are referred to as "first layer", "second layer", and "third layer" in order from the luminal side to the outside, and the inner wall portion surface (surface of the first layer) is referred to as the outermost surface (or epithelial cells). In the spatial structure data obtained by the above described OCT measurement, if the site is a normal site (normal tissue), the strongest scattered light intensity data is obtained at the second layer, the next strongest scattered light intensity data is obtained at the first layer, and the weakest scattered light intensity data is obtained at the third layer.

When a cancer or a lesion part that it is necessary to resect such as a precancerous lesion which is highly likely to turn into a cancer in the future exists at an inner wall portion in the biliary tract or pancreatic duct, morphological changes such as the following lesion types 1 to 6 are pathologically known with respect to the normal site shown in Fig. 7.
1. A papillary protrusion can be formed on an outermost surface.
2. The first layer thickens.
3. Fibrosis progresses.
4. Cells (epithelial cells) proliferate randomly on the outermost surface.
5. A ductal carcinoma occurs.
6. The form of a blood vessel changes, or a new blood vessel can be formed.

By executing the diagnosis support function, the arithmetic processing apparatus 90 detects characteristic structures (forms) that the lesion parts of the above described lesion types 1 to 6 show from among the spatial structure data, and extracts such characteristic structures as feature regions. The following nine types of detection processing, described as detection processing types 1 to 9, are conceivable as processing methods to detect such characteristic structures. The lesion types that can be detected differ according to the respective detection processing types. The detection processing types 1 to 9 described below can detect the following lesion part types, respectively.
Detection processing type 1: lesion types 1 to 3
Detection processing type 2: lesion types 1, 2, and 4
Detection processing type 3: lesion types 1 and 4
Detection processing type 4: lesion type 1
Detection processing type 5: lesion type 1
Detection processing type 6: lesion type 5
Detection processing type 7: lesion type 3
Detection processing type 8: lesion type 3
Detection processing type 9: lesion type 6

First, processing that the arithmetic processing apparatus 90 executes as pre-processing when performing processing for any of the detection processing types 1 to 9 is described. The pre-processing includes noise removal processing that removes noise from spatial structure data that is acquired by OCR measurement. The noise removal processing is carried out, for example, by blur processing.

In this case, the spatial structure data has a structure such that, when three-dimensional space is represented by a voxel space that is divided into voxels as the smallest units, scattered light intensity data at each position of a measurement region corresponding to the positions of the respective voxels is allocated as the voxel value of the respective voxels. A voxel region to which scattered light intensity data is allocated has a substantially cylindrical three-dimensional shape that is similar to the measurement region, and an A axis (A-axis direction), a B axis (B-axis direction), and a C axis (C-axis direction) are similarly defined with respect to the voxel space also.

In the blur processing, each of all of the voxels to which spatial structure data has been allocated is sequentially set as the voxel of interest, and a mean value of scattered light intensity data of voxels within a predetermined region that is centered on the voxel of interest (for example, 9+9+9 = 27 voxels that are situated next to the voxel of interest) is converted as the scattered light intensity data of the voxel of interest. Thus, noise is removed from the spatial structure data.

Subsequently, sheath elimination processing is performed that eliminates scattered light intensity data at the sheath 44 of the OCT probe 40 from the spatial structure data. In the sheath elimination processing, first, when the term "A-axis direction scattered light intensity signals" is taken to refer to signals that exhibit a value of scattered light intensity data at positions (respective positions in the A-axis direction) that a line in the A-axis direction passes through, then differential signals obtained by differentiating A-axis direction scattered light intensity signals are generated with respect to each position of the BC plane.

In this connection, when a line in the A-axis direction is assumed, a large number of such lines exist at different positions in the B-axis direction and the C-axis direction, and when focusing attention on the A-axis direction, a time that attention is focused on all the lines is expressed as "with respect to each position of the BC plane" or the like.

Next, a position in the A-axis direction at which a differential signal exhibits a maximum value is detected with respect to each position of the BC plane. A position at which a differential signal exhibits a maximum value indicates a position of an inner wall face of the sheath 44, and a range from the position of the maximum value to a position that is separated therefrom on the outer side in the A-axis direction by a known sheath thickness amount is a range of the scattered light intensity data at the sheath 44. Subsequently, the scattered light intensity data in that range is converted to a value that is reduced to a noise level. Thus, the scattered light intensity data of the sheath 44 is eliminated from the spatial structure data.

After the above described pre-processing has been executed, the following detection processing according to the respective detection processing types 1 to 9 is executed by the arithmetic processing apparatus 90.

### 1. Detection processing type 1

When a site at which a scattered light intensity of the first layer is outside a range of predetermined values exists at an inner wall portion (inner wall portion of a living organism) of a biliary tract or a pancreatic duct for which spatial structure data has been acquired, the processing of detection processing type 1 detects the site as a lesion part (feature region) that has a characteristic structure.

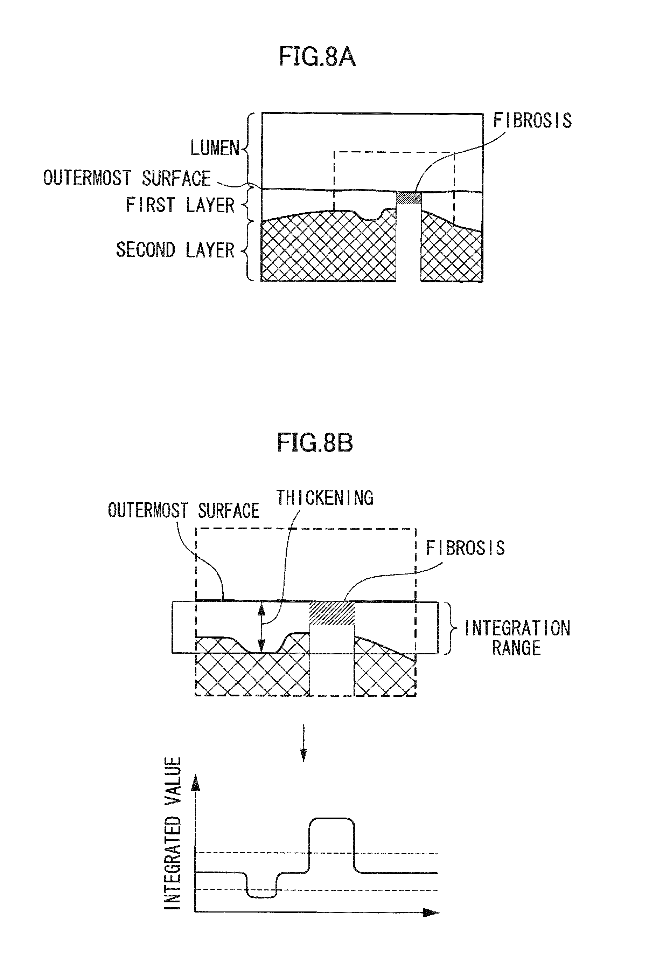

Fig. 8A is a view that, by means of an AC tomogram through the lesion parts, illustrates an example of spatial structure data of an inner wall of a living organism that has lesion parts of lesion types 2 and 3 among the lesion types 1 to 3 that are detected by detection processing type 1. In Fig. 8A, a site is illustrated at which fibrosis has progressed in the first layer. Fig. 8B shows one part of Fig. 8A in an enlarged manner. In Fig. 8B, a site is illustrated at which the first layer is thicker than in the surrounding area. These sites correspond to lesion parts of lesion types 2 and 3.

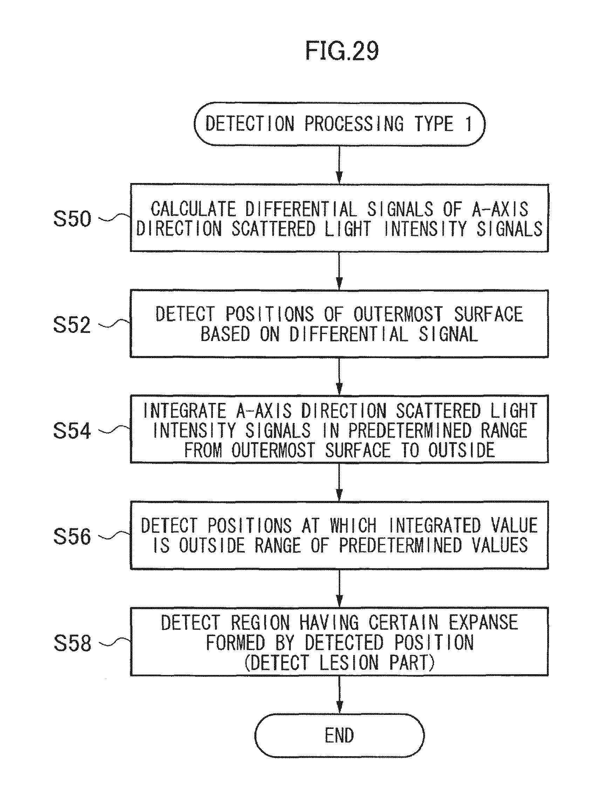

In order to detect lesion parts as feature regions in this manner, processing is performed by the following procedure according to detection processing type 1. Refer to the flowchart shown in Fig. 29.

First, similarly to when performing sheath elimination processing in the pre-processing, differential signals obtained by differentiating A-axis direction scattered light intensity signals are determined with respect to each position of the BC plane (step S50).

Next, positions in the A-axis direction at which the determined differential signals exhibit a maximum value are detected with respect to each position of the BC plane. A position at which a differential signal exhibits a maximum value is a position of a surface of the first layer of the inner wall portion of the living organism, that is, the position of the outermost surface. Thus, a position in the A-axis direction of the outermost surface of the inner wall portion of the living organism is detected with respect to each position of the BC plane as shown in Fig. 8A (step S52).

Note that, with respect to the order of performing the above processing for each position of the BC plane, a method in which a position in the C-axis direction is fixed and a position in the B-axis direction is sequentially changed and the above processing is performed for all positions in the B-axis direction, and thereafter the position in the C-axis direction is shifted and the same processing is repeated, and a method in which processing is performed in the reverse manner thereto are conceivable, and any order may be adopted.

When a position on the outermost surface of the inner wall portion of the living organism is detected, next, with respect to each position of the BC plane, as shown in Fig. 8B, A-axis direction scattered light intensity signals (scattered light intensity data) in a range of a predetermined length (for example, a length range of 100 µm that is a range that includes the second layer) on the outer side in the A-axis direction from the position on the outermost surface are integrated (step S54). As a result, integrated values of scattered light intensities in a range from the outermost surface of the inner wall portion of the living organism to a predetermined depth in the tissue are obtained. Fig. 8C illustrates an example of the distribution of integrated values obtained in this manner.

Next, positions at which an integrated value is outside a range of predetermined values are detected (step S56). More specifically, a position at which an integrated value is greater than a predetermined upper limit value and a position at which an integrated value is less than a predetermined lower limit value are detected. In a case in which a papillary protrusion has arisen on the outermost surface of the inner wall portion or in which the first layer has thickened, as in the case of lesion types 1 and 2, an integrated value is small, while in a case in which fibrosis has progressed, as in the case of lesion type 3, an integrated value is large. Therefore, by setting the threshold values (upper limit value and lower limit value) to appropriate values, positions at which there is a possibility a lesion part exists are detected by this detection process.

Subsequently, detection is performed to identify a region (continuous region that is equal to or greater than a predetermined size) that has a certain three-dimensional expanse that is formed by positions at which it is detected that an integrated value is outside the range of predetermined values is performed. If such a region is detected, finally it is determined that the region in question is a lesion part (region of a lesion part) (step S58).

Processing of detection processing type 1 is performed by the above described processing procedure, and together with detection of a lesion part corresponding to any of lesion types 1 to 3 being performed, a region of a lesion part that is detected is extracted as a feature region to be displayed at a time of displaying lesion parts that is described later.

In this connection, as detection processing that resembles detection processing type 1, processing may also be performed in which, without integrating A-axis direction scattered light intensity signals in a range of a predetermined length on the outer side in the A-axis direction from a position on the outermost surface as described above, positions are detected at which the scattered light intensity data exhibits a larger value than a predetermined threshold value within that range, and when detected positions form a region having a certain three-dimensional expanse, the region is detected as a region of a lesion part, and is extracted as a feature region.

### 2. Detection processing type 2

When a site at which a thickness of the first layer exceeds a predetermined threshold value exists at an inner wall portion (inner wall portion of a living organism) of a biliary tract or a pancreatic duct for which spatial structure data has been acquired, the processing of detection processing type 2 detects the site as a lesion part (feature region) that has a characteristic structure.

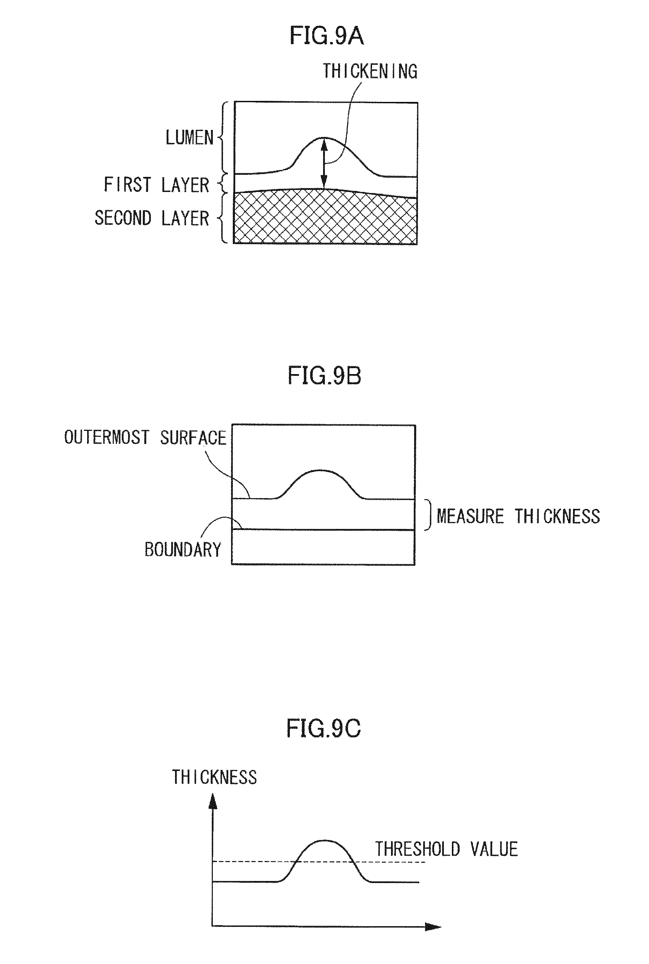

Fig. 9A is a view that, by means of an AC tomogram through the lesion part, illustrates an example of spatial structure data of an inner wall of a living organism that has a lesion part of lesion type 2 among the lesion types 1, 2, and 4 that are detected by detection processing type 2. In the figure, a site is illustrated at which the first layer is thickened, and this site corresponds to a lesion part of lesion type 2.

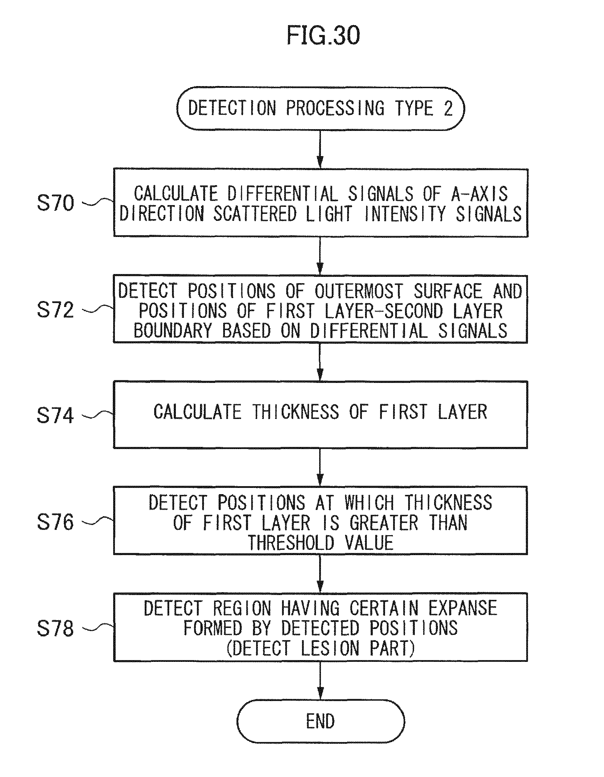

In order to detect such a lesion part, processing is performed by the following procedure according to detection processing type 2. Refer to the flowchart shown in Fig. 30.

First, similarly to when performing sheath elimination processing in the pre-processing, differential signals obtained by differentiating A-axis direction scattered light intensity signals are determined with respect to each position of the BC plane (step S70).

Next, with respect to each position of the BC plane, based on the differential signal, as shown in Fig. 9B, a position on an outermost surface of the inner wall portion of the living organism and a position at a boundary between the first layer and the second layer are detected. For example, because there is a large change in A-axis direction scattered light intensity signals between a position on the outermost surface of an inner wall portion of a living organism and a position at a boundary between the first layer and the second layer, relatively large local maximum values are shown in the differential signals. Hence, with respect to the differential signals, a position at which there is a large local maximum value that is present on an innermost side in the A-axis direction and a position at which there is a large local maximum value that is present on an outermost side are detected as the corresponding positions (step S72).

Next, a distance between the position on the outermost surface of the inner wall portion of the living organism and the position at the boundary between the first layer and the second layer is determined. Thus, a thickness of the first layer at each position of the BC plane is obtained (step S74).

Next, as shown in Fig. 9C, a position on the BC plane at which the thickness of the first layer is a value that is greater than a predetermined threshold value is detected (step S76). Thus, a position of a thickened lesion part, such as a lesion part according to lesion type 2, is detected. Further, since the thickness of the first layer increases in lesion parts according to lesion types 1 and 4 also, positions of those lesion parts are also detected.

Subsequently, detection is performed to identify a region (continuous region that is equal to or greater than a predetermined size) that has a certain three-dimensional expanse that is formed by positions at which the thickness of the first layer is greater than a predetermined threshold value. If such a region is detected, finally it is determined that the region in question is a lesion part (region of a lesion part) (step S78).

Processing of detection processing type 2 is performed by the above described processing procedure, and together with detection of a lesion part corresponding to any of lesion types 1, 2 and 4 being performed, a region of a detected lesion part is extracted as a feature region to be displayed at a time of displaying lesion parts that is described later.

In this connection, as detection processing that resembles detection processing type 2, processing may also be performed in which positions which exhibit scattered light intensity data of a certain fixed range are detected, and when detected positions form a region having a certain three-dimensional expanse, the region is detected as a region of a lesion part, and is extracted as a feature region.

### 3. Detection processing type 3

When a site at which a surface roughness of an outermost surface exceeds a predetermined threshold value exists at an inner wall portion (inner wall portion of a living organism) of a biliary tract or a pancreatic duct for which spatial structure data has been acquired, the processing of detection processing type 3 detects the site as a lesion part (feature region) that has a characteristic structure.

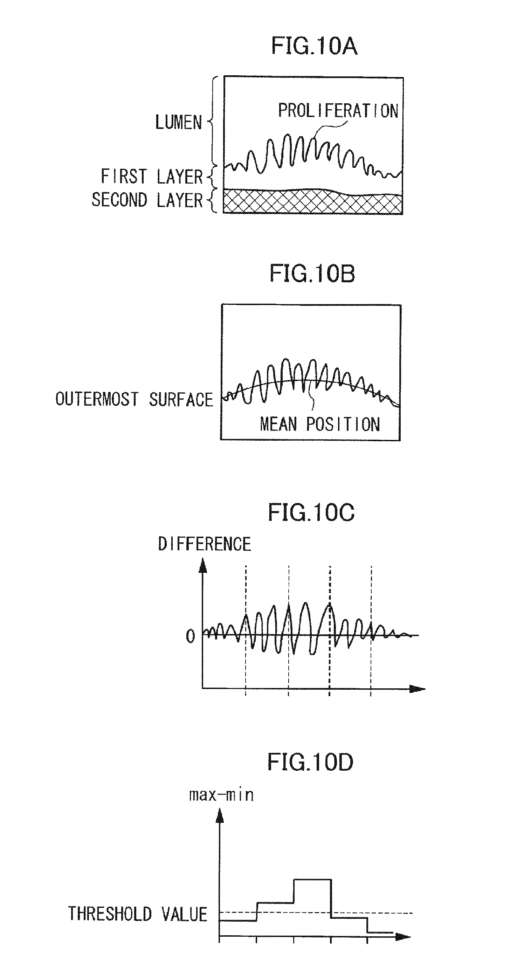

Fig. 10A is a view that, by means of an AC tomogram through the lesion part, illustrates an example of spatial structure data of an inner wall of a living organism that has a lesion part of lesion type 4 among the lesion types 1 and 4 that are detected by detection processing type 3. In the figure, a lesion part of lesion type 4 in which cells randomly proliferate on the outermost surface is illustrated.

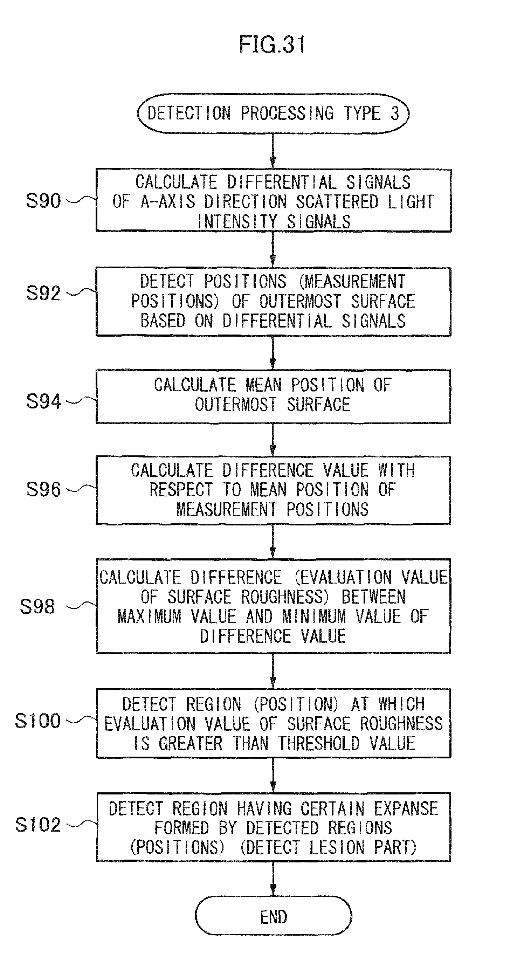

In order to detect such a lesion part, processing is performed by the following procedure according to detection processing type 3. Refer to the flowchart shown in Fig. 31.

First, similarly to when performing sheath elimination processing in the pre-processing, differential signals obtained by differentiating A-axis direction scattered light intensity signals are determined with respect to each position of the BC plane (step S90).

Next, positions in the A-axis direction at which the determined differential signals exhibit a maximum value are detected with respect to each position of the BC plane and, as shown in Fig. 10B, a position in the A-axis direction of the outermost surface of the inner wall portion of the living organism is detected with respect to each position of the BC plane (step S92). In this case, a detected position is referred to as a "measurement position".

Note that, with respect to the order of performing the above processing for each position of the BC plane, a method in which a position in the C-axis direction is fixed and a position in the B-axis direction is sequentially changed and the above processing is performed for all positions in the B-axis direction, and thereafter the position in the C-axis direction is shifted and the same processing is repeated, and a method in which processing is performed in the reverse manner thereto are conceivable, and any order may be adopted.

Next, as shown in Fig. 10B, mean positions in the A-axis direction of the outermost surface are calculated (step S94). More specifically, when a certain position on the BC plane is taken as a position of interest, a calculation region of a predetermined size is set on the BC plane that is centered on the position of interest. A mean value of measurement positions (coordinate values) in the A-axis direction on the outermost surface in that calculation region is then calculated. The mean value calculated in this manner is obtained as a mean position in the A-axis direction on the outermost surface at the position of interest on the BC plane. This processing is repeated while sequentially changing the position of interest on the BC plane, to thereby calculate mean positions in the A-axis direction on the outermost surface over the entire range of the BC plane.

Next, for each position of the BC plane, a difference (difference value) is calculated with respect to a mean position of the measurement positions on the outermost surface (step S96). At this time, if the coordinate values of the measurement positions on the outermost surface are shifted so that, with respect to each position of the BC plane, the coordinate value in the A-axis direction of the mean position is 0 (a fixed position), as shown in Fig. 10C, values of a curve showing the measurement positions on the outermost surface show the difference values.

Subsequently, as shown in Fig. 10C, the BC plane is divided into regions of a predetermined size (the B-axis direction and C-axis direction are divided in predetermined length units), and a difference between a maximum value and a minimum value of the difference values is determined for each region (step S98). The obtained difference is then set as a value (evaluation value) that shows the surface roughness of the outermost surface in each region as shown in Fig. 10D.

Next, regions (positions) in which the surface roughness of the outermost surface in the respective regions exhibits a value that is greater than a predetermined threshold value are detected (step S 100). As a result, a position of a lesion part in which cells randomly proliferate on the outermost surface, as in the case of lesion type 4, is detected. Further, since the surface roughness also increases when a papillary protrusion has arisen on the outermost surface, as in the case of lesion type 1, positions of lesion parts of lesion type 1 are also detected.

Subsequently, detection is performed to identify a region (continuous region that is equal to or greater than a predetermined size) that has a certain expanse that is formed by detected region (position). If such a region is detected, finally it is determined that the region in question is a lesion part (region of a lesion part) (step S102).

Processing of detection processing type 3 is performed by the above described processing procedure, and together with detection of a lesion part corresponding to either lesion type 1 or 4 being performed, a region of a detected lesion part is extracted as a feature region to be displayed at a time of displaying lesion parts that is described later.

In this connection, a method may also be adopted in which, instead of dividing the BC plane into regions of a predetermined size as in the foregoing step S98, equidistant lines are set in either one of the B direction and C direction, a difference between a maximum value and a minimum value of difference values (difference values with respect to the mean positions of the measurement positions of the outermost surface) is determined for each region of a predetermined length on each line, and a difference determined in this manner is taken as a value of the surface roughness in the relevant region. Further, a method of determining a value of surface roughness is not limited to the methods described above. For example, a method may also be adopted in which, after determining difference values from a mean value in a region of a predetermined size as illustrated in Fig. 10C, a mean value of absolute deviations from a mean line within a predetermined region is taken as an evaluation value. Alternatively, any method may be used as long as the method determines a value that shows the roughness of a surface, such as a method that calculates an evaluation value based on a squared value of a deviation, a method that takes a maximum value or a minimum value as an evaluation value, a method that determines an evaluation value based on a standard deviation or variance of a difference value, or a method that takes a number of peaks as an evaluation value. In order to detect random cell proliferation of lesion type 4, since the diameter of a single cell is approximately 10 µm, it is desirable that the size of a predetermined region be such that at least one side thereof is 40 µm or more. On the other hand, in order to detect a papillary protrusion of lesion type 1, it is desirable that at least one side of a predetermined region is 120 µm or more to detect a protrusion of approximately 30 µm. Thus, the size of a predetermined region may be altered to perform detection of different lesion types.

The processing of detection processing type 3 is not limited to detection of a lesion part in the biliary tract or pancreatic duct, and the processing is also effective for detecting a lesion part in other organs that have a flat epithelial structure at a normal time similarly to the biliary tract and pancreatic duct, such as, for example, the bronchial tubes, the pharynx, the esophagus, the stomach, the colon, the esophagus, and the urinary duct. When performing diagnostic support with respect to these sites, diagnosis can be supported by using detection processing type 3 as processing to detect a lesion part (feature region), and using a method similar to the present embodiment to display a detected lesion part.

### 4. Detection processing type 4

When a site that has a substantially globular tissue formation in the vicinity of an outermost surface exists at an inner wall portion (inner wall portion of a living organism) of a biliary tract or a pancreatic duct for which spatial structure data has been acquired, the processing of detection processing type 4 detects the site as a lesion part (feature region) that has a characteristic structure.

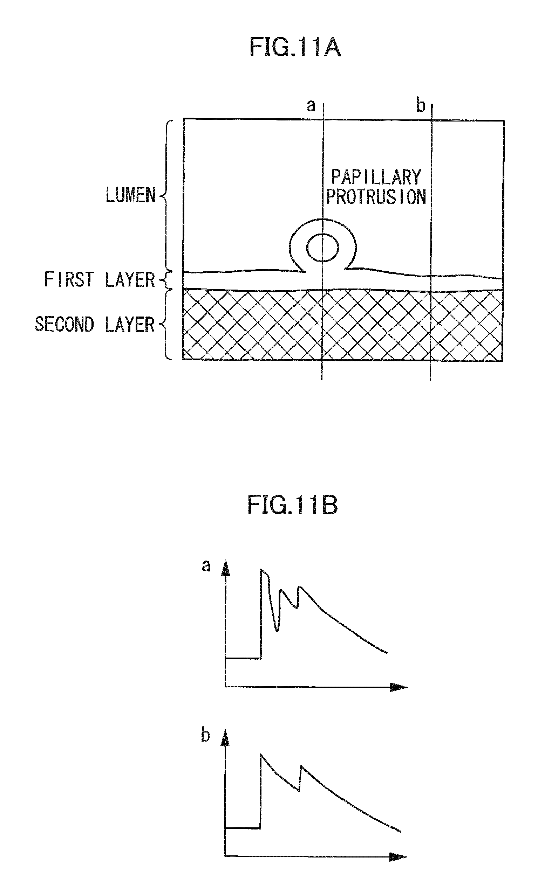

Fig. 11A is a view that, by means of an AC tomogram through the lesion part, illustrates an example of spatial structure data of an inner wall of a living organism that has a lesion part of lesion type 1 that is detected by detection processing type 4. In the figure, a lesion part of lesion type 1 in which a papillary protrusion has arisen on an outermost surface is illustrated.

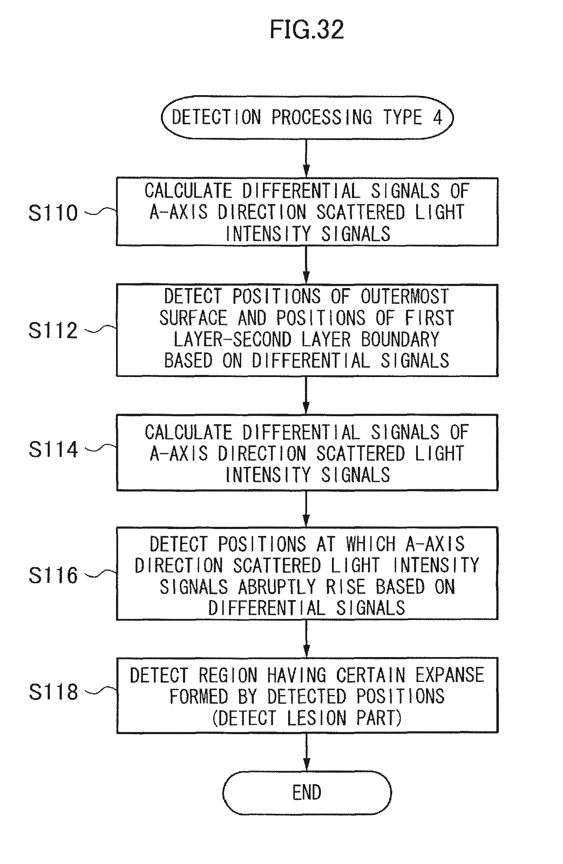

In order to detect such a lesion part, processing is performed by the following procedure according to detection processing type 4. Refer to the flowchart shown in Fig. 32.

First, by the same processing as that of detection processing type 2, detection of positions of an outermost surface of the inner wall portion of the living organism and positions at a boundary between the first layer and the second layer is performed (steps S110 and S112).

Next, differential signals obtained by differentiating A-axis direction scattered light intensity signals are determined with respect to each position of the BC plane (step S114). Note that, differential signals that are determined in step S110 and stored may also be used.

Next, detection of positions at which the slope of A-axis direction scattered light intensity signals abruptly rises within a positional range between an outer side of the outermost surface and an inner side of a boundary between the first layer and the second layer is performed based on differential signals for each position of the BC plane (step S116). For example, when A-axis direction scattered light intensity signals at positions designated by reference characters a and b in Fig. 11A are illustrated in Fig. 11B, it is found that in the A-axis direction scattered light intensity signal at position b at which a lesion part does not exist, there is no position at which the slope rises abruptly in the above described range of positions. In contrast, in the A-axis direction scattered light intensity signal at position a at which a lesion part does exist, there is a position at which the slope rises abruptly in the above described range of positions, and that position is detected.

Subsequently, detection is performed to identify a region (continuous region that is equal to or greater than a predetermined size) that has a certain three-dimensional expanse that is formed by detected positions. If such a region is detected, finally it is determined that the region in question is a lesion part (region of a lesion part) (step S 118).

Processing of detection processing type 4 is performed by the above described processing procedure, and together with detection of a lesion part corresponding to lesion type 1 being performed, a region of a detected lesion part is extracted as a feature region to be displayed at a time of displaying lesion parts that is described later.

### 5. Detection processing type 5

When a site that has a substantially circular (bubble shape) high-scattering region on a BC cross section in the vicinity of an outermost surface exists at an inner wall portion (inner wall portion of a living organism) of a biliary tract or a pancreatic duct for which spatial structure data has been acquired, the processing of detection processing type 5 detects the site as a lesion part (feature region) that has a characteristic structure. Thus, detection of a lesion part of lesion type 1 in which a papillary protrusion has arisen on an outermost surface is performed.

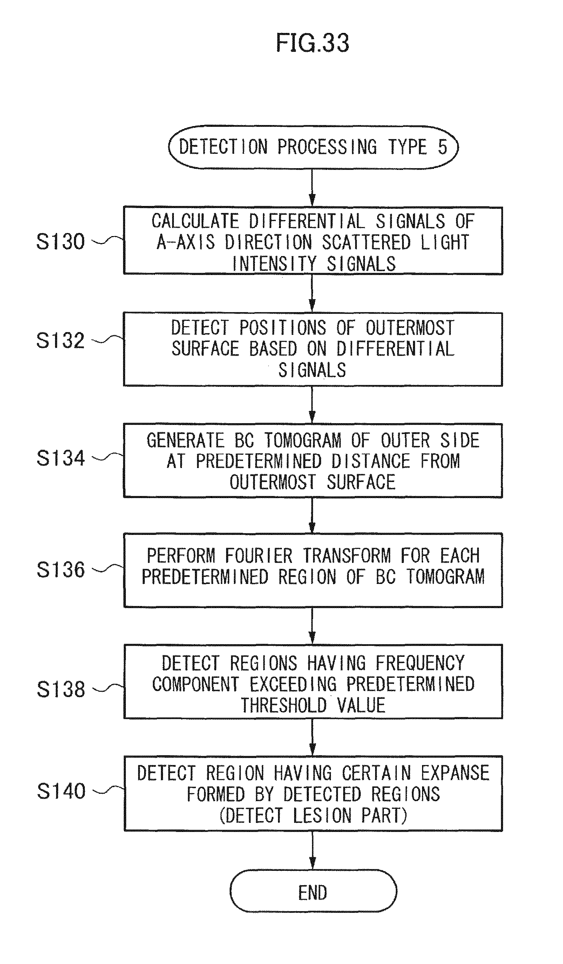

Processing is performed by the following procedure according to detection processing type 5. Refer to the flowchart shown in Fig. 33.

First, similarly to detection processing type 1, for each position of the BC plane, positions in the A-axis direction on the outermost surface of the inner wall portion of the living organism are detected (steps S130 and S132).

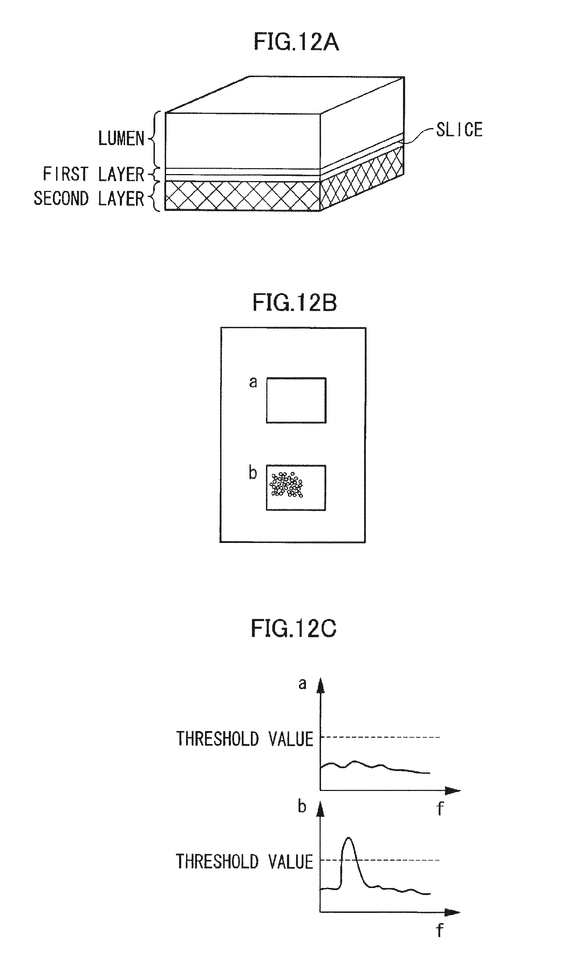

Next, a BC tomogram (reslice image) of a BC cross section at positions separated by a fixed distance in the A-axis direction from positions on the outermost surface as shown in Fig. 12A is generated as illustrated in Fig. 12B (scattered light intensity data at the BC cross section is extracted) (step S 134).

Next, the tomogram is divided into regions of a predetermined size at a BC cross section, and a tomogram (scattered light intensity data) for each region is subjected to a Fourier transform and frequency components of the tomograms of each region are obtained (step S136).

Next, region that have a frequency component that exceeds a predetermined threshold value are detected (step S138). For example, when values of frequency components of each frequency obtained when tomograms of regions designated by reference characters a and b in Fig. 12B are subjected to a Fourier transform are shown in Fig. 12C, it is found that, with respect to the region a in which a lesion part does not exist, there are no frequency component values that exceed the threshold value. In contrast, a bubble shaped pattern that exists in the BC tomogram in the vicinity of the outermost surface is a lesion parts of lesion type 1 in which a papillary protrusion has arisen on the outermost surface, and a frequency peak that exhibits a value that exceeds the threshold value exists in the frequency component of the region b in which the lesion part exists. That frequency peak is detected by the above described processing.

Subsequently, detection is performed to identify a region (continuous region that is equal to or greater than a predetermined size) that has a certain expanse that is formed by regions detected as having a frequency component exceeding a predetermined threshold value. If such a region is detected, finally it is determined that the region in question is a lesion part (region of a lesion part) (step S 140).

Processing of detection processing type 5 is performed by the above described processing procedure, and together with detection of a lesion part corresponding to lesion type 1 being performed, a region of a detected lesion part is extracted as a feature region to be displayed at a time of displaying lesion parts that is described later.

### 6. Detection processing type 6

When a site that has a spherical or hemispherical low-scattering region having a diameter that exceeds a predetermined value exists at an inner wall portion (inner wall portion of a living organism) of a biliary tract or a pancreatic duct for which spatial structure data has been acquired, the processing of detection processing type 6 detects the site as a lesion part (feature region) that has a characteristic structure.

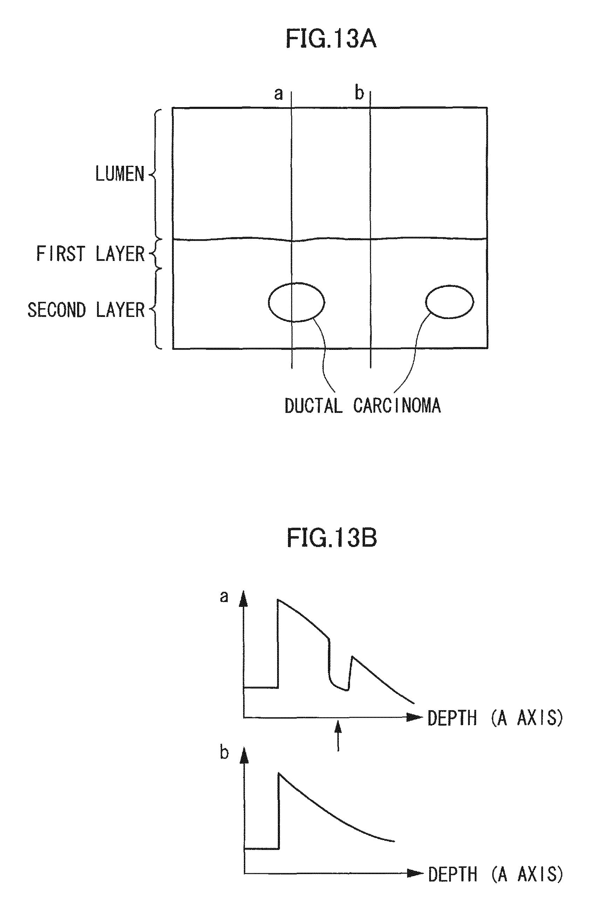

Fig. 13A is a view that, by means of an AC tomogram through the lesion part, illustrates an example of spatial structure data of an inner wall of a living organism that has a lesion part of lesion type 5 that is detected by detection processing type 6. In the figure, a lesion part of lesion type 5 in which a ductal carcinoma has arisen is illustrated.

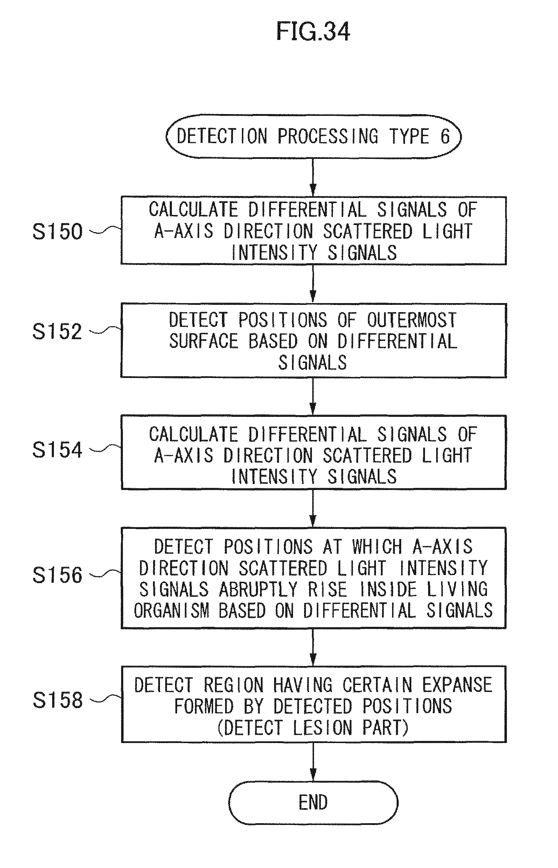

In order to detect such a lesion part, processing is performed by the following procedure according to detection processing type 6. Refer to the flowchart shown in Fig. 34.

First, by the same processing as detection processing type 2, detection of positions of an outermost surface of an inner wall portion of a living organism is performed (steps S150 and S152).

Next, differential signals obtained by differentiating A-axis direction scattered light intensity signals are determined with respect to each position of the BC plane (step S 154). Note that, differential signals that are determined in step S150 and stored may also be used.

Next, detection of positions at which the slope of A-axis direction scattered light intensity signals abruptly rises within the living organism is performed based on differential signals for each position of the BC plane (step S156). For example, when A-axis direction scattered light intensity signals at positions designated by reference characters a and b in Fig. 13A are illustrated in Fig. 13B, it is found that in the A-axis direction scattered light intensity signal at position b at which a lesion part (ductal carcinoma) does not exist, there is no position at which the slope rises abruptly in the above described range of positions. In contrast, in the A-axis direction scattered light intensity signal at position a at which a lesion part does exist, there is a position at which the slope rises abruptly in the above described range of positions, and that position is detected.

Subsequently, detection is performed to identify a region (continuous region that is equal to or greater than a predetermined size) that has a certain three-dimensional expanse that is formed by detected positions. If such a region is detected, finally it is determined that the region in question is a lesion part (region of a lesion part) (step S 158).

Processing of detection processing type 6 is performed by the above described processing procedure, and together with detection of a lesion part corresponding to lesion type 5 being performed, a region of a detected lesion part is extracted as a feature region to be displayed at a time of displaying lesion parts that is described later.

### 7. Detection processing type 7

When a site in which a distance in the A-axis direction from an outermost surface to a position at which a scattered light intensity reaches a signal boundary (a noise level) is shorter than a predetermined threshold value exists at an inner wall portion (inner wall portion of a living organism) of a biliary tract or a pancreatic duct for which spatial structure data has been acquired, the processing of detection processing type 7 detects the site as a lesion part (feature region) that has a characteristic structure.

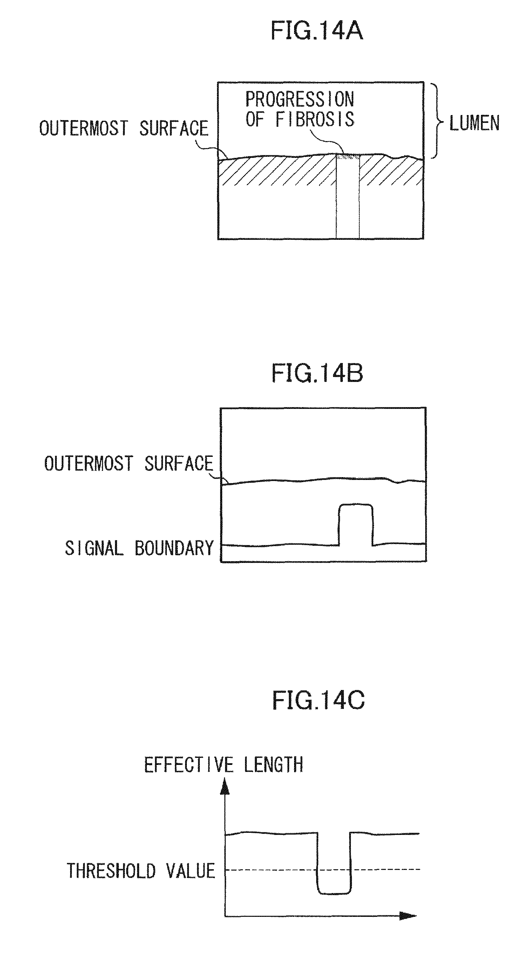

Fig. 14A is a view that, by means of an AC tomogram through the lesion part, illustrates an example of spatial structure data of an inner wall of a living organism that has a lesion part of lesion type 3 that is detected by detection processing type 7. In the figure, a lesion part of lesion type 3 in which fibrosis has progressed is illustrated.

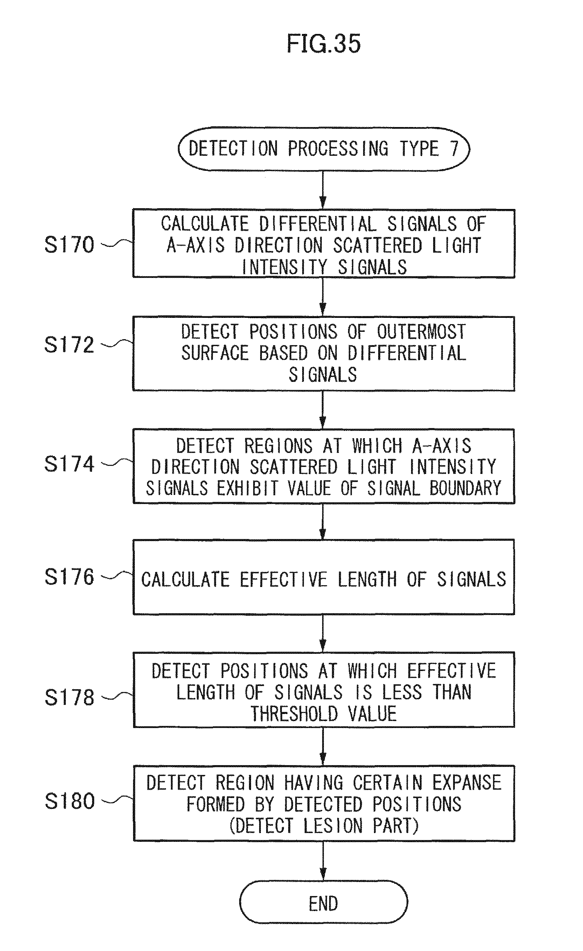

In order to detect such a lesion part, processing is performed by the following procedure according to detection processing type 7. Refer to the flowchart shown in Fig. 35.

First, by the same processing as detection processing type 1, positions in the A-axis direction of the outermost surface of the inner wall portion of the living organism are detected for each position of the BC plane as shown in Fig. 14A (step 170 and 172) .

Next, with respect to each position of the BC plane, a region in which a signal value of an A-axis direction scattered light intensity signal is a value (value less than a predetermined threshold value that is taken as a signal boundary) that shows a signal boundary that is equal to a noise level on an outer side from a certain position in the A-axis direction is extracted as shown in Fig. 14B (step S 174). As a result, with respect to each position of the BC plane, a position of a boundary that is the innermost side in the A-axis direction of a signal boundary region is detected.

Subsequently, with respect to each position of the BC plane, a length (effective length of the signal) in the A-axis direction from the position of the outermost surface of the inner wall portion of the living organism to the position of the boundary of the signal boundary region is calculated as shown in Fig. 14C (step S 176).

Next, as shown in Fig. 14C, a position on the BC plane at which the effective length of the signal in the A-axis direction exhibits a value that is less than a predetermined threshold value is detected (step S 178). When fibrosis progresses as in the case of lesion type 3, the intensity of back-scattered light at that portion increases and the effective length of the signal in the A-axis direction shortens by a corresponding amount. Hence, a position of a lesion part of lesion type 3 can be detected.

Subsequently, detection is performed to identify a region (continuous region that is equal to or greater than a predetermined size) that has a certain expanse that is formed by detected positions. If such a region is detected, finally it is determined that the region in question is a lesion part (region of a lesion part) (step S 180).

More specifically, according to detection processing type 7, an evaluation value is calculated that corresponds to a length from an arbitrary measurement point set on the BC plane (specifically, the surface of the inner wall portion) that is perpendicular to the A-axis direction to a boundary point at which a signal value of a scattered light intensity signal relating to the A-axis direction becomes less than (specifically, becomes a noise level) a value that indicates the signal boundary, and a measurement point at which the evaluation value becomes less than a predetermined threshold value is detected. If a set of the detected measurement points form a continuous region that is equal to or greater than a predetermined size, that region is detected as a lesion part.

Processing of detection processing type 7 is performed by the above described processing procedure, and together with detection of a lesion part corresponding to lesion type 3 being performed, a region of a detected lesion part is extracted and set as a feature region to be displayed at a time of displaying lesion parts that is described later.

### 8. Detection processing type 8

When a site in which a normal layered structure has disappeared exists at an inner wall portion (inner wall portion of a living organism) of a biliary tract or a pancreatic duct for which spatial structure data has been acquired, the processing of detection processing type 8 detects the site as a lesion part (feature region) that has a characteristic structure. Thus, detection of a lesion part of lesion type 3 in which fibrosis has progressed and a layered structure has disappeared is performed.

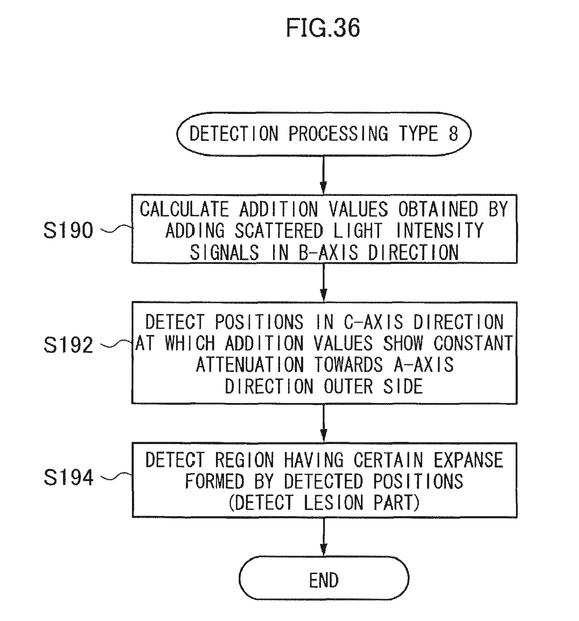

Processing is performed by the following procedure according to detection processing type 8. Refer to the flowchart shown in Fig. 36.

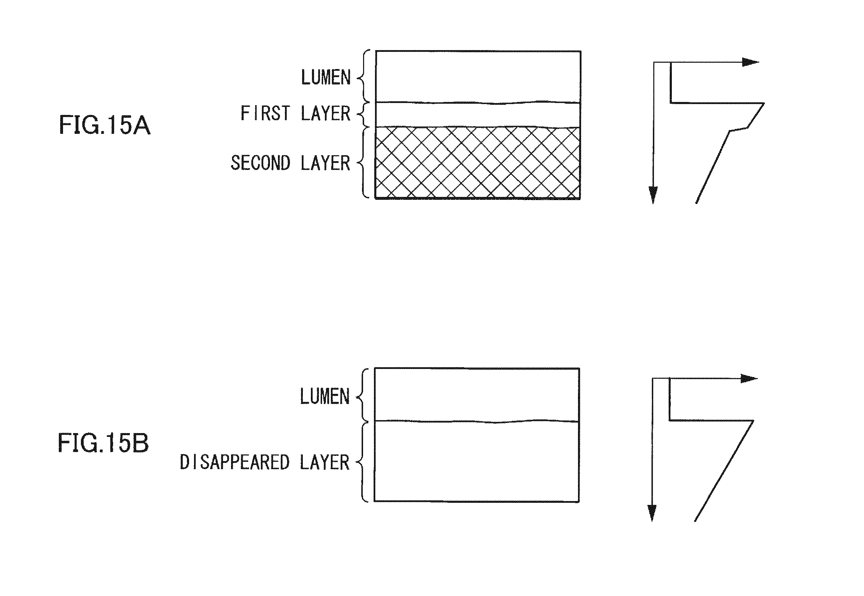

First, scattered light intensity data in the B-axis direction for respective positions on the AC plane is added (step S190). As a result, with respect to respective positions in the C-axis direction, an addition value of scattered light intensity data at respective positions in the A-axis direction is obtained. Fig. 15A shows an addition value for respective positions in the A-axis direction with respect to a certain position in the C-axis direction in a case where this processing is performed for a normal inner wall portion that has a first layer and a second layer. Fig. 15B shows an addition value for respective positions in the A-axis direction with respect to a certain position in the C-axis direction in a case where this processing is performed for an inner wall portion having a lesion part in which fibrosis has progressed and a layered structure has disappeared as in the case of lesion type 5.

Next, it is determined whether or not the addition values show a constant attenuation (case shown in Fig. 15B) towards the outer side in the A-axis direction with respect to each position in the C-axis direction, and if the determined result is affirmative, the position in the C-axis direction is detected (step S 192). Here, it is preferable to also treat a case where the addition values are in a range which is regarded as attenuating in a constant manner towards the outer side in the A-axis direction as a case in which the determined result is affirmative. It is thereby possible to prevent a detection omission of a region in which the layered structure is disappearing.

Subsequently, detection is performed to identify a region (continuous region that is equal to or greater than a predetermined size) that has a certain expanse that is formed by detected positions. If such a region is detected, finally it is determined that the region in question is a lesion part (region of a lesion part) (step S 194). As a result, a site at which a layered structure has disappeared, as shown in Fig. 15B, is detected as a lesion part.

Processing of detection processing type 8 is performed by the above described processing procedure, and together with detection of a lesion part corresponding to lesion type 3 being performed, a region of a detected lesion part is extracted as a feature region to be displayed at a time of displaying lesion parts that is described later.

Note that, as a modification example of detection processing type 8, processing may be performed by reversing the processing for the B axis and the C axis. More specifically, according to this modification example, first, scattered light intensity data in the C-axis direction for each position on the AB plane is added. As a result, an addition value of scattered light intensity data at each position in the A-axis direction is obtained for each position in the B-axis direction. Next, it is determined whether or not addition values show a constant attenuation towards the outer side in the A-axis direction with respect to each position in the B-axis direction, and if the determined result is affirmative, the position in the B-axis direction is detected. Subsequently, detection is performed to identify a region (continuous region that is equal to or greater than a predetermined size) that has a certain expanse that is formed by detected positions. If such a region is detected, finally it is determined that the region in question is a lesion part (region of a lesion part).

Further, a direction of adding scattered light intensity data is not limited to the B-axis direction or the C-axis direction, and adding may be performed with respect to any direction as long as the direction is at least parallel to the BC plane. More specifically, a configuration may also be adopted in which scattered light intensity data is added in a diagonal direction that is parallel to the BC plane and is not orthogonal to the B-axis direction and the C-axis direction.

That is, according to detection processing type 8, scattered light intensity data of a predetermined range at a same depth that relates to the A-axis direction from an arbitrary measurement point set on a BC plane (specifically, the surface of an inner wall portion) that is perpendicular to the A-axis direction is added, and the measurement point in question is detected if the addition values are in a range which is regarded as attenuating in a constant manner towards the outer side in the A-axis direction. Further, if a set of the detected measurement points form a continuous region that is equal to or greater than a predetermined size, that region is detected as a lesion part.

### 9. Detection processing type 9

When a site in which a low-scattering region that is distributed in a linear or reticulate shape has arisen exists at an inner wall portion (inner wall portion of a living organism) of a biliary tract or a pancreatic duct for which spatial structure data has been acquired, the processing of detection processing type 9 detects the site as a lesion part (feature region) that has a characteristic structure.

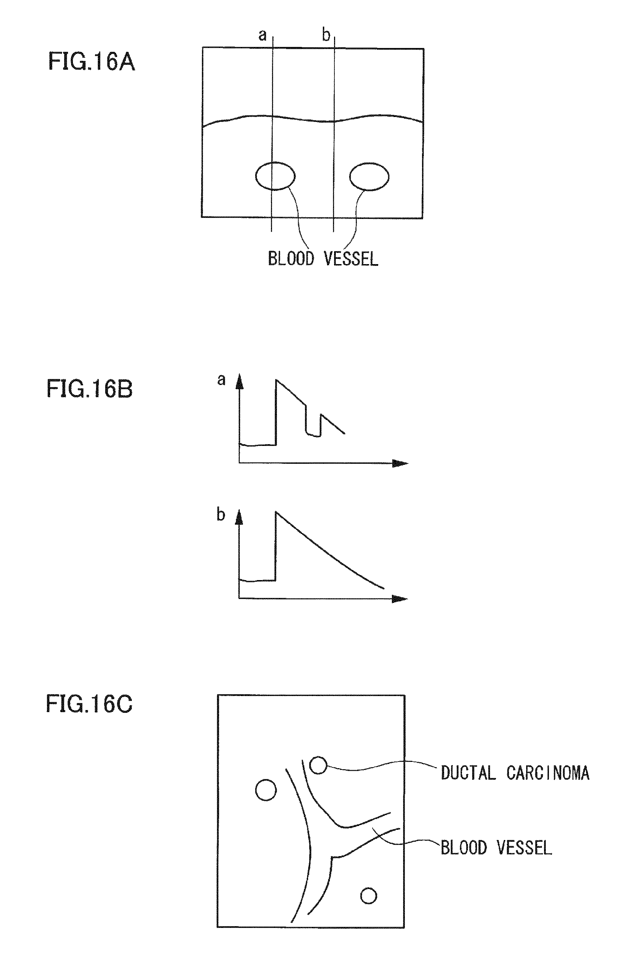

Fig. 16A is a view that, by means of an AC tomogram through the lesion part, illustrates an example of spatial structure data of an inner wall of a living organism that has a lesion part of lesion type 6 that is detected by the present detection processing type 9. Fig. 16C shows a lesion part of lesion type 6 in which a blood vessel (new blood vessel) has arisen in a second layer.

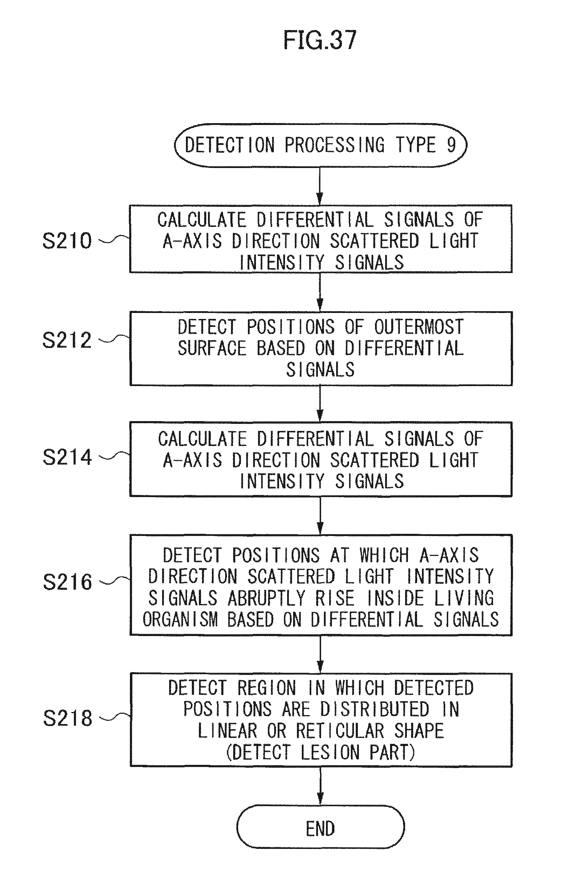

In order to detect such a lesion part, processing is performed by the following procedure according to detection processing type 9. Refer to the flowchart shown in Fig. 37.

First, by processing that is similar to the detection processing type 2, positions on the inner wall surface portion are detected (steps S210 and S212).

Next, differential signals obtained by differentiating A-axis direction scattered light intensity signals are determined with respect to each position of the BC plane (step S214). Note that, differential signals that are determined in step S210 and stored may also be used.

Next, detection of positions at which the slope of an A-axis direction scattered light intensity signal rises abruptly within the living organism is performed based on differential signals for each position of the BC plane (step S216). For example, when A-axis direction scattered light intensity signals at positions designated by reference characters a and b in Fig. 16A are illustrated in Fig. 16B, it is found that in the A-axis direction scattered light intensity signal at position b at which a blood vessel does not exist, there is no position at which the slope rises abruptly in the above described range of positions. In contrast, in the A-axis direction scattered light intensity signal at position a at which a blood vessel does exist, there is a position at which the slope rises abruptly in the above described range of positions, and that position is detected.

Subsequently, detection is performed to identify a region (continuous region that is equal to or greater than a predetermined size) that has a certain three-dimensional expanse that is formed by detected positions and which is distributed in a linear or reticulate shape (see Fig. 16C). If such a region is detected, finally it is determined that the region in question is a lesion part (region of a lesion part) (step S218).

Processing of detection processing type 9 is performed by the above described processing procedure, and together with detection of a lesion part corresponding to lesion type 9 being performed, a region of a detected lesion part is extracted as a feature region to be displayed at a time of displaying lesion parts that is described later.

In this connection, in some cases, when performing OCT measurement, there are portions that the sheath 44 of the OCT probe 40 contacts on the outermost surface of the inner wall portion of the living organism and portions that the sheath 44 of the OCT probe 40 does not contact. If the sheath 44 contacts the outermost surface of the inner wall portion of the living organism, since the inner wall portion is compressed by the pressing effect, in a case where a region is determined to be a lesion part if the thickness of the first layer is greater than a predetermined threshold value, such as in detection processing type 2, even if a lesion part has arisen, there is a risk that the relevant region will not be identified as a lesion part.

Therefore, a configuration may be adopted that detects and separates a region which the sheath 44 contacts and a region which the sheath 44 does not contact on the outermost surface of the inner wall portion of the living organism, and sets a threshold value in accordance with a contact state or non-contact state of the sheath 44 for the respective regions.

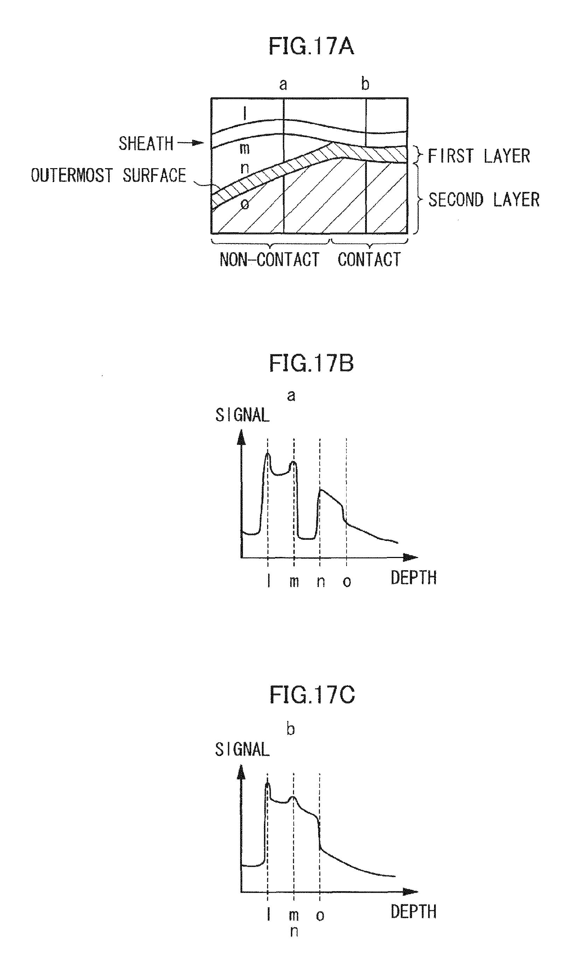

Detection of a region that the sheath 44 contacts (contact region) and a region that the sheath 44 does not contact (non-contact region) is performed as follows. Fig. 17A is a view that illustrates, by means of an AC tomogram, an example of spatial structure data prior to eliminating scattered light intensity data of the sheath 44. In the figure, a contact region and a non-contact region of the sheath 44 are shown. In this spatial structure data, for example, an A-axis direction scattered light intensity signal of a position in the non-contact region that is designated by reference character a in Fig. 17A is a signal as shown in Fig. 17B, and an A-axis direction scattered light intensity signal of a position in the contact region that is designated by reference character b in Fig. 17A is a signal as shown in Fig. 17C. According to this example, in the non-contact region, as shown in Fig. 17B, an inner wall 1 and an outer wall m of the sheath 44, an outermost surface n of the inner wall portion of the living organism, and a boundary 0 between the first layer and the second layer show characteristic changes at different positions. In contrast, in the contact region, as shown in Fig. 17C, since the positions of the outer wall m of the sheath 44 and the outermost surface n are substantially matching, unlike the non-contact region, four characteristic changes do not exist.

Accordingly, a contact region and a non-contact region of the sheath 44 can be detected by differentiating A-axis direction scattered light intensity signals with respect to each position of the BC plane, and detecting positions 1, m, n, and o of the A-axis direction scattered light intensity signals based on the differential signals.

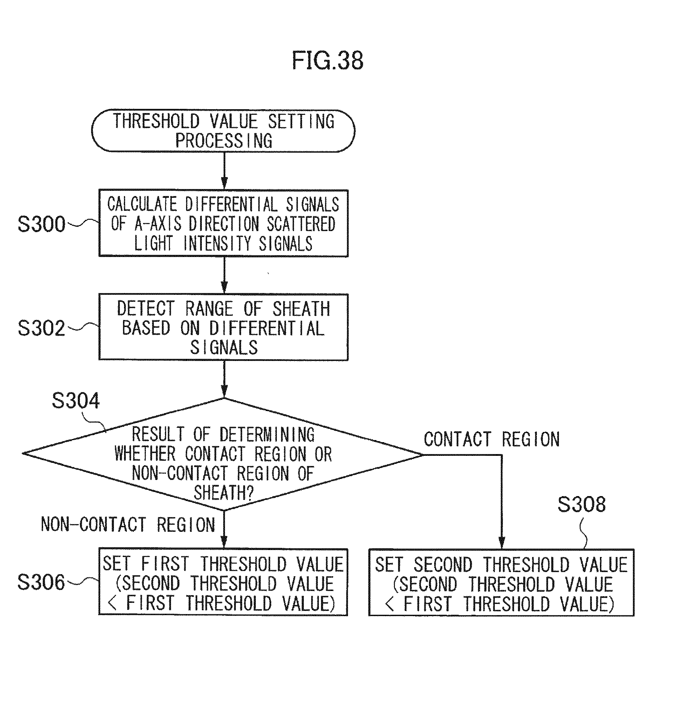

Hereunder, processing procedures in the arithmetic processing apparatus 90 when changing a threshold value of detection processing type 2 in accordance with a contact region and a non-contact region of the sheath 44 are described using the flowchart shown in Fig. 38.

For example, the following processing for distinguishing a contact region and a non-contact region of the sheath 44 is executed before executing the processing of detection processing type 2. First, A-axis direction scattered light intensity signals for each position of the BC plane are formed by spatial structure data including scattered light intensity data of the sheath 44 prior to the above described sheath elimination processing being executed as pre-processing, and the A-axis direction scattered light intensity signals are differentiated with respect to each position of the BC plane to generate differential signals (step S300).

Next, with respect to each position of the BC plane, the range of the sheath 44 is detected based on the differential signals (step S302). More specifically, a position in the A-axis direction at which a differential signal shows a maximum value (or a position of a local maximum value that exists on the innermost side in the A-axis direction) is detected as a position of an inner wall face of the sheath 44, and a range from that position to a position of the outer circumferential face (outer wall face) of the sheath 44 that is separated therefrom in a direction towards the outer side in the A-axis direction by a known thickness amount of the sheath 44 is detected as the range of the sheath 44.

Next, with respect to each position of the BC plane, by determining whether or not a differential signal has a signal value that is a large value (larger value than a predetermined threshold value) that corresponds to the outermost surface of the inner wall portion of the living organism that is outside the range (outer circumferential face) of the sheath 44, it is determined whether or not the relevant position is at a non-contact region or a contact region of the sheath 44 (step S304). More specifically, since an A-axis direction scattered light intensity signal as shown in Fig. 17B is obtained at a non-contact region of the sheath 44, a differential signal has a signal value of a large value (larger value than a predetermined threshold value) at a position that is further to the outer side than the position of the outer circumferential face of the sheath 44. In contrast, since an A-axis direction scattered light intensity signal as shown in Fig. 17C is obtained at a contact region of the sheath 44, a differential signal does not have a signal value of a large value (larger value than a predetermined threshold value) at a position that is further to the outer side than the position of the outer circumferential face of the sheath 44. Accordingly, when it is determined that a differential signal has a signal value of a large value (larger value than a predetermined threshold value) that corresponds to the outermost surface of the inner wall portion of the living organism that is further to the outer side than the outer circumferential face of the sheath 44, it is determined that a position on the BC plane at which the differential signal is obtained is a non-contact region (non-contact position) of the sheath 44. In contrast, when it is determined that a differential signal does not have a signal value of a large value (larger value than a predetermined threshold value) that corresponds to the outermost surface of the inner wall portion of the living organism that is further to the outer side than the outer circumferential face of the sheath 44, it is determined that a position on the BC plane at which the differential signal is obtained is a contact region (contact position) of the sheath 44.

Next, a threshold value that is used for processing of detection processing type 2 is set to a first threshold value with respect to positions on the BC plane that are determined as being non-contact regions of the sheath 44 (step S306). In contrast, the threshold value that is used for processing of detection processing type 2 is set to a second threshold value with respect to positions on the BC plane that are determined as being contact regions of the sheath 44 (step S308).

When the above described processing ends, the processing of detection processing type 2 is executed. At such time, when performing the processing in step S76 in Fig. 30, with respect to positions of the BC plane that are determined as being non-contact regions of the sheath 44, the thickness of the first layer is compared with the first threshold value, and positions of the BC plane at which the thickness of the first layer shows a larger value than the first threshold value are detected as lesion part candidates. In contrast, with respect to positions of the BC plane that are determined as being contact regions of the sheath 44, the thickness of the first layer is compared with the second threshold value, and positions of the BC plane at which the thickness of the first layer shows a larger value than the second threshold value are detected as lesion part candidates.

Since the thickness of the first layer at both a normal site and a lesion part of a contact region of the sheath 44 is compressed (since the thickness decreases) compared to a non-contact region due to the pressing effect of the sheath, the second threshold value is set to a smaller value than the first threshold value.

Therefore, in the processing of detection processing type 2, a threshold value when determining whether or not the thickness of the first layer is normal is set to an appropriate value in accordance with whether the relevant region is a contact region or a non-contact region of the sheath 44, and thus detection of a lesion part is appropriately performed.

In this connection, the processing of detection processing type 2 is not limited to detection of a lesion part in the biliary tract or pancreatic duct, and the processing is also effective for detecting a lesion part in other organs that, similarly to the biliary tract and pancreatic duct, have a layered structure and in which a first layer has an approximately constant thickness at a normal time such as, for example, the bronchial tubes, the pharynx, the esophagus, and the urinary duct. When performing diagnostic support with respect to these sites, diagnosis can be supported by using detection processing type 2 as processing to detect a lesion part (feature region), and using a method similar to the present embodiment to display a detected lesion part and the like. At such time, by changing the threshold value so as to use respectively different threshold values for a contact region and a non-contact region of the sheath 44 as described above, detection of a lesion part is appropriately performed.

Further, processing that distinguishes between a contact region and a non-contact region of the sheath 44 and changes a threshold value so as to use respectively different threshold values for a contact region and a non-contact region can also be effectively applied in the processing of detection processing type 7. More specifically, according to the processing of detection processing type 7, as shown in step S 178 in the flowchart of Fig. 35, a position on the BC plane at which the effective length of a signal in the A-axis direction exhibits a smaller value than a predetermined threshold value is detected as a position of a lesion part. Since the effective length of a signal in the A-axis direction at a normal site and a lesion part is smaller in a contact region of the sheath 44 than in a non-contact region due to the pressing effect of the sheath 44, it is suitable to also set the threshold value that is compared to the effective length of the signal to a smaller value for a contact region of the sheath 44 compared to a non-contact region.

In the above description of the detection processing types 1 to 9, and also in the foregoing description, it is assumed that stronger scattered light intensity data is obtained from the second layer compared to the first layer. However, the opposite case may also arise depending on the measurement conditions. In such a case, the processing of the respective detection processing types 1 to 9 may be changed to take this fact into consideration.

Next, the diagnosis support function of the arithmetic processing apparatus 90 of the OCT apparatus 1 is described.

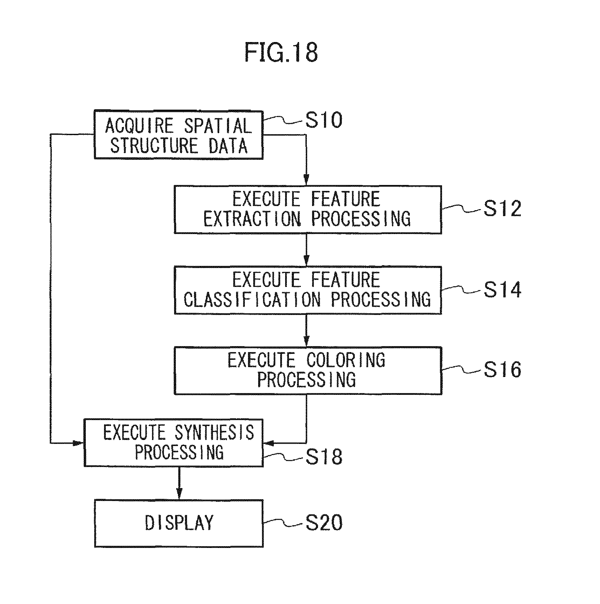

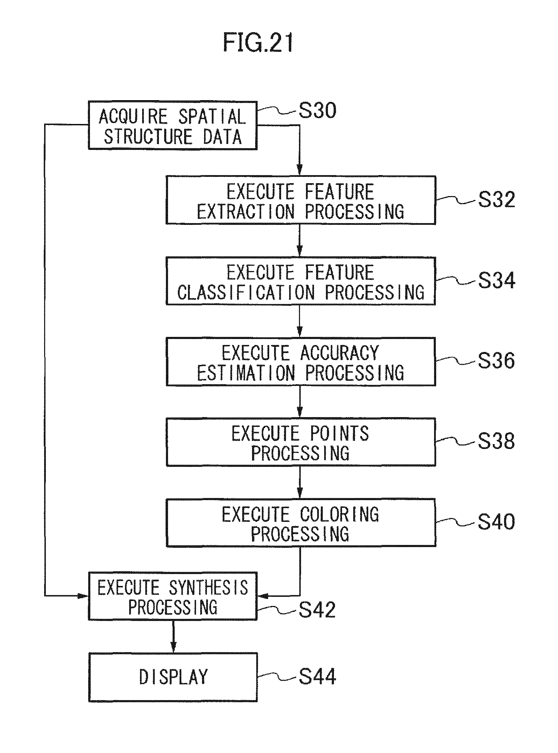

Fig. 18 is a flowchart that illustrates processing procedures according to a first embodiment of the diagnosis support function. Hereunder, the first embodiment of the diagnosis support function is described in accordance with the flowchart shown in Fig. 18. In this connection, the flowchart shown in Fig. 18 also shows functional blocks of the arithmetic processing apparatus 90, and the processing of each step also represents processing as respective processing sections in the arithmetic processing apparatus 90 (the same applies with respect to the flowchart in Fig. 21).

First, the arithmetic processing apparatus 90 acquires spatial structure data of an inner wall portion of a biliary tract or a bile duct (inner wall portion of a living organism) for which diagnosis is to be conducted (step S10). In cases other than those in which the data is to be directly acquired after OCT measurement, it is also possible to acquire spatial structure data that is stored in the database 112.

Next, when the observer instructs execution of the diagnosis support function by operating the input device 92, the arithmetic processing apparatus 90 executes feature extraction processing (step S12). In the feature extraction processing, processing of each of the detection processing types 1 to 9 that are described above is executed, and feature regions suspected of being a lesion part as described above are extracted. However, processing of all of the detection processing types 1 to 9 need not necessarily be executed, and a configuration may be adopted so as to execute processing of any one type or multiple types of the detection processing types 1 to 9, or so as to enable the operator to select the detection processing type(s) to be executed. When executing only processing of a single detection processing type, since only one color is used as a color to be associated with a feature region when performing coloring processing in step S16 that is described later, it is unnecessary to execute feature classification processing in the subsequent step S14.

Next, the arithmetic processing apparatus 90 executes feature classification processing (step S14). In the feature classification processing, the respective feature regions extracted by the feature extraction processing are classified into predetermined classification categories. For example, a method that classifies the feature regions by taking the kinds of detection processing types that extracted the respective feature regions or the kinds of extraction targets (kinds of characteristic shapes) as classification categories, or a method that classifies the feature regions into regions extracted due to a change in a mucous membrane shape (regions extracted by detection processing types 1 to 5 and 8) which are strongly suspected of being a lesion part and regions extracted due to the presence of a glandular duct or vascular network that can also be present at a normal portion and for which the suspicion of being a lesion part is weak (regions extracted by detection processing types 6 and 9), and the like are available. A configuration may also be adopted that allows the operator to appropriately change these classification methods.

Next, the arithmetic processing apparatus 90 executes coloring processing (step S16). According to the coloring processing, color information of colors that differ according to the feature regions belonging to the respective classification categories into which the feature regions are classified by the above described feature classification processing is associated with the respective feature regions.

Next, the arithmetic processing apparatus 90 executes synthesis processing with respect to the spatial structure data and the respective feature regions to which colors have been added (step S 18). According to this synthesis processing, color information that has been associated with the respective feature regions is applied to voxels corresponding to the respective feature regions in the spatial structure data to generate spatial structure data that has feature region information attached thereto (referred to as "spatial structure data with feature region information").

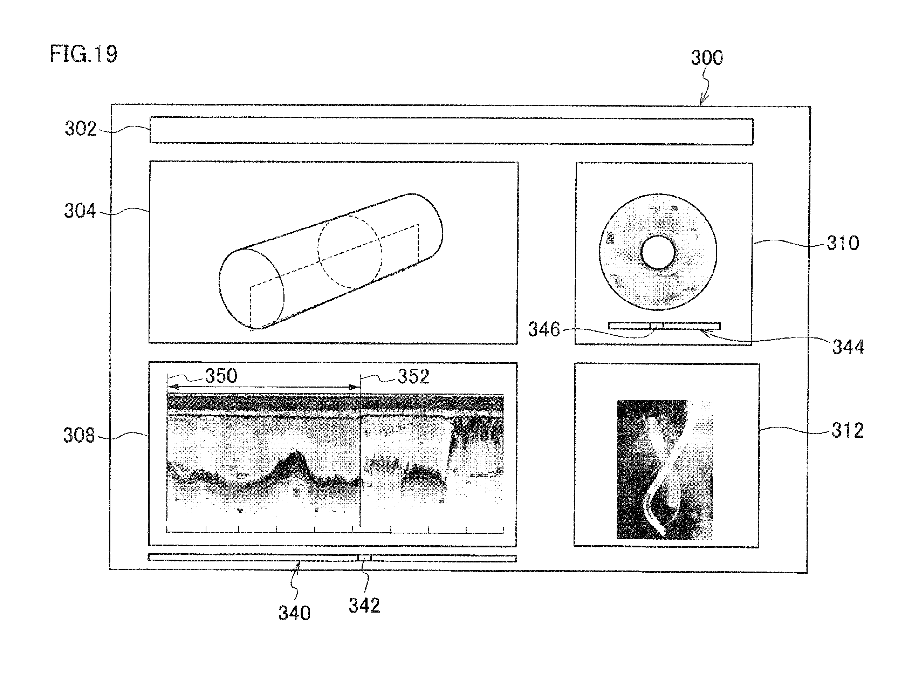

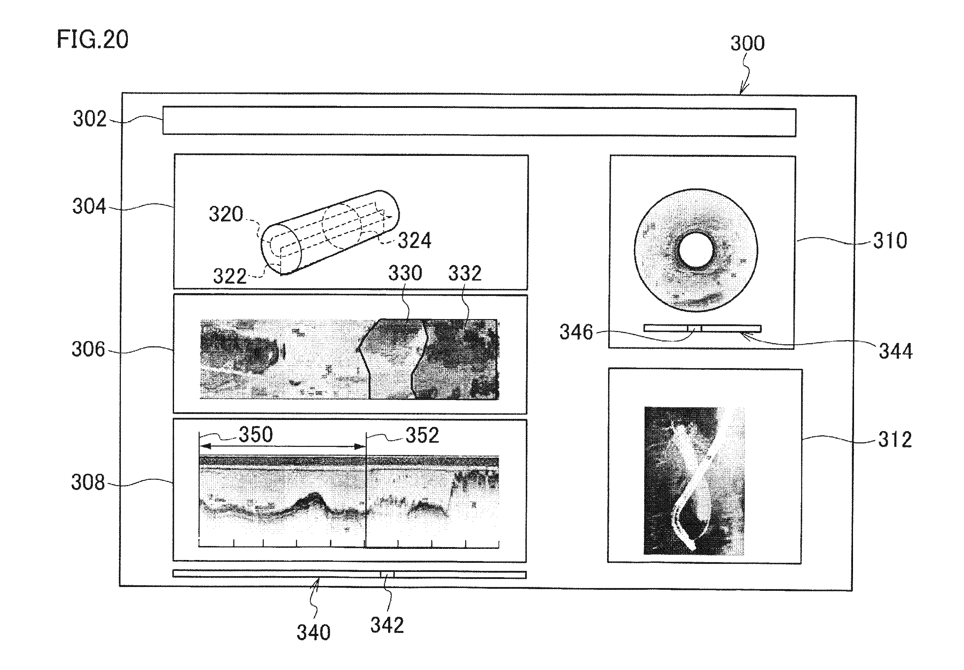

Subsequently, the arithmetic processing apparatus 90 executes display processing that includes processing that visualizes the spatial structure data with feature region information and displays a resulting image on the monitor 100 (step S20). According to this display processing, processing is performed that, for example, as shown in Fig. 19 or Fig. 20, generates a display screen that displays a plurality of forms of spatial structure images, and displays the generated display screen on the monitor 100.

When the configurations of the display screens shown in Fig. 19 and Fig. 20 are compared, it can be seen that all the components of the display screen shown in Fig. 19 are included in Fig. 20. Hence, components shown in Fig. 19 are designated by the same reference numbers as like components in Fig. 20, and a description of the components shown in Fig. 19 is omitted here, and only the configuration of the display screen shown in Fig. 20 is described.

A display screen 300 shown in Fig. 20 includes a character information display section 302 that displays character information, a three-dimensional image display section 304 that displays spatial structure data as a three-dimensional image, a BC tomogram display section 306 that displays a BC tomogram, an AC tomogram display section 308 that displays an AC tomogram, an AB tomogram display section 310 that displays an AB tomogram, and a modality image display section 312 that displays a modality image.

Various kinds of character information, such as the date on which measurement was performed and the patient information, is displayed on the character information display section 302.

A spatial structure image that displays spatial structure data in a manner that gives a three-dimensional appearance thereto, such as a three-dimensional image that is an image of the entire spatial structure data as viewed from an external diagonal direction (image of a "perspective mode" that is described later), or a three-dimensional image in which it appears as if the measurement region is being viewed with the endoscope that is inserted into the lumen of the biliary tract or pancreatic duct (image of an "endoscope mode" that is described later), is displayed on the three-dimensional image display section 304. Although a three-dimensional image of the "perspective mode" is shown in Fig. 20, the observer can change the form (display mode) of the three-dimensional image to be displayed by operating the input device 92. To display the three-dimensional image on the three-dimensional image display section 304, the arithmetic processing apparatus 90 executes display processing with respect to spatial structure data with feature region information, and executes visualization processing in the selected display mode. Further, in the three-dimensional image displayed on the three-dimensional image display section 304, a BC cross-section line 320 that shows a position and a range of a BC tomogram that is displayed on the BC tomogram display section 306, an AC cross-section line 322 that shows a position and a range of an AC tomogram that is displayed on the AC tomogram display section 308, and an AB cross-section line 324 that shows a position and a range of an AB tomogram that is displayed on the AB tomogram display section 310 are synthesized with the spatial structure image and displayed.

In the BC tomogram display section 306, a BC tomogram as shown in the figure is displayed in which voxel values (scattered light intensity data and color information) of the spatial structure data with feature region information at the position of the BC cross-section line 320 that is displayed in the three-dimensional image display section 304 are visualized by display processing of the arithmetic processing apparatus 90. The BC tomogram shown in Fig. 20 includes two feature regions 330 and 332 that were extracted by feature extraction processing (step S12 in Fig. 18) and classified into different classification categories by the feature classification processing (step S 14 in Fig. 18). The respective colors that are associated with the feature regions 330 and 332 are superimposed in a semitransparent state on the image of the inner wall portion of the living organism and displayed. Although not shown in the drawings, this kind of colored display of feature regions is also performed in a similar manner when including feature regions in a three-dimensional image of the three-dimensional image display section 304, an AC tomogram of the AC tomogram display section 308, and an AB tomogram of the AB tomogram display section 310. If the colored display of feature regions hinders observation of an image of the inner wall portion of the living organism, the observer can erase the coloring by performing a predetermined operation. In such case, an image of each display section is regenerated and displayed using the spatial structure data in the state prior to performing synthesis processing with respect to the spatial structure data and each colored feature region. More specifically, in a case where the diagnosis support function is not executed also, a display screen configured in the same manner as in Fig. 19 or Fig. 20 is displayed by the display function of the arithmetic processing apparatus 90. At such time, the spatial structure data is used as it is, and an image of each display section is displayed. Even after the diagnosis support function has been executed, as appropriate, the observer can switch to a display in which the diagnosis support function is not used.

The observer can change the position of the BC cross-section line 320 by operating the input device 92. For example, by performing an operation to drag the BC cross-section line 320 in the three-dimensional image display section 304 using a mouse, the position of the BC cross-section line 320 can be changed in the direction of the A axis and B axis. Further, by placing the BC tomogram display section 306 in a selected state and moving a slider 342 of a position selection bar 340 that is below the image from side to side, the observer can change the position of the BC cross-section line 320 in the B-axis direction. When the position of the BC cross-section line 320 is changed, a BC tomogram of the changed position is regenerated from the spatial structure data with feature region information, and the display of the BC tomogram on the BC tomogram display section 306 is updated. In this connection, the region of the BC cross section to be displayed as a BC cross-sectional image can also be enlarged or reduced by an operation of the input device 92 performed by the observer that changes the size of the range of the BC cross-section line 320.

In the AC tomogram display section 308, an AC tomogram is displayed in which voxel values (scattered light intensity data and color information) of the spatial structure data with feature region information at the position of the AC cross-section line 322 that is displayed in the three-dimensional image display section 304 are visualized by display processing of the arithmetic processing apparatus 90.

Similarly to BC cross-section line 320, the observer can change the position of the AC cross-section line 322 by operating the input device 92. For example, by performing an operation to drag the AC cross-section line 322 in the three-dimensional image display section 304 using a mouse, the position of the AC cross-section line 322 can be changed in the direction of the B axis. Further, by placing the AC tomogram display section 308 in a selected state and moving the slider 342 of the position selection bar 340 that is below the image from side to side, the observer can change the position of the AC cross-section line 322 in the B-axis direction. When the position of the AC cross-section line 322 is changed, an AC tomogram of the changed position is regenerated from the spatial structure data with feature region information, and the display of the AC tomogram on the AC tomogram display section 308 is updated. In this connection, the region of the AC cross section to be displayed as an AC cross-sectional image can also be enlarged or reduced by an operation of the input device 92 performed by the observer that changes the size of the range of the AC cross-section line 322.

Further, in the AC tomogram display section 308, a scale that shows the actual dimensions in the C-axis direction of the AC tomogram is displayed, and two resection lines, described later, are displayed on the AC tomogram.

In the AB tomogram display section 310, an AB tomogram is displayed in which voxel values (scattered light intensity data and color information) of the spatial structure data with feature region information at the position of the AB cross-section line 324 that is displayed in the three-dimensional image display section 304 are visualized by display processing of the arithmetic processing apparatus 90.

Further, similarly to AC cross-section line 322 and the like, the observer can change the position of the AB cross-section line 324 by operating the input device 92. For example, by performing an operation to drag the AB cross-section line 324 in the three-dimensional image display section 304 using a mouse, the position of the AB cross-section line 324 can be changed in the direction of the C axis. Furthermore, by placing the AB tomogram display section in a selected state and moving a slider 346of a position selection bar 344 that is inside the AB tomogram display section 310 from side to side, the observer can change the position of the AB cross-section line 324 in the C-axis direction. In this connection, the region of the AB cross section to be displayed as an AB cross-sectional image can also be enlarged or reduced by an operation of the input device 92 performed by the observer that changes the size of the range of the AB cross-section line 324.

A modality image of the same patient or the like that is acquired with medical equipment (modality) other than the OCT apparatus 1, such as a CT fluoroscopic apparatus, an MRI apparatus, or an EUS apparatus is displayed in the modality image display section 312. A modality image to be displayed can be selected by the observer from image data stored in the database 112 that is connected to via a communication line. The arithmetic processing apparatus 90 acquires the selected image data from the database 112 and displays the image on the modality image display section 312.

The display screen as described above is displayed on the monitor 100 by the display processing performed by the arithmetic processing apparatus 90. The observer refers to the various kinds of spatial structure images (three-dimensional images and tomograms) displayed on the display screen to identify a lesion part. At that time, the observer can change the position or size of the BC cross-section line 320, the AC cross-section line 322, and the AB cross-section line 324 to observe a tomogram of an arbitrary position in the measurement region in more detail to identify a lesion part. Further, the length in the C-axis direction of the measurement region normally reaches a range that is 10 to 1000 times greater than the length in the A-axis direction. Consequently, displaying an image of the entire measurement region including the C axis at the same time on the three-dimensional image display section 304 as an image that shows, for example, which range in the entire measurement region is shown by an image that is enlarged and displayed, such as a BC tomogram, an AC tomogram, and an AB tomogram, contributes significantly to enhancing the operability. Further, since a feature region that is suspected of being a lesion part is colored and displayed in a manner in which the feature region can be noticed at a glance, the observation time is significantly reduced compared to a case of observing the entire measurement region. Since the colors applied to feature regions differ for the respective feature regions that are classified into predetermined classification categories and are displayed, the workload of the observer decreases. When a configuration is adopted so as to color code and display the respective feature regions in accordance with the strength of suspicion of being a lesion part, the effect is further enhanced.

When performing observation, the observer places emphasis on observing a boundary region between a normal portion and a feature region that is a particularly important region in a feature region extracted by the feature extraction processing (step S12), and determines a boundary of the lesion part or surgical resection lines. At this time, the observer can use a mouse or the like to set the positions of the two resection lines 350 and 352 displayed on the AC tomogram display section 308 in Fig. 20 to desired positions. The two resection lines 350 and 352 show the two ends of the region to be resected, and although the two resection lines 350 and 352 are set to positions at the two ends of AC tomogram in a prescribed state, it is possible to move and set the resection lines 350 and 352 at desired positions. The observer can mark positions at both ends of resection regions that are sequentially decided by means of the resection lines 350 and 352.

Further, an X-ray fluoroscopic image of the vicinity of a measurement site that is generated by the X-ray fluoroscopic apparatus 110 when the OCT probe 40 of the OCT apparatus 1 is inserted into a measurement site of the biliary tract or the pancreatic duct and measurement is performed can be displayed on the modality image display section 312. At such time, since the markers 68A and 68B that indicate the start point and end point of C scanning applied to the OCT probe 40 are appear on the X-ray fluoroscopic image as the result of contrast imaging, the observer can ascertain the position of the measurement region in the entire biliary tract or pancreatic duct by taking the positions of the markers 68A and 68B as reference marks. Further, since it is possible to know which positions in the measurement region that the positions of the resection lines 350 and 352 that are set on the AC tomogram display section 308 in Fig. 20 are at by means of the scale, the observer can also ascertain which range of the entire biliary tract or pancreatic duct the resection region is. As processing of the arithmetic processing apparatus 90, it is also possible to superimpose and display the positions of the resection lines 350 and 352 that are set on the AC tomogram display section 308 on the X-ray fluoroscopic image, by taking the positions of the markers 68A and 68B on the X-ray fluoroscopic image on the modality image display section 312 as reference marks. Further, by adopting a configuration in which the positions of the resection lines 350 and 352 that are ultimately decided are stored in the database 112, or in which the display screen of the monitor 100 is attached as it is to the electronic clinical record to enable confirmation on the electronic clinical record, confirmation when describing the relevant diagnosis to the patient or when confirming the diagnosis with another physician can be simply conducted.

Next, a second embodiment of the diagnosis support function is described according to a flowchart illustrating processing procedures shown in Fig. 21.

First, the arithmetic processing apparatus 90 acquires spatial structure data of an inner wall portion (inner wall portion of a living organism) of a biliary tract or a bile duct for which diagnosis is to be conducted (step S30). In cases other than those in which the data is to be directly acquired after OCT measurement, it is also possible to acquire spatial structure data that is stored in the database 112.

Next, when the observer instructs execution of the diagnosis support function by operating the input device 92, the arithmetic processing apparatus 90 executes feature extraction processing (step S32). In the feature extraction processing, processing of each of the detection processing types 1 to 9 that are described above is executed, and a feature region that is suspected of being a lesion part as described above is extracted. However, processing of all of the detection processing types 1 to 9 need not necessarily be executed, and a configuration may be adopted so as to execute processing of any plurality of the detection processing types 1 to 9, or so as to enable the operator to select the detection processing type(s) to be executed.

Next, the arithmetic processing apparatus 90 executes feature classification processing (step S34). In the feature classification processing, the respective feature regions extracted by the feature extraction processing are classified into predetermined classification categories. For example, a method that classifies the feature regions by taking the kinds of detection processing types that extracted the respective feature regions or the kinds of extraction targets (kinds of characteristic shapes) as classification categories, or a method that classifies the feature regions into regions extracted due to a change in a mucous membrane shape (regions extracted by detection processing types 1 to 5 and 8) which are strongly suspected of being a lesion part and regions extracted due to the presence of a glandular duct or vascular network that can be also present at a normal portion and for which the suspicion of being a lesion part is weak (regions extracted by detection processing types 6 and 9), and the like are available. A configuration may also be adopted that allows the operator to appropriately change these classification methods.

Next the arithmetic processing apparatus 90 executes probability estimation processing (step S36). According to the probability estimation processing, points (1 to 10) are assigned in accordance with a degree of possibility of being a lesion part to each classification category into which the respective feature regions have been classified by the feature classification processing. For example, the following categories a to d are utilized as classification categories.
a. Feature region in which a layered structure has disappeared
b. Feature region in which a first layer has thickened
c. Feature region with abnormal surface roughness
d. Feature region in which a lumen is present

At this time, in accordance with the degree of possibility of the feature region being a lesion part (cancer), ten points are assigned to a feature region belonging to classification category a, three points are assigned to a feature region belonging to classification category b, three points are assigned to a feature region belonging to classification category c, and two points are assigned to a feature region belonging to classification category d.

Note that a configuration may also be adopted so as to change the number of points in consideration of a value (feature value) that is used in ultimately determining whether or not a region is a feature region in the respective detection processing types 1 to 9.

Next, the arithmetic processing apparatus 90 performs points processing (step S38). In the points processing, a degree of risk is set based on the number of points assigned to each feature region. For example, if the number of points assigned to a feature region is ten or more, a degree of risk A that indicates a high possibility of being a lesion part (cancer) is set for the relevant feature region; if the number of points assigned to a feature region is five or more and less than ten, a degree of risk B that indicates a moderate possibility of being a lesion part is set for the relevant feature region; and if the number of points assigned to a feature region is three or more and less than five, a degree of risk C that indicates that the possibility of being a lesion part is low but that attention is required is set for the relevant feature region. Further, with respect to an overlapping region in which a plurality of feature regions overlap, the number of points assigned to the feature regions are added, and the addition value is assigned to the overlapping region. One of the degrees of risk A to C is then set for the overlapping region based on the number of points assigned to that region.

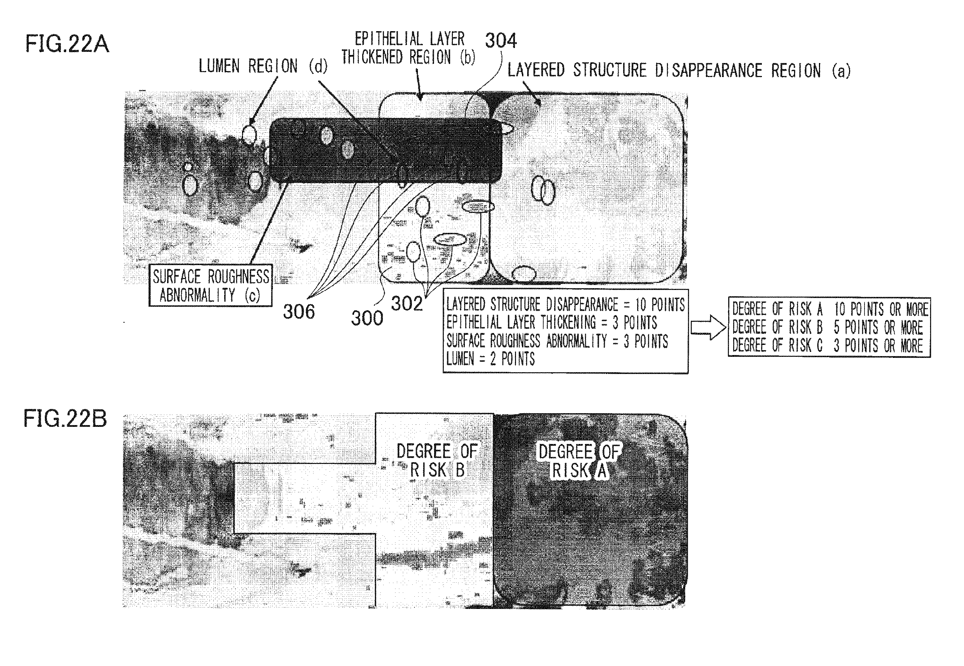

In Fig. 22A, a BC tomogram is shown that is to be displayed on a display section corresponding to the BC tomogram display section 306 shown in Fig. 20. It is assumed that feature regions extracted by the feature extraction processing are present in the BC tomogram, and that the feature regions are classified into the above described classification categories a to d by the feature classification processing. In the same figure, for example, in a feature region of classification category b, 3 points are assigned to a non-overlapping region 300 which is not overlapping with another feature region, 3+3 = 6 points are assigned to an overlapping region 302 that overlaps with only a feature region of classification category c, 3+2 = 5 points are assigned to an overlapping region 304 that overlaps with a feature region of classification category d, and 3+3+2 = 8 points are assigned to an overlapping region 306 that overlaps with a feature region of both classification category b and classification category c. Points are assigned to non-overlapping regions and overlapping regions of other feature regions also in the same manner. Subsequently, a degree of risk A, B, or C is set in accordance with the number of points with respect to the non-overlapping regions and overlapping regions to which points have been assigned (hereunder, the non-overlapping regions and overlapping regions are respectively referred to as an "evaluation region"). The respective evaluation regions for which a degree of risk A, B, or C has been set in this manner are shown in Fig. 22B.

In this connection, a configuration may also be adopted in which the number of points assigned to each evaluation region prior to conversion to a degree of risk is taken as it is as a value of the degree of risk.

Next, the arithmetic processing apparatus 90 executes coloring processing (step S40). According to the coloring processing, color information of different colors that are in accordance with the degree of risk is associated with the respective evaluation regions for which a degree of risk has been set.

Next, the arithmetic processing apparatus 90 executes synthesis processing with respect to the spatial structure data and the respective evaluation regions to which colors have been added. According to this synthesis processing, color information that has been associated with the respective evaluation regions is applied to voxels corresponding to the respective evaluation regions in the spatial structure data to generate spatial structure data with feature region information.

Subsequently, the arithmetic processing apparatus 90 executes display processing that includes processing that visualizes the spatial structure data with feature region information and displays a resulting image on the monitor 100. According to this display processing, similarly to the above described first embodiment, processing is performed that, for example, as shown in Fig. 19 or Fig. 20, generates a display screen that displays a plurality of kinds of spatial structure images, and displays the generated display screen on the monitor 100.

The configuration of the display screen, and operations to perform diagnosis using the display screen and setting of resection lines and the like are the same as in the first embodiment, and therefore a description thereof is omitted here. However, coloring of feature regions on a three-dimensional image displayed on the three-dimensional image display section 304, on a BC tomogram displayed on the BC tomogram display section 306, on an AC tomogram displayed on the AC tomogram display section 308, and on an AB tomogram displayed on the AB tomogram display section 310 that are shown in Fig. 19 and Fig. 20 is different to the first embodiment. According to the second embodiment, for example, in the BC tomogram displayed on the BC tomogram display section 306, as shown in Fig. 22B, the respective evaluation regions are color coded in accordance with a degree of risk that shows the degree of possibility of being a lesion part (cancer) and displayed. Therefore, the observer can ascertain at a glance a region to be observed with particular emphasis, and thus the workload of the observer is reduced.

Next, among the forms of displaying (display modes) images on the monitor 100 that are obtained by subjecting spatial structure data to visualization processing, display modes which are considered to be particularly preferable when implementing a colored display of feature regions by means of the above described spatial structure data with feature region information will be described. Note that the configuration of a display screen that is displayed on the monitor 100 is not necessarily limited to the configuration shown in Fig. 19 or Fig. 20, and a form in which an image of the respective display modes described hereunder is displayed on the screen of the monitor 100 is not limited to a specific form. Further, the respective display modes are not limited to a case in which feature regions are displayed with colors, and the display modes can also be applied to a display when the diagnosis support function is not used.

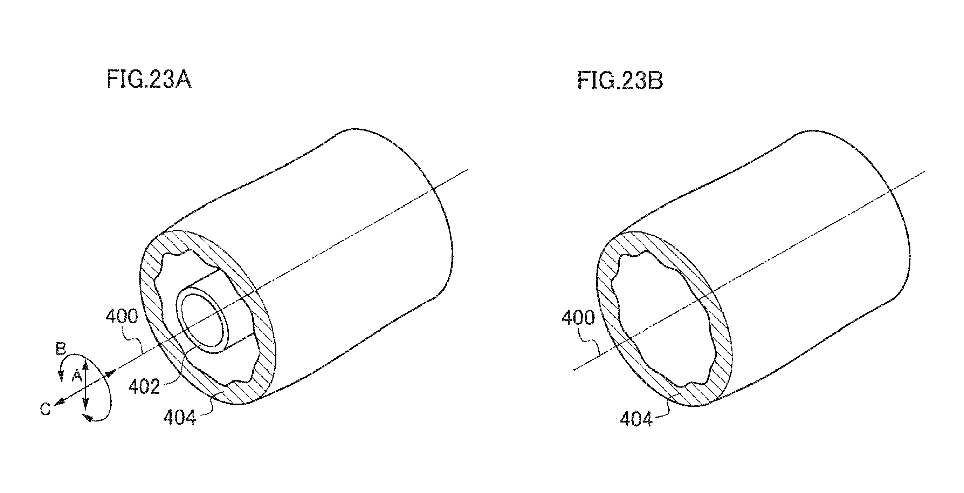

As shown in Fig. 23A, when spatial structure data (spatial structure data with feature region information) is represented by means of voxel space, the spatial structure data has a substantially cylindrical three-dimensional shape, similarly to the measurement region. A central axis 400 of the spatial structure data is a position (long axis of the OCT probe 40) at which the light-emitting end (optical lens 52) moves when performing C scanning. Scattered light intensity data for the sheath 44 of the OCT probe 40 exists in a cylindrical region in the vicinity of the central axis 400, and scattered light intensity data for the inner wall portion exists in on the outside thereof. Scattered light intensity data for a cavity has small values, and the data is treated as not existing. When a cavity region of a lumen portion is excluded, the spatial structure data is constituted by a cylindrical region 402 of the sheath 44 that is on the inner side, and a cylindrical region 404 of the inner wall portion that is on the outer side. The arithmetic processing apparatus 90 of the OCT apparatus 1 generates spatial structure images of various kinds of display forms that can be displayed on the monitor 100 by performing various kinds of visualization processing (rendering processing on the spatial structure data, and displays the generated images on the monitor 100. For example, the kinds of display modes that are particularly favorable when implementing a colored display of feature regions according to the diagnosis support function include: (1) an "endoscope mode", (2) a "perspective mode", (3) a "long-axis cross section mode", and (4) a "display mode" that are described below.

In this connection, in the "endoscope mode" and "perspective mode", spatial structure images are generated using spatial structure data, as shown in Fig. 23B, that is obtained by eliminating scattered light intensity data of the sheath 44 from spatial structure data in which scattered light intensity data of the sheath 44 (data of the region 402) exists, as shown in Fig. 23A. The processing that eliminates the scattered light intensity data of the sheath 44 is as described above.

### (1) "Endoscope mode"

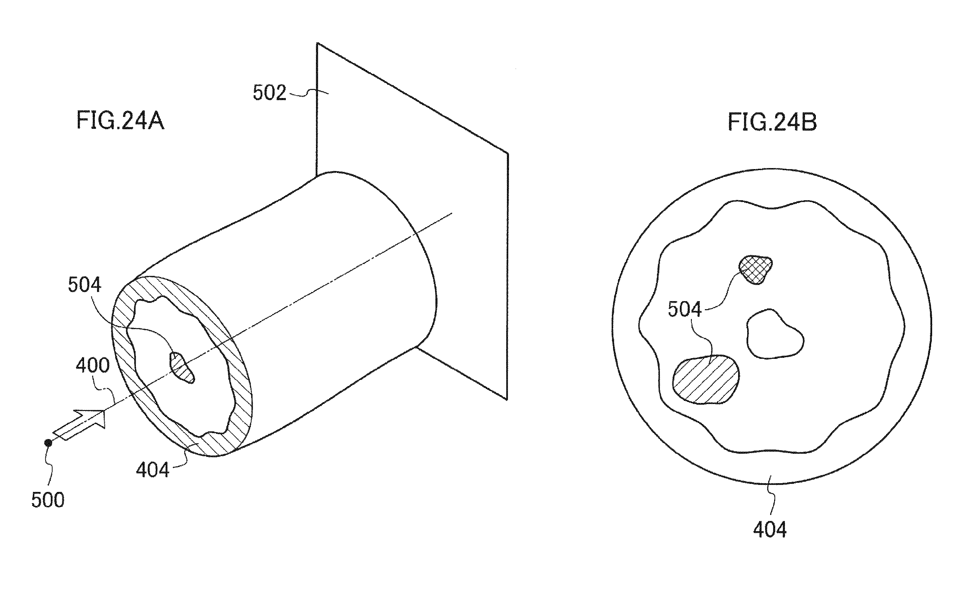

The endoscope mode is a mode that displays a three-dimensional image in which it appears as if the measurement region is being viewed with the endoscope that is inserted into the lumen of the biliary tract or pancreatic duct, and corresponds to a three-dimensional image that is displayed on the three-dimensional image display section 304 on the display screen in Fig. 19 and Fig. 20. According to the endoscope mode, as shown in Fig. 24A, with respect to spatial structure data, projection (perspective projection or central projection) processing is performed in which a projection centre 500 is set on the central axis 400 (or in the vicinity thereof), and a plane of projection 502 is set perpendicularly to the central axis 400, and as shown in Fig. 24B, a three-dimensional image that is similar to an image that is captured with an endoscope is generated and displayed.

When an image is generated in the endoscope mode using spatial structure data with feature region information as spatial structure data at a time of executing the diagnosis support function, as shown in Fig. 24B, colors (associated colors) are added to extracted feature regions 504 and displayed.

In this connection, in the endoscope mode, although normally only an image in which the surface of spatial structure data is projected (surface rendered) is displayed, a configuration may be adopted that allows the observer to switch between and display images generated by integral projection in which scattered light intensity data existing on the path of a light beam from the projection centre 500 to the plane of projection 502 is added up (integrated) and projected, maximum intensity projection (MIP) that projects maximum values of scattered light intensity data existing on the path of a light beam, and minimum intensity projection (MinIP) that projects minimum values of scattered light intensity data existing on the path of a light beam. A configuration may also be adopted that allows the observer to change the position of the projection centre 500, the position of the plane of projection 502, and the orientation thereof.

### (2) "Perspective mode"

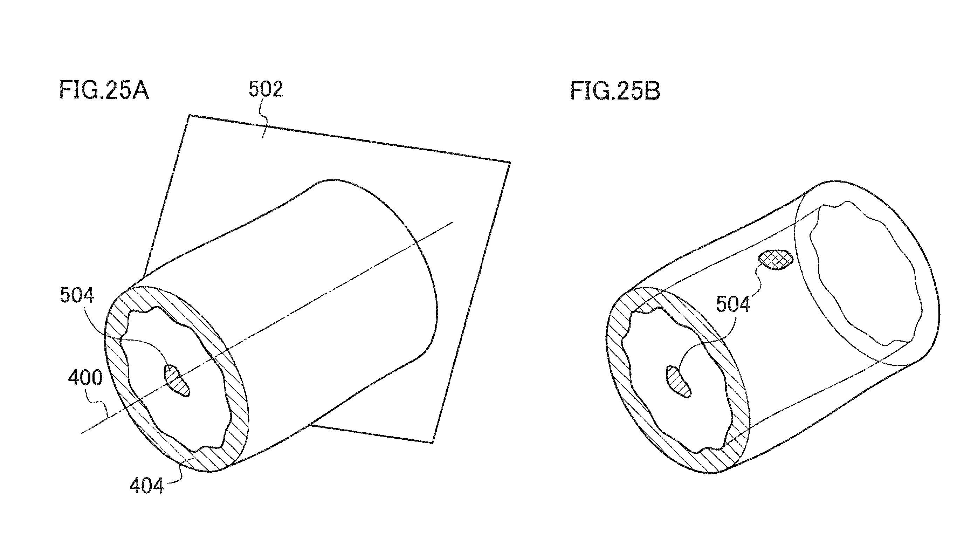

The perspective mode is a mode that displays a three-dimensional image of the entire measurement region of the biliary tract or pancreatic duct as viewed from an external diagonal direction, and corresponds to a three-dimensional image that is displayed on the three-dimensional image display section 304 on the display screen in Fig. 19 and Fig. 20. According to the perspective mode, as shown in Fig. 25A, with respect to spatial structure data, parallel projection processing is performed in which a plane of projection 520 is set that is not parallel with or orthogonal to the central axis 400 thereof, and as shown in Fig. 25B, a three-dimensional image in which all of the cylindrical spatial structure data that is viewed from a diagonal is generated and displayed. Further, according to the perspective mode, all of the spatial structure data is made semi-transparent (by volume rendering processing or the like) and displayed, and overlapping portions at the front and rear of the spatial structure data are also transparent.

When an image is generated in the perspective mode using spatial structure data with feature region information as spatial structure data, as shown in Fig. 25B, colors (associated colors) are added to extracted feature regions 504 and displayed.

In this connection, with respect to the perspective mode also, similarly to the endoscope mode, a configuration may be adopted that allows the observer to switch between and display images generated by integral projection in which scattered light intensity data existing on the path of a light beam to the plane of projection 502 is added up and projected, maximum intensity projection (MIP) that projects maximum values of scattered light intensity data existing on the path of a light beam, and minimum intensity projection (MinIP) that projects minimum values of scattered light intensity data existing on the path of a light beam. A configuration may also be adopted that allows the observer to change the position and orientation of the plane of projection 502.

### (3) "Long-axis cross section mode"

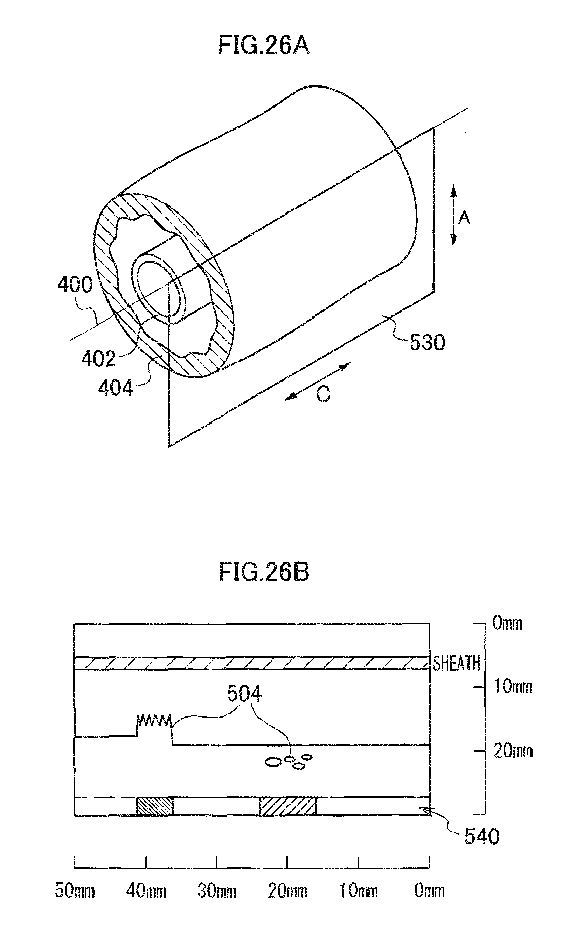

The long-axis cross section mode is a mode that displays a tomogram (AC tomogram) of a cross section obtained by reslicing a measurement region of the biliary tract or pancreatic duct in the long axis direction (C-axis direction) of the OCT probe 40, and corresponds to an image that is displayed on the AC tomogram display section 308 on the display screen shown in Fig. 19 or Fig. 20. According to the long-axis cross section mode, as shown in Fig. 26A, with respect to spatial structure data including scattered light intensity data of the sheath 44 (region 402), a plane (AC plane) that takes the central axis 400 thereof as one side is set as a cutting plane 530. Spatial structure data (scattered light intensity data) of an AC cross section that has been sectioned at the cutting plane 530 is subjected to visualization processing and, as shown in Fig. 26B, an AC tomogram is displayed together with a scale.

Further, when generating an image in the long-axis cross section mode using spatial structure data with feature region information as spatial structure data, as shown in Fig. 26B, a feature region display section 540 is displayed on the lower side of the AC tomogram, and colors (associated colors) are added to positions corresponding to feature regions and displayed. A configuration may also be adopted in which a cross section that has the most feature regions with respect to the entire circumference is automatically detected and the position thereof is set as the position of the cutting plane 530, and the apparatus may also be equipped with a function that automatically moves the cutting plane 530.

In this connection, with respect to the long-axis cross section mode also, a configuration may be adopted that allows the observer to switch between and display images generated not only based on scattered light intensity data at a cross section but also images that are generated by adding scattered light intensity data in a direction that is perpendicular to a cross section and performing visualization processing thereon, as well as images that are generated by performing visualization processing with respect to only maximum values or minimum values of scattered light intensity data at a cross section. A configuration may also be adopted that allows the observer to change a position in the B-axis direction of the cutting plane 530 (AC cross section), a range that is displayed on the monitor 100 as an AC tomogram, and a display magnification factor and the like.

### (4) "Display mode"

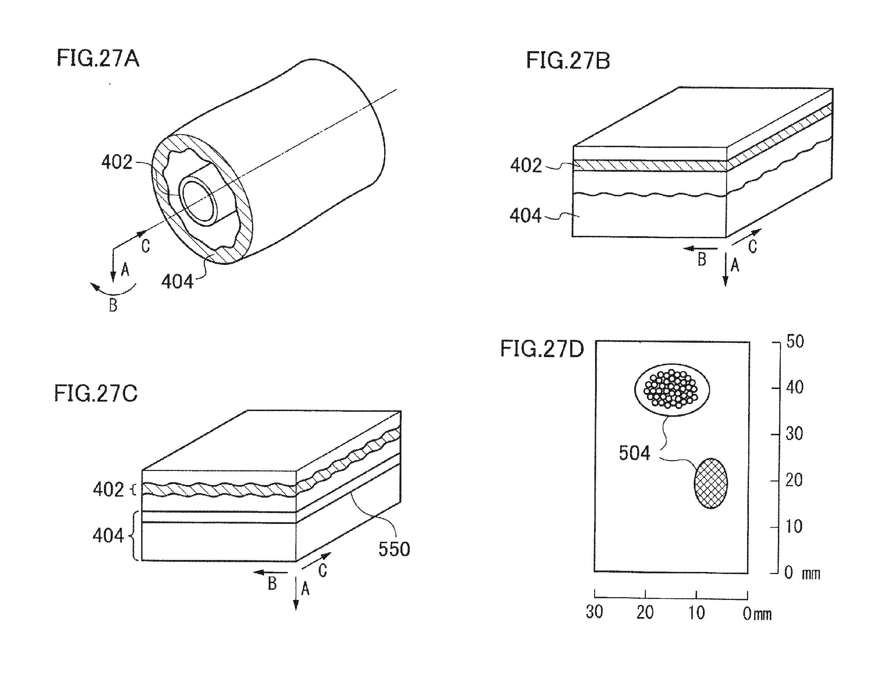

The display mode is a mode that displays a BC cross-sectional image of a cross section in which the surface (outermost surface) of an inner wall portion of a measurement region of the biliary tract or pancreatic duct, or the vicinity of the surface, has been resliced, and corresponds to an image that is displayed on the BC tomogram display section 306 in Fig. 20. According to the display mode, as shown in Fig. 27A, with respect to spatial structure data including scattered light intensity data of the sheath 44 (region 402), scattered light intensity data of voxels (voxels of a BC cross section) that are arranged at the same position in the A-axis direction (depth direction) is expanded as voxel values of voxels on the same plane. Further, the scale is converted so that a length in the B-axis direction at each position in the A-axis direction becomes a standard length, and spatial structure data of a rectangular parallelepiped shape is generated as shown in Fig. 27B. More specifically, for each line in the A-axis direction that passes through respective positions of the BC plane in the cylindrical spatial structure data shown in Fig. 27A, data that is obtained by arranging the scattered light intensity data on each line by taking the BC plane as a level surface and taking each line in the A-axis direction as parallel forms the spatial structure data of a rectangular parallelepiped shape that is shown in Fig. 27B.

Subsequently, by processing that detects positions of the outermost surface of the inner wall portion (region 404) and flattens the outermost surface, scattered light intensity data of voxels that are aligned on a line in the A-axis direction with respect to each position of the BC plane is shifted in the A-axis direction so that, as shown in Fig. 27C, scattered light intensity data of the outermost surface is arranged at voxels of a BC cross section at a predetermined position in the A-axis direction. The scattered light intensity data of the BC cross section or scattered light intensity data of a BC cross section 550 of the vicinity thereof (inner wall portion side) is subjected to visualization processing, and a BC tomogram is displayed as shown in Fig. 27D.

When an image is generated in the display mode using spatial structure data with feature region information as spatial structure data, as shown in Fig. 27D, colors (associated colors) are added to extracted feature regions 504 and displayed.

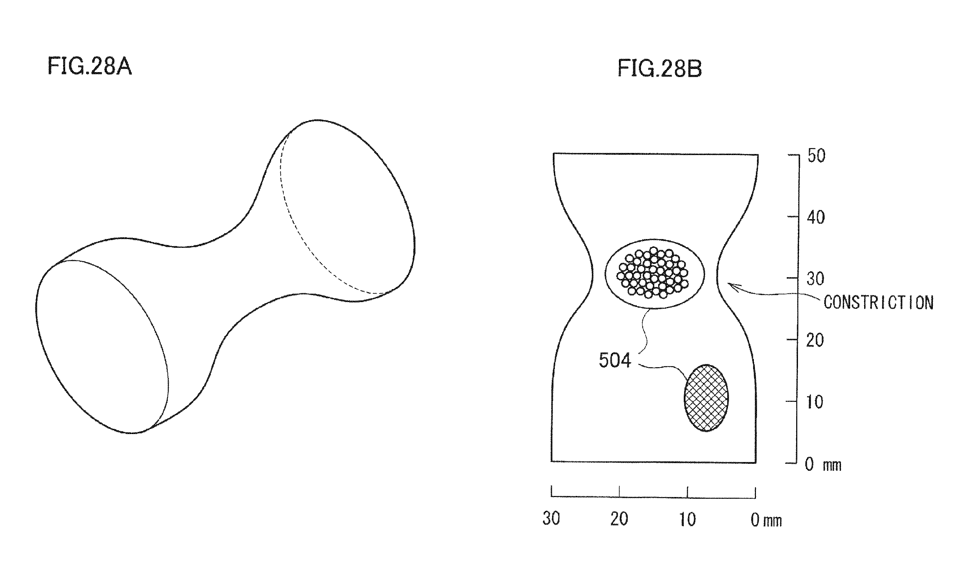

Note that, a configuration may also be adopted so as to perform processing that detects the outermost surface of the inner wall portion with respect to the cylindrical spatial structure data shown in Fig. 27A, develops the detected outermost surface on a plane surface, and displays an image in which the scattered light intensity data of the outermost surface is visualized. Thus, in a case where a constriction site exists in a lumen inside a measurement region and an outermost surface with a shape as shown in Fig. 28A is detected, since an image as shown in Fig. 27B is displayed, the constriction site can be ascertained instantly. When using spatial structure data with feature region information as spatial structure data, as shown in Fig. 27B, colors (associated colors) are added to the feature regions 504 and displayed.

Further, with respect to the display mode also, a configuration may be adopted that allows the observer to switch between and display images generated by adding scattered light intensity data in a direction that is perpendicular to an intraductal wall surface (cross section) and performing visualization processing thereon, and images generated by performing visualization processing with respect to only maximum values or minimum values of scattered light intensity data at a surface. A configuration may also be adopted that allows the observer to change a position in the A-axis direction of the BC cross section 550, a range that is displayed on the monitor 100 as a BC cross-sectional image, and a display magnification factor and the like.

## Claims

1. A diagnosis support apparatus (1), comprising:
a spatial structure data acquisition means (30) that acquires spatial structure data comprising tomographic information of a three-dimensional region of an inner wall portion within a living organism that has a flat surface at a normal time that is obtained by performing optical coherence tomography measurement with respect to the inner wall portion;
a surface roughness calculation means (90) that calculates an evaluation value of a surface roughness at respective positions on the surface of the inner wall portion based on the spatial structure data that is acquired by the spatial structure data acquisition means;
a lesion part extraction means (90) that extracts a region of a lesion part based on a position on the surface at which the evaluation value that is calculated by the surface roughness calculation means exceeds a predetermined threshold value; and
a lesion part display means (100) that displays information showing the region of the lesion part that is extracted by the lesion part extraction means on an image in which the spatial structure data is visualized.

2. The diagnosis support apparatus (1) according to claim 1, wherein the surface roughness calculation means (90) comprises:
a surface detection means that detects a position of the surface based on a change in a value of the spatial structure data with respect to a depth direction of the inner wall portion; and
a mean position calculation means that determines a mean position in the depth direction in a predetermined range of the surface based on positions of the surface that are detected by the surface detection means;
wherein when a mean position that is calculated by the mean position calculation means in the predetermined range of the surface is taken as a fixed position in the depth direction, a difference amount between a deepest position and a shallowest position of the surface with regard to the depth direction is calculated as the evaluation value.

3. The diagnosis support apparatus (1) according to claim 1 or 2, wherein when a continuous region of a size that is equal to or greater than a predetermined size is formed by positions on the surface at which the evaluation value exceeds a predetermined threshold value, the lesion part extraction means extracts the region as a region of a lesion part.

4. The diagnosis support apparatus (1) according to claim 1, 2, or 3, wherein the lesion part display means (100) applies a predetermined color to a region of the lesion part on an image in which the spatial structure data is visualized and displays a resulting image.

5. The diagnosis support apparatus (1) according to any one of claims 1 to 4, wherein, as an image in which the spatial structure data is visualized, the lesion part display means (100) displays at least any one image among a group comprising a three-dimensional image obtained by perspective projection processing, a three-dimensional image obtained by parallel projection processing, and a tomogram of a predetermined cross section.

6. The diagnosis support apparatus (1) according to any one of claims 1 to 5, wherein the inner wall portion is an inner wall portion of a biliary tract, a pancreatic duct, a bronchial tube, a pharynx, an esophagus, or a urinary duct that has a flat epithelial structure at a normal time.

7. A diagnosis support apparatus (1), comprising:
a light intensity data acquisition means (30) that acquires light intensity data of an inner wall portion within a living organism that is obtained by performing optical coherence tomography measurement with respect to the inner wall portion;
an evaluation value calculation means (90) that, based on light intensity data acquired by the light intensity data acquisition means, calculates an evaluation value corresponding to a length from a measurement point that is set on a surface of the inner wall portion to a boundary point at which the light intensity data with regard to a depth direction of the inner wall portion becomes a noise level;
a detection means (90) that compares a size of the evaluation value that is calculated by the evaluation value calculation means and a size of a predetermined threshold value, and detects a measurement point at which the evaluation value is less than the threshold value as a lesion part; and
a lesion part display means (100) that displays information showing a region of the lesion part that is detected by the detection means on an image in which the light intensity data is visualized.

8. The diagnosis support apparatus (1) according to claim 7, wherein the detection means (90) detects a measurement point at which the evaluation value is less than the threshold value, and when a set of the measurement points forms a continuous region of a size that is equal to or greater than a predetermined size, the detection means detects the region as a lesion part.

9. The diagnosis support apparatus (1) according to claim 7 or 8, further comprising:
a contact region distinguishing means (90) that, based on the light intensity data, with respect to the surface of the inner wall portion, distinguishes between an optical probe contact region which an optical probe that irradiates a measuring light on the inner wall portion contacts and an optical probe non-contact region which the optical probe does not contact at a time of the optical coherence tomography measurement; and
a threshold value changing means (90) that changes the threshold value in the detection means so as to set respectively different threshold values for the optical probe contact region and the optical probe non-contact region that are distinguished by the contact region distinguishing means.

10. The diagnosis support apparatus (1) according to claim 9, further comprising:
a probe detection means (30) that detects a position of an outer circumferential face of the optical probe based on the light intensity data;
wherein when a position of the outer circumferential face of the optical probe that is detected by the probe detection means and a position of the surface of the inner wall portion that is detected by the surface detection means are in a range in which the positions are regarded as being at an identical position, the contact region distinguishing means determines that a region in question is an optical probe contact region, and in other cases the contact region distinguishing means determines that a region in question is an optical probe non-contact region.

11. The diagnosis support apparatus (1) according to claim 9 or 10, wherein, as the threshold value, the threshold value changing means sets a value for the optical probe contact region that is smaller than a value for the optical probe non-contact region.

12. The diagnosis support apparatus (1) according to any one of claims 7 to 11, wherein the lesion part display means (100) applies a predetermined color to the lesion part on an image in which the light intensity data is visualized and displays a resulting image.

13. The diagnosis support apparatus (1) according to any one of claims 7 to 12, wherein the light intensity data acquisition means (90) acquires light intensity data of a three-dimensional region of the inner wall portion.

14. The diagnosis support apparatus (1) according to claim 13, wherein, as an image in which the light intensity data is visualized, the lesion part display means (100) displays at least any one image among a group comprising a three-dimensional image obtained by perspective projection processing, a three-dimensional image obtained by parallel projection processing, and a tomogram of a predetermined cross section.

15. The diagnosis support apparatus (1) according to any one of claims 7 to 14, wherein the inner wall portion is an inner wall portion of a biliary tract, a pancreatic duct, a bronchial tube, a pharynx, an esophagus, a stomach, a colon, a uterus, or a urinary duct.

16. A diagnosis support method, comprising:
a light intensity data acquisition step of acquiring light intensity data of an inner wall portion within a living organism that is obtained by performing optical coherence tomography measurement with respect to the inner wall portion;
an evaluation value calculation step of, based on light intensity data acquired by the light intensity data acquisition step, calculating an evaluation value that shows a length from a measurement point that is set on a surface of the inner wall portion to a boundary point at which the light intensity data with regard to a depth direction of the inner wall portion becomes a noise level;
a detection step of comparing a size of the evaluation value that is calculated by the evaluation value calculation step and a size of a predetermined threshold value, and detecting a measurement point at which the evaluation value is less than the threshold value as a lesion part; and
a lesion part display step of displaying information showing a region of the lesion part that is detected by the detection step on an image in which the light intensity data is visualized.

17. A lesion part detection apparatus (1), comprising:
a light intensity data acquisition means (30) that acquires light intensity data of an inner wall portion within a living organism that is obtained by performing optical coherence tomography measurement with respect to the inner wall portion;
an evaluation value calculation means (90) that, based on light intensity data acquired by the light intensity data acquisition means, calculates an evaluation value corresponding to a length from a measurement point that is set on a surface of the inner wall portion to a boundary point at which the light intensity data with regard to a depth direction of the inner wall portion becomes a noise level; and
a detection means (90) that compares a size of the evaluation value that is calculated by the evaluation value calculation means and a size of a predetermined threshold value, and detects a measurement point at which the evaluation value is less than the threshold value as a lesion part.

18. A lesion part detection method, comprising:
a light intensity data acquisition step of acquiring light intensity data of an inner wall portion within a living organism that is obtained by performing optical coherence tomography measurement with respect to the inner wall portion;
an evaluation value calculation step of, based on light intensity data that is acquired by the light intensity data acquisition step, calculating an evaluation value corresponding to a length from a measurement point that is set on a surface of the inner wall portion to a boundary point at which the light intensity data with regard to a depth direction of the inner wall portion becomes a noise level; and
a detection step of comparing a size of the evaluation value that is calculated by the evaluation value calculation step and a size of a predetermined threshold value, and detecting a measurement point at which the evaluation value is less than the threshold value as a lesion part.

19. A diagnosis support apparatus (1), comprising:
a light intensity data acquisition means (30) that acquires light intensity data of an inner wall portion within a living organism that has a layered structure that is obtained by performing optical coherence tomography measurement with respect to the inner wall portion;
an addition means (90) that, based on light intensity data that is acquired by the light intensity data acquisition means, adds light intensity data of a predetermined range at a same depth with regard to a depth direction of the inner wall portion from a measurement point set on a surface of the inner wall portion;
a detection means (90) that detects the measurement point as a lesion part when an addition value that is calculated by the addition means shows a predetermined change along the depth direction; and
a lesion part display means (100) that displays information showing the lesion part that is detected by the detection means on an image in which the light intensity data is visualized.

20. The diagnosis support apparatus (1) according to claim 19, wherein when the addition value that is calculated by the addition means is in a range that is regarded as attenuating in a constant manner along the depth direction, the detection means detects the measurement point as a lesion part.

21. The diagnosis support apparatus (1) according to claim 19 or 20, wherein the detection means (90) detects a measurement point at which an addition value that is calculated by the addition means shows a predetermined change along the depth direction, and when a set of the measurement points forms a continuous region that is equal to or greater than a predetermined size, the detection means detects the region as a lesion part.

22. The diagnosis support apparatus (1) according to any one of claims 19 to 21, wherein the lesion part display means (100) applies a predetermined color to a region of the lesion part on an image in which the light intensity data is visualized and displays a resulting image.

23. The diagnosis support apparatus (1) according to any one of claims 19 to 22, wherein the light intensity data acquisition means (30) acquires light intensity data of a three-dimensional region of the inner wall portion.

24. The diagnosis support apparatus (1) according to any one of claims 19 to 23, wherein, as an image in which the light intensity data is visualized, the lesion part display means (100) displays at least any one image among a group comprising a three-dimensional image obtained by perspective projection processing, a three-dimensional image obtained by parallel projection processing, and a tomogram of a predetermined cross section.

25. The diagnosis support apparatus (1) according to any one of claims 19 to 24, wherein the inner wall portion is an inner wall portion of a biliary tract, a pancreatic duct, a bronchial tube, a pharynx, an esophagus, a stomach, a colon, a uterus, or a urinary duct.

26. A diagnosis support method, comprising:
a light intensity data acquisition step of acquiring light intensity data of an inner wall portion within a living organism that has a layered structure that is obtained by performing optical coherence tomography measurement with respect to the inner wall portion;
an addition step of, based on light intensity data that is acquired by the light intensity data acquisition step, adding light intensity data of a predetermined range at a same depth with regard to a depth direction of the inner wall portion from a measurement point set on a surface of the inner wall portion;
a detection step of detecting the measurement point as a lesion part when an addition value that is calculated by the addition step shows a predetermined change along the depth direction; and
a lesion part display step of displaying information showing the lesion part that is detected by the detection step on an image in which the light intensity data is visualized.

27. A lesion part detection apparatus (1), comprising:
a light intensity data acquisition means (30) that acquires light intensity data of an inner wall portion within a living organism that has a layered structure that is obtained by performing optical coherence tomography measurement with respect to the inner wall portion;
an addition means (90) that, based on light intensity data that is acquired by the light intensity data acquisition means, adds light intensity data of a predetermined range at a same depth with regard to a depth direction of the inner wall portion from a measurement point set on a surface of the inner wall portion; and
a detection means (90) that detects the measurement point as a lesion part when an addition value that is calculated by the addition means shows a predetermined change along the depth direction.

28. A lesion part detection method, comprising:
a light intensity data acquisition step of acquiring light intensity data of an inner wall portion within a living organism that has a layered structure that is obtained by performing optical coherence tomography measurement with respect to the inner wall portion;
an addition step of, based on light intensity data that is acquired by the light intensity data acquisition step, adding light intensity data of a predetermined range at a same depth with regard to a depth direction of the inner wall portion from a measurement point set on a surface of the inner wall portion; and
a detection step of detecting the measurement point as a lesion part when an addition value that is calculated by the addition step shows a predetermined change along the depth direction.

29. A diagnosis support apparatus (1), comprising:
a light intensity data acquisition means (30) that acquires light intensity data of an inner wall portion within a living organism that is obtained by performing optical coherence tomography measurement with respect to the inner wall portion;
an extraction means (90) that extracts a plurality of feature regions which are suspected of being a lesion part based on light intensity data that is acquired by the light intensity data acquisition means;
a weight assigning means (90) that assigns points in accordance with a degree of possibility of being a lesion part to each feature region that is extracted by the extraction means;
a degree of risk setting means (90) that sets a degree of risk at respective positions of the inner wall portion based on points that are assigned to each feature region by the weight assigning means; and
a display means (100) that, on an image in which the light intensity data is visualized, assigns a different color for each degree of risk at the respective positions of the inner wall portion for which a degree of risk is set by the degree of risk setting means, and displays a resulting image.

30. The diagnosis support apparatus (1) according to claim 29, further comprising:
a classifying means (90) that classifies the plurality of feature regions extracted by the extraction means into predetermined categories;
wherein the weight assigning means (90) assigns points to each feature region in accordance with a degree of possibility of being a lesion part with respect to each category into which the plurality of feature regions are classified by the classifying means.

31. The diagnosis support apparatus (1) according to claim 30, wherein the classifying means (90) classifies each feature region according to a kind of detection processing that extracts each feature region.

32. The diagnosis support apparatus (1) according to claim 30 or 31, wherein the classifying means (90) classifies the plurality of feature regions into at least any one category among a group comprising a region in which a layered structure of the inner wall portion has disappeared, a region in which an outermost surface layer of the inner wall portion is thickened, a region in which a surface roughness of a surface of the inner wall portion is abnormal, and a region in which a lumen exists in the inner wall portion.

33. The diagnosis support apparatus (1) according to any one of claims 30 to 32, further comprising:
a criterion setting means (90) that sets a criterion for classifying the plurality of feature regions into predetermined categories;
wherein the classifying means (90) classifies the plurality of feature regions according to the criterion that is set by the criterion setting means.

34. The diagnosis support apparatus (1) according to any one of claims 29 to 33, wherein, with respect to an overlapping region in which at least some of the plurality of feature regions overlap, the degree of risk setting means adds points that are assigned to each feature region, respectively, and sets a degree of risk with respect to the overlapping region based on the addition value.

35. The diagnosis support apparatus (1) according to any one of claims 29 to 34, wherein the light intensity data acquisition means acquires light intensity data of a three-dimensional region of the inner wall portion.

36. The diagnosis support apparatus (1) according to any one of claims 29 to 35, wherein, as an image in which the spatial structure data is visualized, the display means (100) displays at least any one image of a group comprising a three-dimensional image obtained by perspective projection processing, a three-dimensional image obtained by parallel projection processing, and a tomogram of a predetermined cross section.

37. The diagnosis support apparatus (1) according to any one of claims 29 to 36, wherein the inner wall portion is an inner wall portion of a biliary tract, a pancreatic duct, a bronchial tube, a pharynx, an esophagus, a stomach, a colon, a uterus, or a urinary duct.

38. A diagnosis support method, comprising:
a light intensity data acquisition step of acquiring light intensity data of an inner wall portion within a living organism that is obtained by performing optical coherence tomography measurement with respect to the inner wall portion;
an extraction step of extracting a plurality of feature regions which are suspected of being a lesion part based on light intensity data that is acquired by the light intensity data acquisition step;
a weight assigning step of assigning points in accordance with a degree of possibility of being a lesion part to each feature region that is extracted by the extraction step;
a degree of risk setting step of setting a degree of risk at respective positions of the inner wall portion based on points that are assigned to each feature region by the weight assigning step; and
a display step of assigning a different color for each degree of risk at the respective positions of the inner wall portion for which a degree of risk is set by the degree of risk setting step on an image in which the light intensity data is visualized, and displaying a resulting image.

39. A lesion part detection apparatus (1), comprising:
a light intensity data acquisition means (30) that acquires light intensity data of an inner wall portion within a living organism that is obtained by performing optical coherence tomography measurement with respect to the inner wall portion;
an extraction means (90) that extracts a plurality of feature regions which are suspected of being a lesion part based on light intensity data that is acquired by the light intensity data acquisition means;
a weight assigning means (90) that assigns points in accordance with a degree of possibility of being a lesion part to each feature region that is extracted by the extraction means; and
a degree of risk setting means (90) that sets a degree of risk at respective positions of the inner wall portion based on points that are assigned to each feature region by the weight assigning means.

40. A lesion part detection method, comprising:
a light intensity data acquisition step of acquiring light intensity data of an inner wall portion within a living organism that is obtained by performing optical coherence tomography measurement with respect to the inner wall portion;
an extraction step of extracting a plurality of feature regions which are suspected of being a lesion part based on light intensity data that is acquired by the light intensity data acquisition step;
a weight assigning step of assigning points in accordance with a degree of possibility of being a lesion part to each feature region that is extracted by the extraction step; and
a degree of risk setting step of setting a degree of risk at respective positions of the inner wall portion based on points that are assigned to each feature region by the weight assigning step.

41. A diagnosis support apparatus (1), comprising:
a light intensity data acquisition means (30) that acquires light intensity data of an inner wall portion within a living organism that has a first layer of at least a predetermined thickness on a surface thereof that is obtained by performing optical coherence tomography measurement with respect to the inner wall portion;
a detection means (90) that, based on light intensity data that is acquired by the light intensity data acquisition means, compares a thickness of the first layer at a measurement point that is set on the surface of the inner wall portion and a predetermined threshold value, and detects the measurement point as a lesion part when the thickness of the first layer is a value that is greater than the threshold value;
a lesion part display means (100) that displays information that shows a lesion part that is detected by the detection means on an image in which the light intensity data is visualized;
a contact region distinguishing means (90) that, based on the light intensity data, with respect to the surface of the inner wall portion, distinguishes between an optical probe contact region which an optical probe that irradiates a measuring light on the inner wall portion contacts and an optical probe non-contact region which the optical probe does not contact at a time of the optical coherence tomography measurement; and
a threshold value changing means (90) that changes the threshold value in the detection means so as to set respectively different threshold values for the optical probe contact region and the optical probe non-contact region that are distinguished by the contact region distinguishing means.

42. The diagnosis support apparatus (1) according to claim 41, further comprising:
a probe detection means (30) that detects a position of an outer circumferential face of the optical probe based on the light intensity data;
wherein when a position of the outer circumferential face of the optical probe that is detected by the probe detection means and a position of the surface of the inner wall portion are in a range in which the positions are regarded as being at an identical position, the contact region distinguishing means determines that a region in question is an optical probe contact region, and in other cases the contact region distinguishing means determines that a region in question is an optical probe non-contact region.

43. The diagnosis support apparatus (1) according to claim 41 or 42, wherein, as the threshold value, the threshold value changing means sets a value for the optical probe contact region that is smaller than a value for the optical probe non-contact region.

44. The diagnosis support apparatus (1) according to claim 41, 42, or 43, wherein the detection means (90) comprises:
a surface detection means that detects a surface of the first layer based on the light intensity data;
a boundary detection means that detects a boundary between the first layer and a second layer that adjoins the first layer in a depth direction;
a thickness calculation means that calculates a thickness of the first layer based on a difference in the depth direction between a surface that is detected by the surface detection means and a boundary that is detected by the boundary detection means; and
a comparison means that compares a thickness of the first layer that is calculated by the thickness calculation means at a measurement point that is set on the surface of the inner wall portion and the threshold value.

45. The diagnosis support apparatus (1) according to any one of claims 41 to 44, wherein the detection means (90) detects a measurement point at which a thickness of the first layer is a value that is greater than the threshold value, and when a set of the measurement points form a continuous region of a predetermined size, the detection means detects the region as a lesion part.

46. The diagnosis support apparatus (1) according to any one of claims 41 to 45, wherein the lesion part display means (100) applies a predetermined color to a region of the lesion part on an image in which the light intensity data is visualized, and displays a resulting image.

47. The diagnosis support apparatus (1) according to any one of claims 41 to 46, wherein, as an image in which the light intensity data is visualized, the lesion part display means (100) displays at least any one image among a group comprising a three-dimensional image obtained by perspective projection processing, a three-dimensional image obtained by parallel projection processing, and a tomogram of a predetermined cross section.

48. The diagnosis support apparatus (1) according to any one of claims 41 to 47, wherein the inner wall portion is an inner wall portion of a biliary tract, a pancreatic duct, a bronchial tube, a pharynx, an esophagus, a stomach, a colon, a uterus, or a urinary duct.

49. The diagnosis support apparatus (1) according to any one of claims 41 to 48, wherein the light intensity data acquisition means acquires light intensity data of a three-dimensional region of the inner wall portion.

50. A diagnosis support method, comprising:
a light intensity data acquisition step of acquiring light intensity data of an inner wall portion within a living organism that has a first layer of at least a predetermined thickness on a surface thereof that is obtained by performing optical coherence tomography measurement with respect to the inner wall portion;
a detection step of, based on light intensity data that is acquired by the light intensity data acquisition step, comparing a thickness of the first layer at a measurement point that is set on the surface of the inner wall portion and a predetermined threshold value, and detecting the measurement point as a lesion part when the thickness of the first layer is a value that is greater than the threshold value;
a lesion part display step of displaying information that shows a lesion part that is detected by the detection step on an image in which the light intensity data is visualized;
a contact region distinguishing step of, based on the light intensity data, with respect to the surface of the inner wall portion, distinguishing between an optical probe contact region which an optical probe that irradiates a measuring light on the inner wall portion contacts and an optical probe non-contact region which the optical probe does not contact at a time of the optical coherence tomography measurement; and
a threshold value changing step of changing the threshold value in the detection step so as to set respectively different threshold values for the optical probe contact region and the optical probe non-contact region that are distinguished by the contact region distinguishing step.

51. A lesion part detection apparatus (1), comprising:
a light intensity data acquisition means (30) that acquires light intensity data of an inner wall portion within a living organism that has a first layer of at least a predetermined thickness on a surface thereof that is obtained by performing optical coherence tomography measurement with respect to the inner wall portion;
a detection means (90) that, based on light intensity data that is acquired by the light intensity data acquisition means, compares a thickness of the first layer at a measurement point that is set on the surface of the inner wall portion and a predetermined threshold value, and detects the measurement point as a lesion part when the thickness of the first layer is a value that is greater than the threshold value;
a contact region distinguishing means (90) that, based on the light intensity data, with respect to the surface of the inner wall portion, distinguishes between an optical probe contact region which an optical probe that irradiates a measuring light on the inner wall portion contacts and an optical probe non-contact region which the optical probe does not contact at a time of the optical coherence tomography measurement; and
a threshold value changing means (90) that changes the threshold value in the detection means so as to set respectively different threshold values for the optical probe contact region and the optical probe non-contact region that are distinguished by the contact region distinguishing means.

52. A lesion part detection method, comprising:
a light intensity data acquisition step of acquiring light intensity data of an inner wall portion within a living organism that has a first layer of at least a predetermined thickness on a surface thereof that is obtained by performing optical coherence tomography measurement with respect to the inner wall portion;
a detection step of, based on light intensity data that is acquired by the light intensity data acquisition step, comparing a thickness of the first layer at a measurement point that is set on the surface of the inner wall portion and a predetermined threshold value, and detecting the measurement point as a lesion part when the thickness of the first layer is a value that is greater than the threshold value;
a contact region distinguishing step of, based on the light intensity data, with respect to the surface of the inner wall portion, distinguishing between an optical probe contact region which an optical probe that irradiates a measuring light on the inner wall portion contacts and an optical probe non-contact region which the optical probe does not contact at a time of the optical coherence tomography measurement; and
a threshold value changing step of changing the threshold value in the detection step so as to set respectively different threshold values for the optical probe contact region and the optical probe non-contact region that are distinguished by the contact region distinguishing step.
